# EUROPEAN PATENT APPLICATION

(11) **EP 4 840 469 A1**
(43) Date of publication of application: **18.11.2026**
(21) Application number: 25176924.6
(22) Date of filing: 16.05.2025
(51) Int. Cl.: A61K 47/64, A61P 31/00, A61P 35/00, A61P 37/02, C07K 7/08

(54) **CONJUGATES OF A MICROPEPTIDE KILLSWITCH**

(71) Applicant: Max-Planck-Gesellschaft zur Förderung der Wissenschaften e.V., 80539 München (DE)
(72) Inventor: The designation of the inventor has not yet been filed
(74) Representative: Weickmann & Weickmann PartmbB

(57) **Abstract**

The present invention relates to conjugates of a peptide which is capable of immobilizing a biomolecule that is a component of a biomolecular condensate and of disrupting the function of the condensate.

## Description

The present invention relates to conjugates of a peptide which is capable of immobilizing a biomolecule that is a component of a biomolecular condensate and of disrupting the function of the condensate.

### Background

Cells organize their biochemistry within localized membraneless compartments generally referred to as biomolecular condensates ^{1,2}. Condensates are concentrates of various biopolymers, including proteins and nucleic acids, and are thought to have emergent properties and functions beyond those of their individual constituent molecules ¹⁻⁷. For example, condensates may create microenvironments that facilitate the partitioning of small molecules into the condensates, in a manner not explained by stoichiometric interactions ⁸⁻¹³. Moreover, recent studies suggest that the material and viscoelastic properties of condensates impact the activities of their constituent molecules ¹⁴⁻¹⁸. These findings, together with substantial in vitro biochemistry experiments, support an emergent view that the material state of molecules within condensates is inherently linked to biological functions that emerge from concentrating them in one location ¹⁹. However, manipulating condensate material properties, and experimental manipulation of emergent condensate functions in vivo has been a major challenge.

Probing condensate properties and function in vivo is challenging because of the limitations of currently existing tools. Several technologies have been developed to induce condensation of proteins in cells in the past few years ^{16,20-27}. Manipulation of the material properties and functions of condensates in cells nevertheless has been more difficult. Genetic perturbation experiments that attempt to separate condensate formation from biological activity have been useful ²⁸⁻³⁰, but the readout of such experiments occurs dozens of cell generations after the genetic manipulation, by which time cells have adapted to the genetic changes.

Small molecules that selectively target specific condensates are mostly lacking. Aliphatic alcohols, such as 1,6-hexanediol are commonly used, but such compounds disrupt weak hydrophobic interactions without any specificity, and thus dissolve condensates in a nonspecific manner ⁵. For a few condensates, e.g., stress-granules, selective molecules have been discovered ^{9,17,31-33,} but for most condensates such compounds remain to be identified. A few studies have reported protein-tagging approaches to dissolve ectopic condensates ^{23,34,35}. Deep light stimulation of proteins fused to the photo-oligomerizing Cry2-domain has been used to drive nucleoli into an arrested state ^{21,36}, but such approaches rely on ectopic overexpression of condensate-forming proteins whose behavior is known to significantly differ from those of native proteins ^{5,37}.

In summary, current approaches do not inform on the material properties of endogenous condensates, nor do they inform on the mechanistic underpinning of how material properties of molecules in endogenous condensate enable emergent functions. Condensate alterations have been linked to hundreds of human diseases ³⁸⁻⁴⁰.

It is an object of the present invention, to provide broadly applicable approaches to target endogenous condensates, and change their material properties and provide important insights into emergent functions of condensates implicated in a large variety of processes in health and disease.

### SUMMARY OF THE INVENTION

The present invention to conjugates, compositions and methods for controlling the dynamics of a biomolecular condensate.

A first aspect of the present invention relates to a conjugate comprising:
(a) a peptide which is capable of immobilizing a biomolecule that is a component of a biomolecular condensate and of disrupting the function of the biomolecular condensate;
(b) optionally a linker; and
(c) a targeting moiety capable of targeting the conjugate to a biomolecular condensate.

In certain embodiments, the conjugate is a fusion polypeptide.

A further aspect of the present invention relates to a nucleic acid molecule encoding the conjugate and a vector comprising a nucleic acid molecule encoding the conjugate.

A further aspect of the present invention relates to a recombinant cell transformed or transfected with the nucleic acid molecule.

A further aspect of the present invention relates to a pharmaceutical composition comprising the conjugate, the nucleic acid molecule or the vector as an active agent and a pharmaceutically acceptable carrier.

A further aspect of the present invention relates to the conjugate, the nucleic acid molecule, the vector or the pharmaceutical composition for use as a medicament.

Still a further aspect of the present invention relates to the in vitro use of the conjugate, the nucleic acid molecule, or the vector for arresting the dynamics of a mammalian cell.

Still a further aspect of the present invention is a method of treating a disorder associated with the presence or with an increased amount of a pathological biomolecular condensate, comprising administering to a subject in thereof an effective amount of the conjugate, the nucleic acid molecule, the vector or the pharmaceutical composition.

### EMBODIMENTS OF THE INVENTION

In the following, embodiments of the invention are disclosed as part of the description.
1. A conjugate comprising:
   (a) a peptide which is capable of immobilizing a biomolecule that is a component of a biomolecular condensate and of disrupting the function of the biomolecular condensate;
   (b) optionally a linker; and
   (c) a targeting moiety capable of targeting the conjugate to a biomolecular condensate.
2. The conjugate of embodiment 1, wherein the targeting moiety (c) is a polypeptide.
3. The conjugate of embodiment 1 or 2, wherein the targeting moiety (c)
   (i) is a condensate-forming polypeptide,
   (ii) is a moiety that binds to a component of a biomolecular condensate, or
   (iii) is a moiety that is capable of cell penetration.
4. The conjugate of any one of embodiments 1-3, which is a fusion polypeptide wherein component (a), component (b), if present, and component (c) are covalently bound to each other.
5. The conjugate of any one of embodiments 1-4, wherein the peptide (a), when expressed as HMGB1 variant fused to a GFP tag, localizes to the nucleolus of a mammalian cell and causes nucleolar arrest.
6. The conjugate of any one of embodiments 1-5, wherein the targeting polypeptide (c) does not contain an amino acid sequence from a HMGB1 polypeptide.
7. The conjugate of any one of embodiments 1-6, wherein the peptide (a) has a length of about 10 to about 100 amino acids, of about 12 to about 50 amino acids, of about 15 to about 20 amino acids.
8. The conjugate of any one of embodiments 1-7, wherein the peptide (a) is capable of self-association.
9. The conjugate of any one of embodiments 1-8, wherein the peptide (a) comprises an amino acid sequence having at least 2, particularly at least three aromatic amino acid residues selected from F, W and Y.
10. The conjugate of any one of embodiments 1-9, wherein the peptide (a) comprises an amino acid sequence having at least 2, particularly at least three aromatic amino acid residues selected from F.
11. The conjugate of any one of embodiments 1-10, wherein the peptide (a) comprises or consists of (i) an amino acid sequence selected from:
   - MMMMNKLVLAQFFFSCL (SEQ ID NO: 1)
   - MMMMNKLVLAQAFFSCL(SEQ ID NO: 2)
   - MMMMNKLVLAQFAFSCL(SEQ ID NO: 3)
   - MMMMNKLVLAQFFFSAL(SEQ ID NO: 4)
   - EDEDNKLVLAQFFFSCL (SEQ ID NO: 5)
   - MMMFMNKFLVLAQFSCL (SEQ ID NO: 6)
   - MFMMMNFKLVLAFQSCL (SEQ ID NO: 7)
   - MMFMMNKLFVLAQSCFL (SEQ ID NO: 8)
   - MMMMFFFNKLVLAQSCL (SEQ ID NO: 9)
   - MMMMNKLVFFFLAQSCL (SEQ ID NO: 10)
   - MMLALKSCFVMMQLFFN (SEQ ID NO: 11)
   - AMMNFFQLMFLVKSMLC (SEQ ID NO: 12)
   - FMAFLNVLLKMQCMSFM (SEQ ID NO: 13)
   - CFMLNKFLVMQMSFMLA (SEQ ID NO: 14)
   - CVKLSMNAQFLLMFMMF (SEQ ID NO: 15)
   - MMMMNKLVLAQYYYSCL(SEQ ID NO: 16)
   - MMMMNKLVLAQWWWSCL(SEQ ID NO: 17) or

   (ii) an amino acid sequence comprising at least 14, at least 15 or at least 16 contiguous amino acids from an amino acid sequence according to (i); or
   (iii) an amino acid sequence which has a sequence identity of at least 85%, or of at least 90% to an amino acid sequence according to (i) and/or (ii).
12. The conjugate of any one of embodiments 1-11, wherein the linker (b) is present.
13. The conjugate of any one of embodiments 1-12, wherein the linker (b) is a peptide having a length of at least 2 amino acids, particularly about 2 to about 50 amino acids or about 6 to about 10 amino acids.
14. The conjugate of any one of embodiments 1-13, wherein the polypeptide linker (b) consists of amino acids selected from G, S, A, D, E, I, L, V, K, N, Q, H, R, P, T, M, and C, particularly from G and S.
15. The conjugate of any one of embodiments 1-14, wherein the targeting moiety (c) is capable of specifically targeting the conjugate to a transcriptional condensate.
16. The conjugate of any one of embodiments 1-15, wherein the targeting moiety (c) is capable of specifically targeting the conjugate to a condensate caused by, associated with or accompanied by a pathological condition.
17. The conjugate of embodiment 16, wherein the condensate is a cancer-associated condensate or a pathogen-associated condensate.
18. The conjugate of any one of embodiments 1-17, wherein the targeting moiety (c) (i) is a condensate-forming polypeptide.
19. The conjugate of any one of embodiments 1-17, wherein the targeting moiety (c) (ii) is an immunoglobulin molecule specifically recognizing a component of a biomolecular condensate.
20. The conjugate of embodiment 19, wherein the targeting moiety(c) (ii) is a single-chain immunoglobulin molecule, particularly a VHH antibody.
21. The conjugate of any one of embodiments 1-17, wherein the targeting moiety (c) (iii) is a cell-penetrable peptide that accumulates in the nucleolus.
22. A nucleic acid molecule encoding the conjugate of any one of embodiments 1-21.
23. The nucleic acid molecule of embodiment 22 in operative linkage with an expression control sequence.
24. A vector comprising the nucleic acid molecule of embodiment 22 or 23.
25. A recombinant cell transformed or transfected with the nucleic acid molecule of embodiment 22 or 23 or the vector of embodiment 24.
26. A pharmaceutical composition comprising the conjugate of any one of embodiments 1-21, the nucleic acid molecule of embodiment 22 or 23 or the vector of embodiment 24 as an active agent and a pharmaceutically acceptable carrier.
27. The conjugate of any one of embodiments 1-21, the nucleic acid molecule of embodiment 22 or 23, the vector of embodiment 24 or the pharmaceutical composition of embodiment 26 for use as a medicament.
28. The conjugate of any one of embodiments 1-21, the nucleic acid molecule of embodiment 22 or 23, the vector of embodiment 24 or the pharmaceutical composition of embodiment 26 for the use of embodiment 27 in human medicine.
29. The conjugate of any one of embodiments 1-21, the nucleic acid molecule of embodiment 22 or 23, the vector of embodiment 24 or the pharmaceutical composition of embodiment 26 for use in the treatment of a disorder associated with the presence or with an increased amount of a pathological biomolecular condensate.
30. The conjugate of any one of embodiments 1-21, the nucleic acid molecule of embodiment 22 or 23, the vector of embodiment 24 or the pharmaceutical composition of embodiment 26 for use in the treatment of a hyperproliferative disease, particularly cancer.
31. The conjugate of any one of embodiments 1-21, the nucleic acid molecule of embodiment 22 or 23, the vector of embodiment 24 or the pharmaceutical composition of embodiment 26 for use in the treatment of an autoimmune disease..
32. The conjugate of any one of embodiments 1-21, the nucleic acid molecule of embodiment 22 or 23, the vector of embodiment 24 or the pharmaceutical composition of embodiment 26 for use in the treatment of a disease caused by an infection with a pathogen.
33. In vitro use of the conjugate of any one of embodiments 1-21, the nucleic acid molecule of embodiment 22 or 23 or the vector of embodiment 24 for arresting the dynamics of a mammalian cell.
34. A kit comprising the conjugate of any one of embodiments 1-21, the nucleic acid molecule of embodiment 22 or 23 or the vector of embodiment 24 optionally further comprising an instruction manual for use.
35. A method of treating a disorder associated with the presence or with an increased amount of a pathological biomolecular condensate, comprising administering to a subject in thereof an effective amount of the conjugate of any one of embodiments 1-21, the nucleic acid molecule of embodiment 22 or 23, the vector of embodiment 24 or the pharmaceutical composition of embodiment 26.
36. The method of embodiment 35, wherein the subject is a human patient.

### DETAILED DESCRIPTION

The present inventors have developed a peptide designated herein as the "killswitch", which is capable of immobilizing a biomolecule that is a component of a biomolecular condensate and thereby disrupting the function of the biomolecular condensate. When conjugated to a heterologous targeting moiety, the killswitch can be used as a universal approach to probe endogenous condensates, and demonstrate direct links between condensate microenvironments and function in cells.

The inventors had previously identified a pathogenic frameshift variant of the nuclear protein HMGB1 ¹ that generates a HMGB1 protein with new C-terminal tail which leads to the mispartitioning of the mutant HMGB1 protein into the nucleolus and arrests the dynamics of the nucleolus, ultimately leading to cell death. The nucleolar arrest appears to be caused by the C-terminal 17 amino acids¹.

Surprisingly, the inventors found that the 17-amino acid micropeptide sequence, hereafter referred to as the original killswitch, could be engineered into a generally applicable tool to alter material properties of specific biomolecular condensates, and as a consequence, to probe condensate microenvironments and functions in living cells.

In the scientific literature a "biomolecular condensate" is understood as a an entity that is not bound by a membrane that concentrates specific types of biomolecules as described by Mittag and Pappu (Mol Cell. 2022 Jun 16;82(12):2201-2214. doi: 10.1016/j.molcel.2022.05.018) and Banani et al. (Nat Rev Mol Cell Biol. 2017 May;18(5):285-298. doi: 10.1038/nrm.2017.7., 2017), the contents of which are herein incorporated by reference.

In embodiments, the term "biomolecular condensate" or "condensate" refers to a membraneless assembly or cluster of biomolecules including proteins and nucleic acids. In certain embodiments, the biomolecular condensate is formed by phase separation. In further embodiments, the biomolecular condensate is not formed by phase separation. In certain embodiments, the biomolecular condensate comprises a network of its components spanning the volume of the condensate.

In embodiments, the term "biomolecular condensate" refers to any type of cytoplasmic or nuclear condensate, including but not limited to a transcriptional condensate, a chromatin condensate, a nucleolar condensate, a heterochromatin condensate, a transcription factor cluster, a splicing factor condensate, a nuclear speckle, an oncoprotein condensates, an RNA polymerase II condensate, and a viral protein condensate, among others.

In the present application, the term "killswitch" particularly refers to a non-natural hydrophobic peptide comprising aromatic amino acid residues with an ability to self-associate. In embodiments, the killswitch peptide has no homology to human proteins. When recruited to endogenous and disease-specific condensates in human cells, the killswitch is capable of arresting the dynamics of the condensate-forming biomolecules, e.g., proteins.

Without wishing to be bound by theory, the killswitch might essentially work as a "glue" that makes the targeted components, e.g. proteins, stick together within the biomolecular condensate. As a result, the biomolecular condensate, which is typically very dynamic becomes rigid, as components, e.g., proteins, on the inside are immobilized.

An important element of the biological effect is that there is a sufficient amount of the condensate forming protein that needs to be bound by the killswitch in order to have an effect on the condensate. The inventors have found an unexpected, synergistic effect of the targeting above a threshold concentration of the killswitch. This was very unexpected.

Targeting the killswitch to fusion oncoprotein condensates inhibited the dynamics of effector proteins in the condensates, altered condensate composition, and inhibited proliferation of condensate-driven leukemia cells. In adenoviral nuclear condensates, the killswitch inhibited partitioning of capsid protein into condensates, and suppressed viral particle assembly.

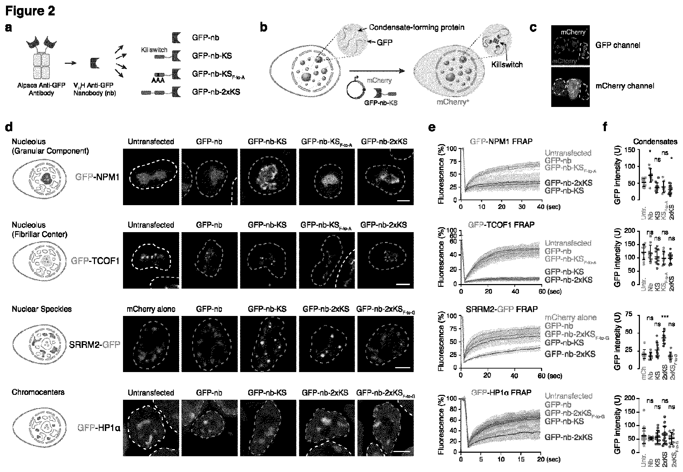

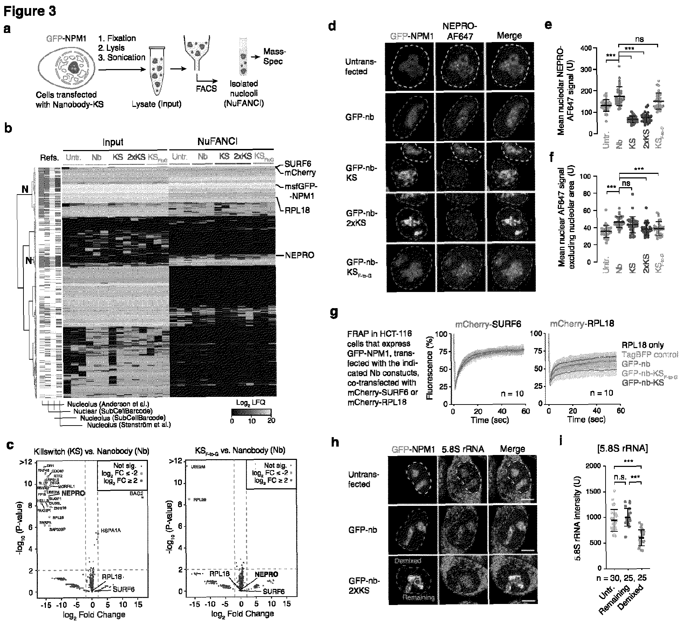

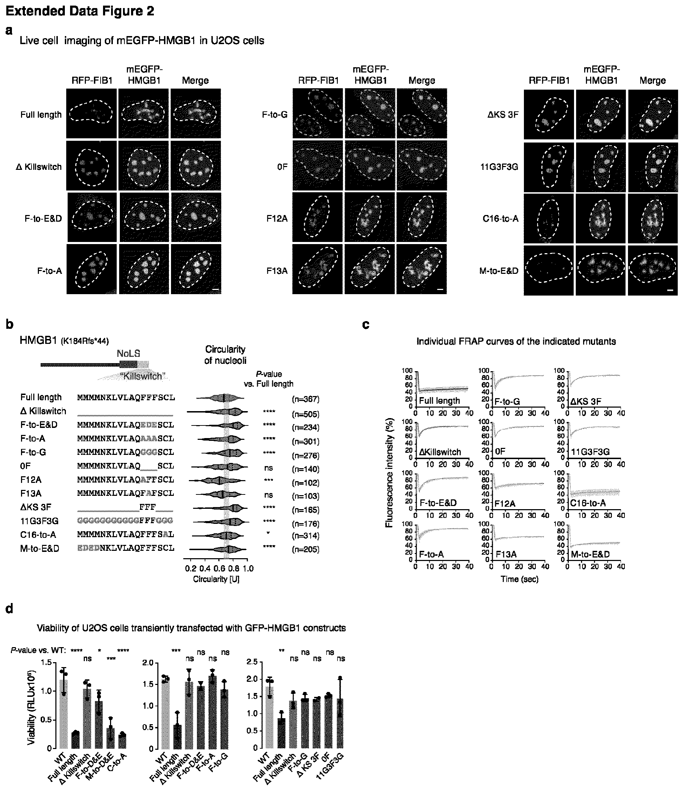

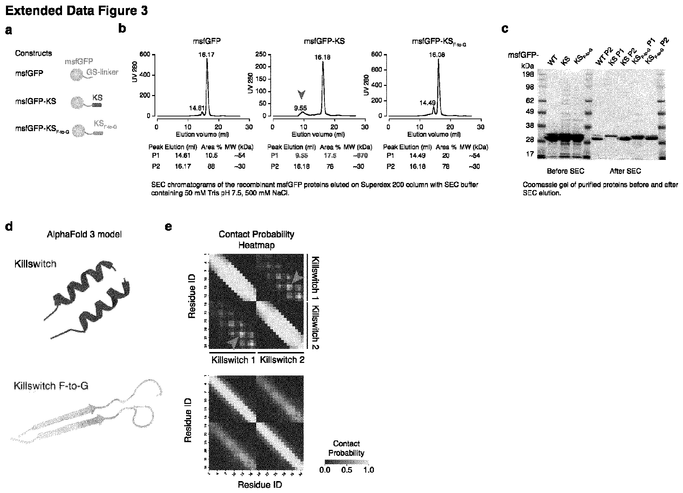

The inventors have targeted the killswitch to four different endogenous condensates in human (mammalian) cells, and show that the killswitch efficiently immobilizes the targeted condensate-forming protein Targeting the killswitch to the nucleolar protein NPM1 altered nucleolar composition, and inhibited the dynamics of a ribosomal protein within a nucleolus. **(****Fig. 2****).** The result of the immobilization is a loss-of-function of the condensates for the nucleolus **(****Fig. 3****).**

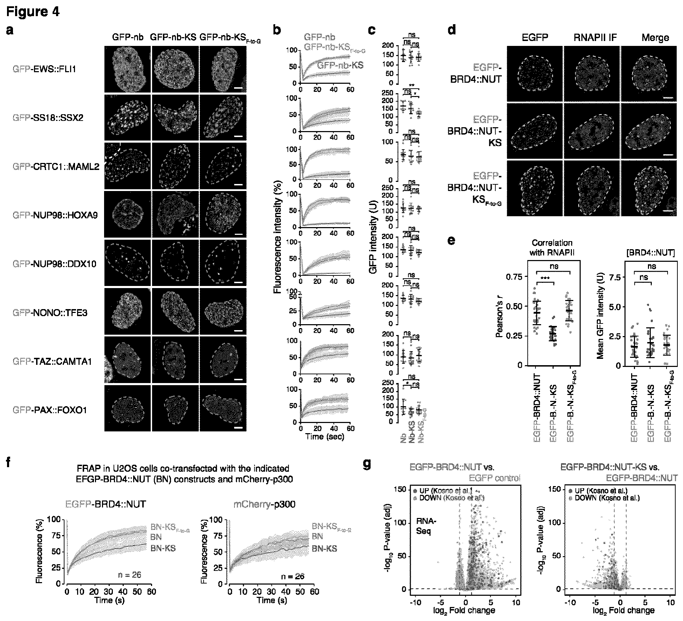

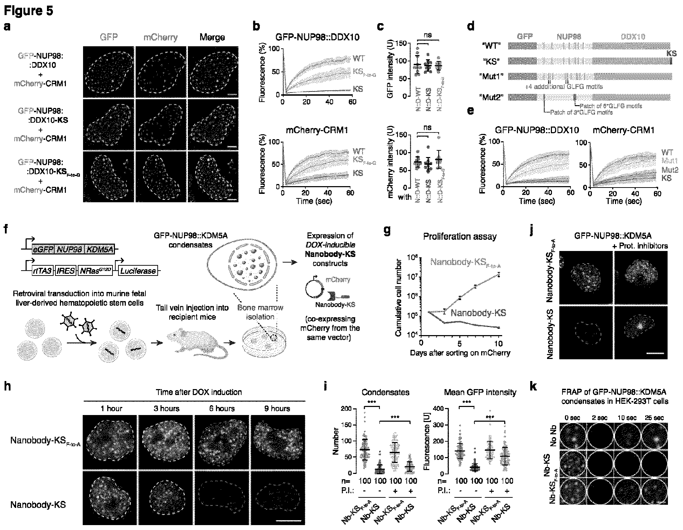

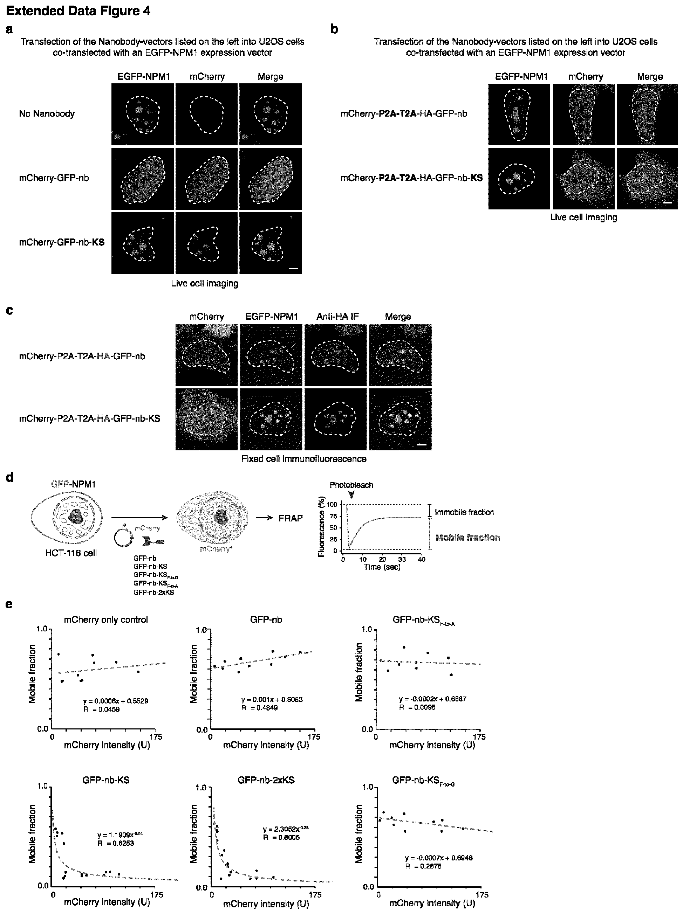

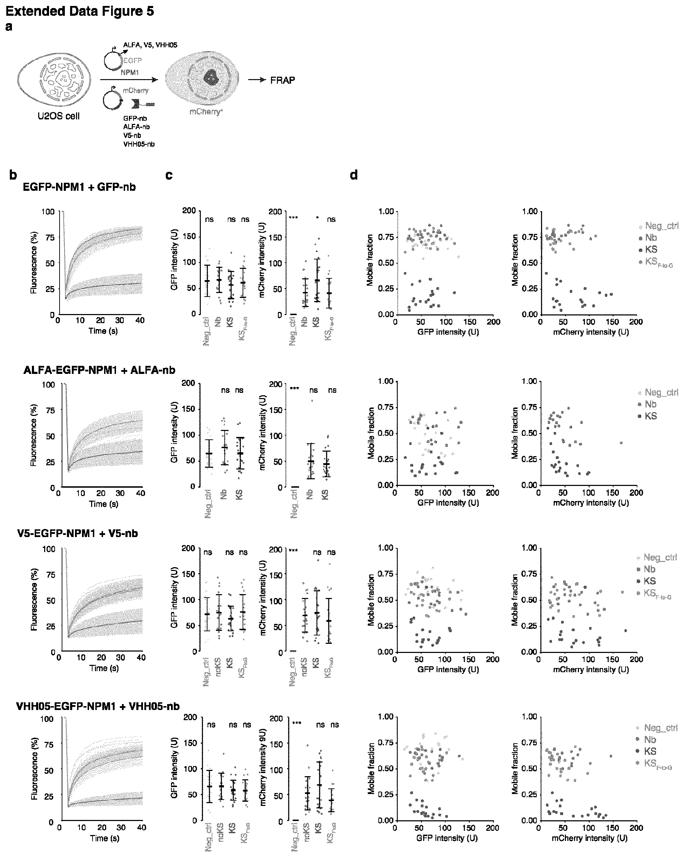

The inventors have also targeted the killswitch to several cancer-specific condensates that are formed by fusion-oncoproteins. In all about 10 cases, the killswitch effected an immobilization of the fusion oncoprotein in the condensates **(****Fig. 4****).** In a murine ex vivo leukemia model, the killswitch effected a complete inhibition of the proliferation of leukemia cells **(****Fig. 5****).**

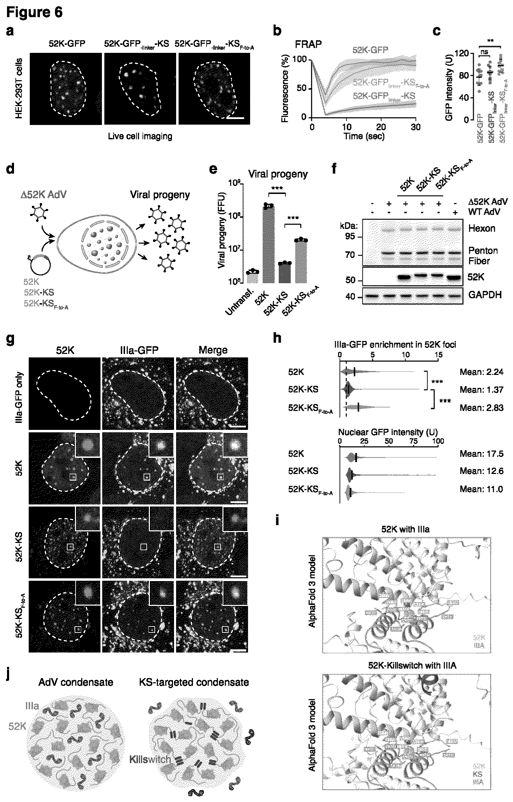

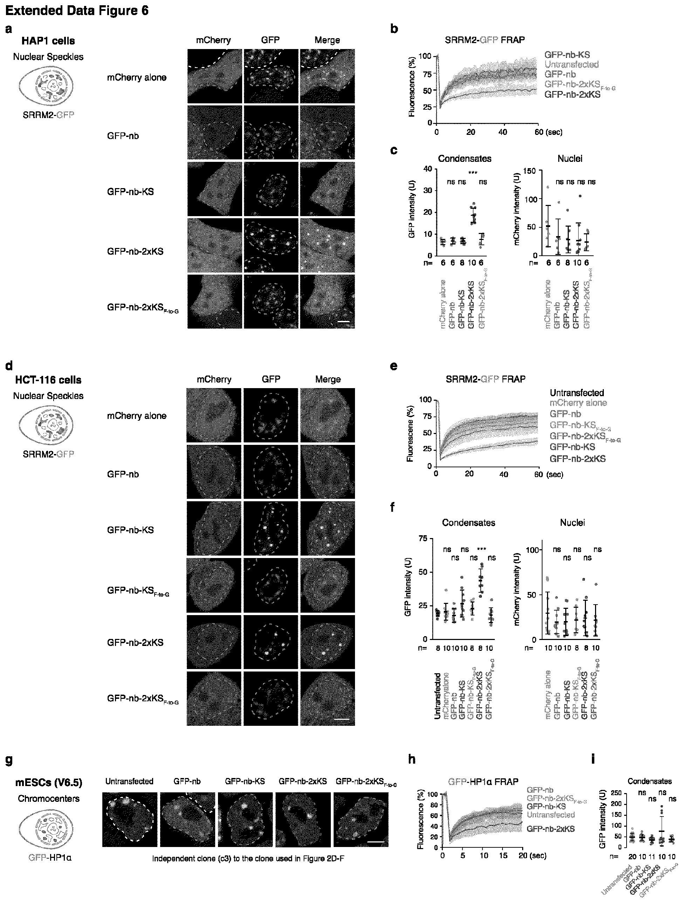

The inventors have also targeted the killswitch to adenoviral condensates, and found that the killswitch is sufficient to inhibit the formation of infectious viral particles in human cells **(****Fig. 6****).**

These results suggest that the microenvironment within biomolecular condensate has an important contribution to non-stoichiometric enrichment and the dynamics of effector proteins. The killswitch peptide is a widely applicable tool to alter the material properties of endogenous condensates, and as a consequence, to probe functions of condensates linked to diverse physiological and pathological processes in living systems.

The present invention relates to a conjugate comprising a killswitch peptide (a), optionally a linker (b); and a targeting moiety (c) capable of targeting the conjugate to a biomolecular condensate.

In embodiments, the conjugate comprises a single killswitch peptide (a). In further embodiments, the conjugate may comprise several, e.g., 2, 3 or 4, killswitch peptides (a). In those embodiments, the killswitch peptides (a) may be sequentially ordered one after the other or they may be located at different positions of the conjugate.

In the conjugate, a killswitch peptide (a) may be attached to a targeting moiety (c) either directly or via a linker (b). Typically the attachment comprises a covalent bond.

In embodiments, the conjugate is a fusion polypeptide wherein the targeting moiety (c) is a polypeptide and the at least one killswitch peptide (a) is covalently bound thereto either directly or via the linker (b). In specific embodiments, the fusion polypeptide is a genetic fusion polypeptide, e.g., comprising a single polypeptide chain.

When the conjugate is a fusion protein, the targeting moiety (c) may be positioned at the N-terminus and the at least one killswitch peptide (a) at the C-terminus optionally connected by a peptide linker (b). In other embodiments, the at least one killswitch peptide (a) may be positioned at the N-terminus and the targeting moiety (c) at the C-terminus optionally connected by a peptide linker (b).

In further embodiments, the targeting moiety (c) is a polypeptide and the at least one killswitch peptide (a) is covalently bound to an amino acid side chain thereof either directly or via the linker (b). The attachment of the killswitch peptide (a) to an amino acid side chain, e.g., an amino, thiol, or carboxy side chain, of the targeting moiety polypeptide may be effected according to techniques known for the manufacture of antibody-drug-conjugates, e.g., by means of click chemistry.

In further specific embodiments, the conjugate comprises a nucleic acid molecule as targeting moiety (c) capable of binding, e.g., hybridizing to a component of the biomolecular conjugate. The at least one killswitch peptide (a) may be attached to the 5' and/or 3' end of the nucleic acid molecule either directly or via the linker (b).

In embodiments, the conjugate consists of a killswitch peptide (a), optionally a linker (b); and a targeting moiety (c) capable of targeting the conjugate to a biomolecular condensate. In further embodiments, the conjugate may comprise an additional moiety , e.g., a labelling group a peptide such as a cyclic peptide, a small organic molecule, etc.

In embodiments, the targeting moiety (c) is different from a HMGB1 polypeptide or a portion thereof, e.g., a portion having a length of at least 5, at least 10, at least 20 or at least 50 amino acids of a HMGB1 polypeptide. In further embodiments, the conjugate is free from HMGB1 amino acid sequences, e.g., having a length of at least 5, at least 10, at least 20 or at least 50 amino acids.

In certain embodiments, the killswitch peptide (a) in the conjugate of the present invention has a length of about 10 to about 100 amino acids, or of about 12 to about 50 amino acids or of about 15 to about 20 amino acids. In particular embodiments, the killswitch peptide has a length of 17 amino acids.

The activity of a killswitch peptide (a) may be determined by its ability to immobilize a biomolecule that is a component of a biomolecular condensate. In certain embodiments, the activity of a killswitch peptide (a) may be determined when expressed as HMGB1 variant, e.g., as HMGB1 variant fused to a GFP tag, by its capability of localizing to the nucleolus of a mammalian cell and causing nucleolar arrest. In particular embodiments, a transgene encoding a GFP-tagged HMGB1 killswitch peptide is transiently expressed in a human osteosarcoma U2OS cell (ATTC HTB-96), and the nucleolar dynamics is quantified as the ratio of mobile versus immobile fractions of GFP-HMGB1 in nucleoli using fluorescence recovery after photobleaching (FRAP). In embodiments, a nucleolar arrest may be characterized by a significant difference in the observed diffusion rate measured by FRAP.

The inventors identified several active variants of the original killswitch peptide (SEQ ID NO: 1). A mutation of the cluster of three phenylalanine residues to negatively charged residues, alanines or glycines was sufficient to abolish activity. Mutation of a single phenylalanine did not lead to a loss of activity, suggesting that at least two phenylalanines are sufficient for the killswitch function. The phenylalanines themselves, however, are not sufficient for the killswitch effect, as mutation of the other 14 residues into glycines, or fusing three phenylalanines to a mutant HMGB1 lacking the killswitch sequence also resulted in a loss of activity. The cysteine and the methionines are not necessary for the killswitch effect.

Shuffling the killswitch peptide sequence further revealed that clustering of the phenylalanine residues was in itself not necessary, but enhanced nucleolar arrest Similar results were observed when the killswitch was recruited to nucleoli via ectopic expression of the nucleolar protein NPM1 genetically fused to the killswitch sequence. These results suggest that phenylalanines drive nucleolar arrest, but require the additional sequence context of the killswitch. Exchange of phenylalanines by other aromatic amino acids, i.e., tyrosine or tryptophan, does not abolish the killswitch effect. Phenylalanines, however, have the strongest effect.

In specific aspect, the killswitch peptide is capable of self-association which can be determined by a droplet assay and size-exclusion chromatography (SEC) in vitro. For example., the self-association capacity may be determined on a GFP-tagged, recombinant killswitch protein by its ability to form droplets in a concentration-dependent manner in the presence of physiological salt, e.g.,125 mM NaCl, and a crowding agent, e.g., 10% PEG 8000). Further, the self-association capacity may be determined by size-exclusion chromatography. In embodiments, self-association may be characterized by a significant difference in the observed saturation concentration in a droplet assay and/or by a significant increase in the portion of the protein eluted as multimer in SEC.

Accordingly in certain embodiments, the killswitch peptide (a) is capable of self-association as measured by a droplet assay, e.g., resulting in an at least 2-fold, an at least 5-fold or an at least 10-fold lower saturation concentration measured in a test tube over the control condition that does not include the peptide.

Further, it was confirmed that the killswitch peptide may be a helix-prone peptide that self-interacts and that phenylalanines and/or other aromatic amino acids are necessary for the killswitch-killswitch interactions.

Accordingly, in certain embodiments, the killswitch peptide (a) comprises an amino acid sequence having at least 2, particularly at least three aromatic amino acid residues selected from F, W and Y. In specific embodiments, the killswitch peptide (a) comprises an amino acid sequence having at least 2, particularly at least three aromatic amino acid residues selected from F. The aromatic amino acids may be clustered, e.g., directly adjacent to each other or dispersed throughout the peptide sequence, e.g., being separated by at least one and up to six non-aromatic amino acids.

In further specific embodiments, the killswitch peptide (a) comprises or consists of
(i) an amino acid sequence selected from:
   - MMMMNKLVLAQFFFSCL (SEQ ID NO: 1)
   - MMMMNKLVLAQAFFSCL(SEQ ID NO: 2)
   - MMMMNKLVLAQFAFSCL(SEQ ID NO: 3)
   - MMMMNKLVLAQFFFSAL(SEQ ID NO: 4)
   - EDEDNKLVLAQFFFSCL (SEQ ID NO: 5)
   - MMMFMNKFLVLAQFSCL (SEQ ID NO: 6)
   - MFMMMNFKLVLAFQSCL (SEQ ID NO: 7)
   - MMFMMNKLFVLAQSCFL (SEQ ID NO: 8)
   - MMMMFFFNKLVLAQSCL (SEQ ID NO: 9)
   - MMMMNKLVFFFLAQSCL (SEQ ID NO: 10)
   - MMLALKSCFVMMQLFFN (SEQ ID NO: 11)
   - AMMNFFQLMFLVKSMLC (SEQ ID NO: 12)
   - FMAFLNVLLKMQCMSFM (SEQ ID NO: 13)
   - CFMLNKFLVMQMSFMLA (SEQ ID NO: 14)
   - CVKLSMNAQFLLMFMMF (SEQ ID NO: 15)
   - MMMMNKLVLAQYYYSCL(SEQ ID NO: 16)
   - MMMMNKLVLAQWWWSCL(SEQ ID NO: 17) or
(ii) an amino acid sequence comprising at least 14, at least 15 or at least 16 contiguous amino acids from an amino acid sequence according to (i); or
(iii) an amino acid sequence which has a sequence identity of at least 85%, or of at least 90% to an amino acid sequence according to (i) and/or (ii).

In certain embodiments, the conjugate of the present invention comprises a linker (b) for connecting the killswitch peptide (a) and the targeting moiety (c). For example, the linker (b) is a peptidic linker having a length of at least 2 amino acids, particularly about 2 to about 50 amino acids, or about 6 to about 10 amino acids. In certain embodiments, the linker may comprise or consist of amino acids selected from G, S, A, D, E, I, L, V, K, N, Q, H, R, P, T, M, and C, particularly from G and S. Specific examples of linkers are shown in SEQ ID NO: 18 and 19.

The targeting moiety (c) of the conjugate can be any moiety capable of entering a biomolecular conjugate, e.g., a polypeptide, a nucleic acid, a carbohydrate, or a lipid, or any combination thereof and targeting a component of the biomolecular condensate. In specific embodiments, the targeting moiety (c) is a polypeptide, which binds to a component of the biomolecular conjugate. In further specific embodiments, the targeting moiety (c) is a nucleic acid, e.g., a DNA, RNA or a nucleic analogue, which binds to a component of the biomolecular conjugate, e.g., by hybridization to a complementary nucleic acid in the biomolecular conjugate.

In certain embodiments, the targeting moiety (c) is a condensate-forming polypeptide, e.g., a phase separation-related protein including phase separation (PS) proteins and membraneless organelles (MLOs) related proteins. A collection of phase separation-related proteins is found in the database PhaSepDB Version 2.1 of June 2022, the content of which is herein incorporated by reference. In embodiments, a condensate-forming polypeptide has a partitioning ratio into a condensate that is statistically significantly higher than 1, e.g., at least about 2, at least about 3 or at least about 5, wherein the partitioning ratio refers to the concentration of the polypeptide within the target condensate over the concentration of the polypeptide outside the condensate.

In certain embodiments, the targeting moiety (c) is a moiety, e.g., a polypeptide that binds to a component of a biomolecular condensate. For example, the targeting moiety may be an immunoglobulin molecule specifically recognizing a component of a biomolecular condensate.

The term immunoglobulin molecule as used herein refers to a polypeptide comprising at least one polypeptide chain and having at least one antigen-binding domain, particularly at least one antigen-binding domain comprising a plurality of Complement-determining regions (CDRs) which recognize a target antigen with high specificity and affinity.

In certain embodiments, the immunoglobulin molecule is an antibody or an antigen-binding fragment thereof. In certain embodiments, the immunoglobulin molecule is a single-chain antibody which consists of a single polypeptide chain. In certain embodiments, a single-chain antibody comprises a heavy chain or a fragment thereof fused on a light chain or a fragment thereof. In particular embodiments, a single-chain antibody comprises a heavy chain only. A single chain antibody comprises at least one antigen-binding domain, e.g., one or two antigen binding domains.

In certain embodiments, the single-chain antibody of the present invention is in a monovalent format, i.e., it has a single binding site for its target.

In more particular embodiments, the immunoglobulin molecule is a single-domain antibody, i.e., an antibody which comprises a heavy chain only and binds to its target antigen with a single antigen-binding domain. In those embodiments, the antigen-binding domain comprises heavy chain variable domain CDRs only, particularly at least a heavy chain variable domain CDR3 and more particularly three heavy chain variable domains CDR1, CDR2 and CDR3. A typical example of a single-domain antibody is a VHH antibody, e.g., a camelid-derived VHH antibody (also known as nanobody). In the following, the term "VHH antibody" encompasses all types of "single-domain antibodies" if not indicated otherwise.

AVHH antibody may be a monovalent heavy chain-only antibody comprising a CDR1 domain, a CDR2 domain and a CDR3 domain linked by framework regions including, but not being limited to, whole VHH antibodies, e.g., native VHH antibodies comprising framework regions derived from camelids, and modified VHH antibodies comprising modified framework regions, VHH antibody fragments and VHH antibody fusion proteins, e.g. a fusion protein with an immunoglobulin or non-immunoglobulin peptide or polypeptide.

The immunoglobulin molecule, e.g., the VHH antibody, of the conjugate may be used to target the killswitch to any biomolecular condensate. In embodiments, the immunoglobulin molecule, e.g., the VHH antibody, may be directed against a labeling group used for selectively labeling of predetermined condensates. For example, the inventors developed a conjugate comprising the killswitch fused to an anti-GFP VHH antibody (GFP-nb) which enables recruitment of the killswitch to any GFP-labelled condensate. In further embodiments, the killswitch may be conjugated to an immunoglobulin, e.g., a VHH antibody, directed against a component of a condensate caused by, associated with or accompanied by a pathological condition. For example, the condensate may be a cancer-associated condensate or a pathogen-associated, e.g., a virus-associated condensate.

In embodiments, the targeting moiety may bind a cancer-specific protein, e.g., a fusion protein resulting from a cancer-associated associated chromosomal rearrangement, e.g., as described in Tripathi et al.; Shirnekhi et al., Jevtic et al, and French et al. ⁵⁴⁻⁵⁷, the contents of which are herein incorporated by reference. For example, the cancer-specific protein may be selected from an NUP98, EWS, SS18, CRTC1, NONO, TAZ, PAX3, and BRD fusion oncoprotein, such as EWS::FLI1, SS18::SSX2, CRTC1::MAML2, NUP98::HOXA9, NUP98::DDX10, NUP98::NSD1, NUP98::KDM5A, NONO::TFE3, TAZ::CAMTA1, PAX3::FOXO1, and BRD4::NUT.

In embodiments, the targeting moiety may bind a pathogen-specific protein, e.g., a viral protein that is a component of a pathogen-specific condensate, e.g., as described in Glon et al., Biomolecular condensates with liquid properties formed during viral infections, Microbes and Infection 26 (8), 2024,105402, https://doi.org/10.1016/j.micinf.2024.105402, the content of which is herein incorporated by reference. For example, the pathogen-specific protein may be an adenoviral protein, e.g., the adenoviral 52K protein.

In further embodiments, the targeting moiety is a cell-penetrable peptide that accumulates in the nucleolus. In embodiments, the cell-penetrable peptide is a poly-cationic peptide capable of crossing the plasma membrane directly or by endocytosis, e.g., as described in Baoum AA, Berkland C. Calcium condensation of DNA complexed with cell-penetrating peptides offers efficient, noncytotoxic gene delivery. J Pharm Sci. 2011 100 (5):1637-42. doi: 10.1002/jps.22407, the content of which is herein incorporated by reference.

A further aspect of the present invention relates to a nucleic acid molecule encoding the conjugate. The nucleic acid molecule may be a single- or double-stranded nucleic acid molecule, e.g., a DNAor RNA molecule. In certain embodiments, the recombinant nucleic acid molecule is in operative linkage with an expression control sequence, e.g., an expression control sequence allowing expression in prokaryotic or eukaryotic cell, particularly in a mammalian cell including a human cell.

A further aspect of the present invention relates to a vector, comprising said nucleic acid molecule as indicated above in operative linkage with an expression control sequence, particularly with a heterologous expression control sequence.

In certain embodiments, the vector is a non-viral vector, e.g. a plasmid, or a viral vector for therapeutic use, e.g., a vector adapted for introduction and propagation in a eukaryotic cell, e.g., mammalian cell.

In further embodiments, the vector is a non-viral or viral vector for producing the conjugate in a suitable recombinant host cell.

A further aspect of the present invention relates to a recombinant cell comprising a nucleic acid molecule or a vector as described above. The nucleic acid molecule or the vector may be present extrachromosomally or integrated into the cell genome. The recombinant cell may be obtained by introducing a nucleic acid molecule or vector as described above into the cell by transformation, transfection, or infection according to known procedures.

The recombinant cell may be a prokaryotic host cell or a eukaryotic host cell, e.g., an insect or mammalian cell, particularly a human cell.

A further aspect of the present invention relates to a pharmaceutical composition comprising the conjugate, the nucleic acid molecule, or the vector as an active agent. The pharmaceutical composition further comprises a pharmaceutically acceptable carrier as known in the art. In embodiments, the pharmaceutical composition is configured for delivering a polypeptide, i.e., the conjugate as such, or for delivering a nucleic acid molecule or vector encoding the conjugate which is then expressed within a target cell or target organism. The delivery methods include but are not limited to delivery by viral vectors, e.g., retroviral vectors, lentiviral vectors, or adeno-associated viral vectors, or by non-viral vectors, e.g., lipid particles such as nucleic acid-containing lipid nanoparticles (LNPs) or protein containing LNPs.

A further aspect of the present invention relates to the conjugate, the nucleic acid molecule or for use as a medicament, e.g., as a medicament for use in human medicine.

In embodiments, the medicament is used in the treatment of a disorder associated with the presence or with an increased amount of a pathological biomolecular condensate, e.g., in the treatment of a hyperproliferative disease, particularly cancer, in the treatment of an autoimmune disease, or in the treatment of a disease caused by an infection with a pathogen.

Specific examples of disorders include cancer associated with the occurrence of fusion oncoproteins as herein described above, a viral infection, e.g., an adenoviral infection, ...

A further aspect of the invention relates to a method of treating a disorder associated with the presence or with an increased amount of a pathological biomolecular condensate, comprising administering to a subject in thereof, e.g., a human subject, an effective amount of the conjugate, the nucleic acid molecule, the vector or the pharmaceutical composition.

Still a further aspect of the invention relates to the in vitro use of the conjugate, the nucleic acid molecule or the vector for arresting the dynamics of a mammalian cell. This aspect is particularly relevant for research applications.

Still a further aspect of the invention relates to a kit, e.g. a kit of reagents, comprising the conjugate, the nucleic acid molecule or the vector optionally further comprising an instruction manual for use.

Further, the present invention shall be described in more detail by the following Figures and Examples.

### Figure Legends

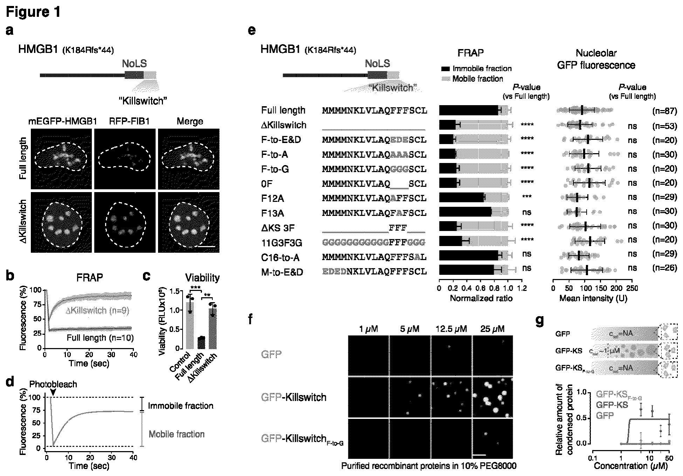

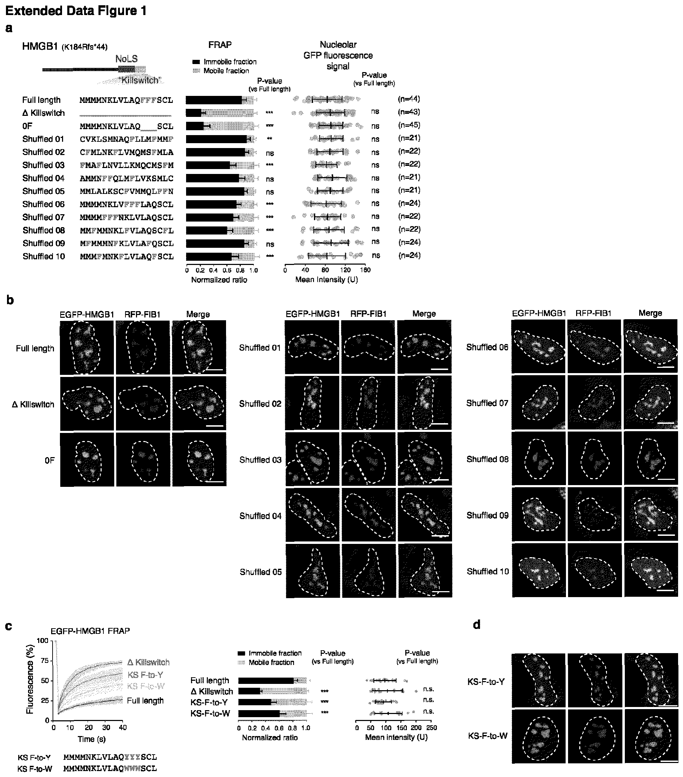

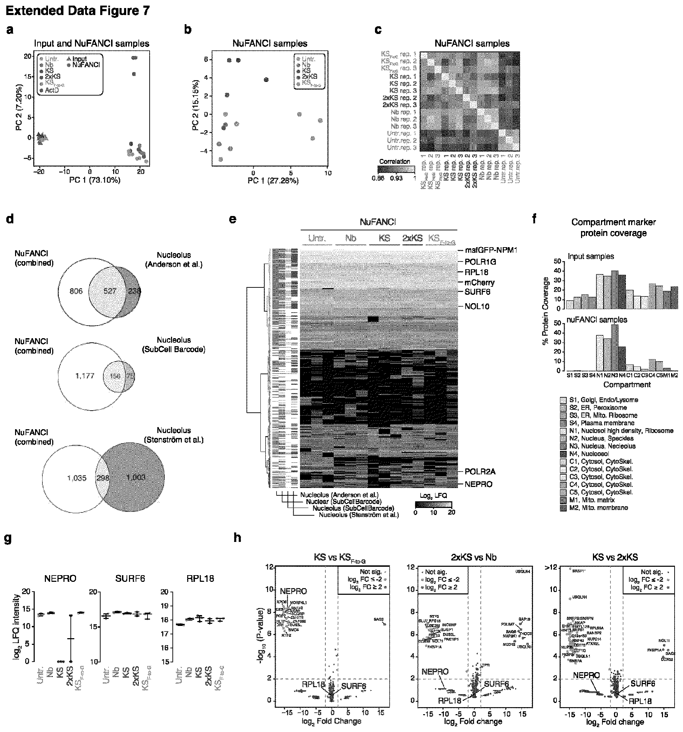

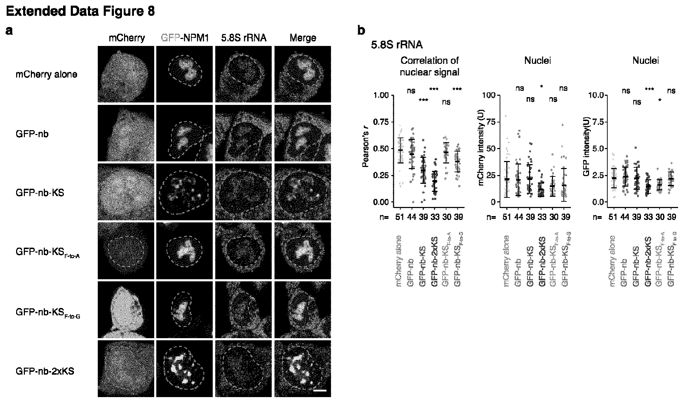

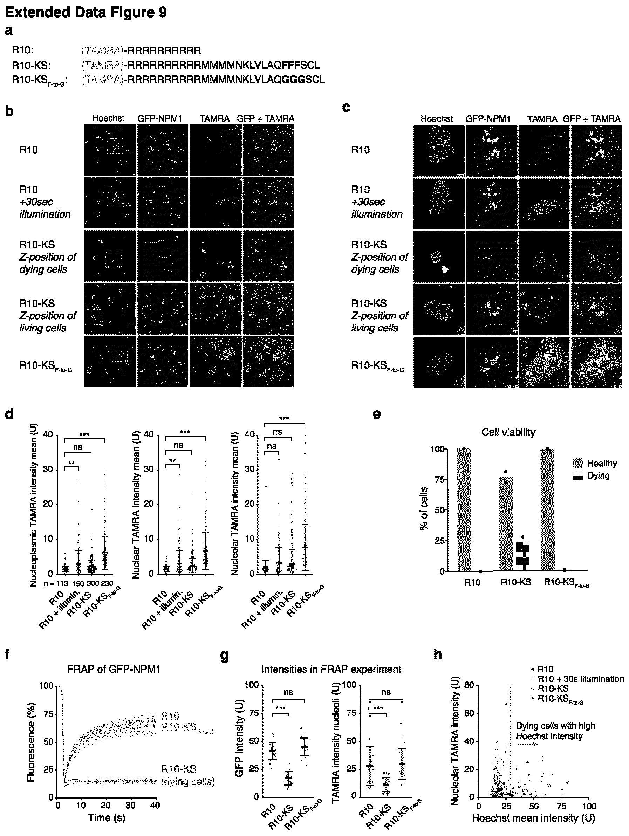

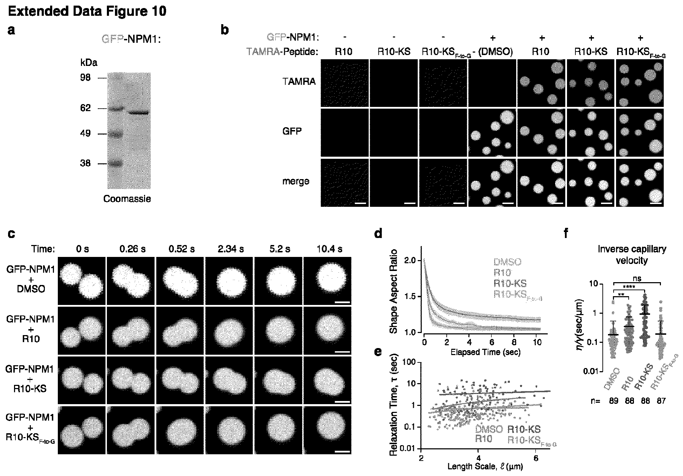

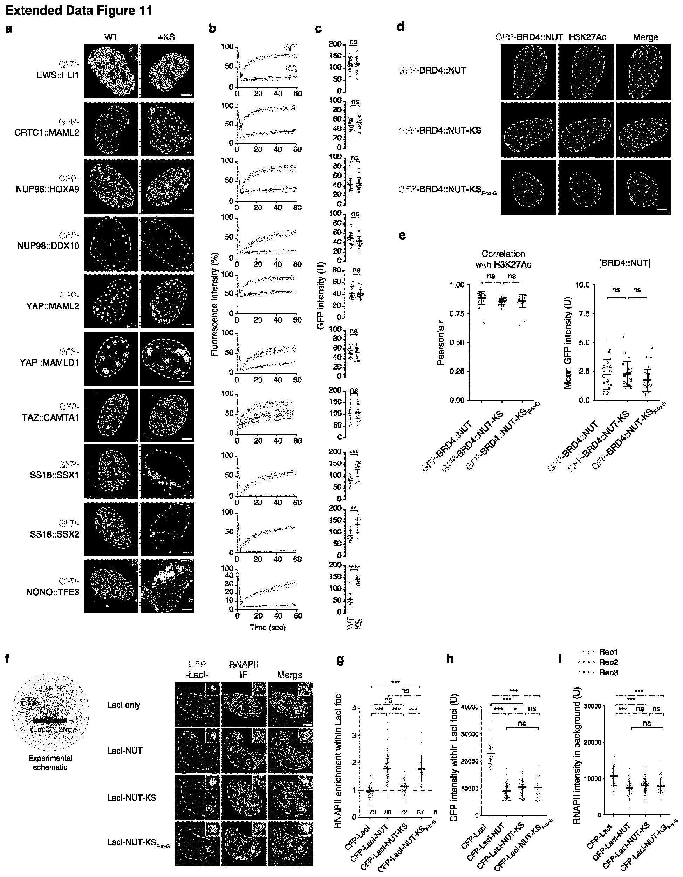

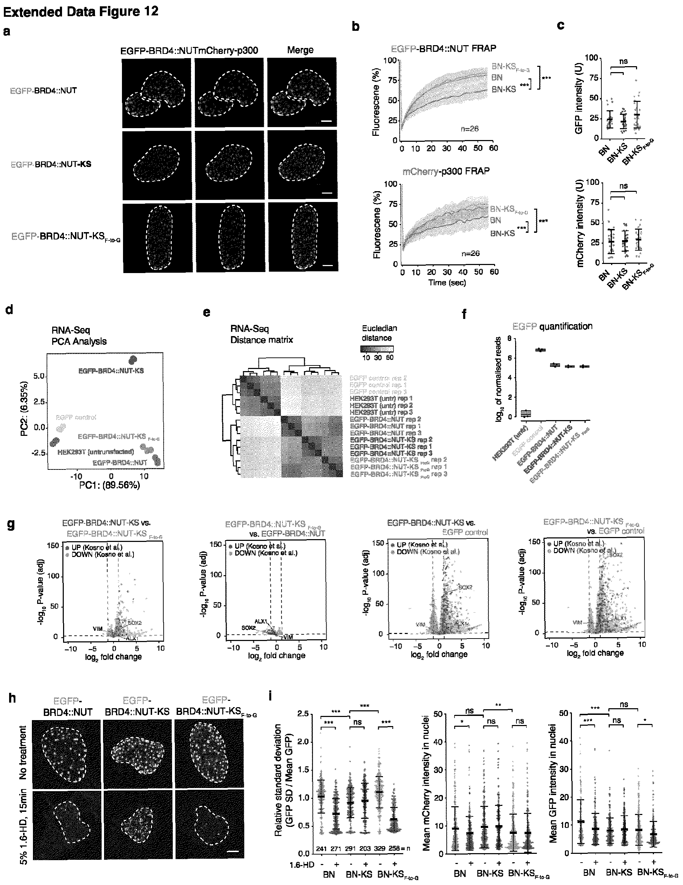

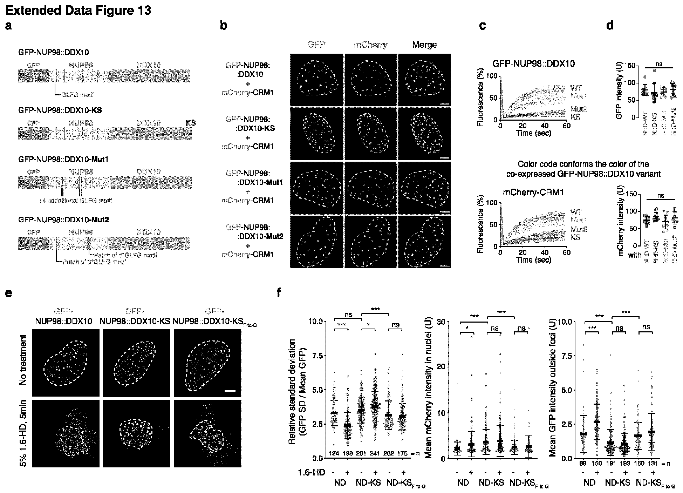

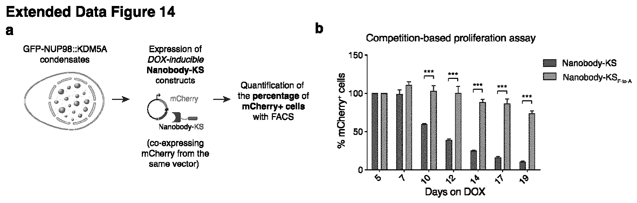

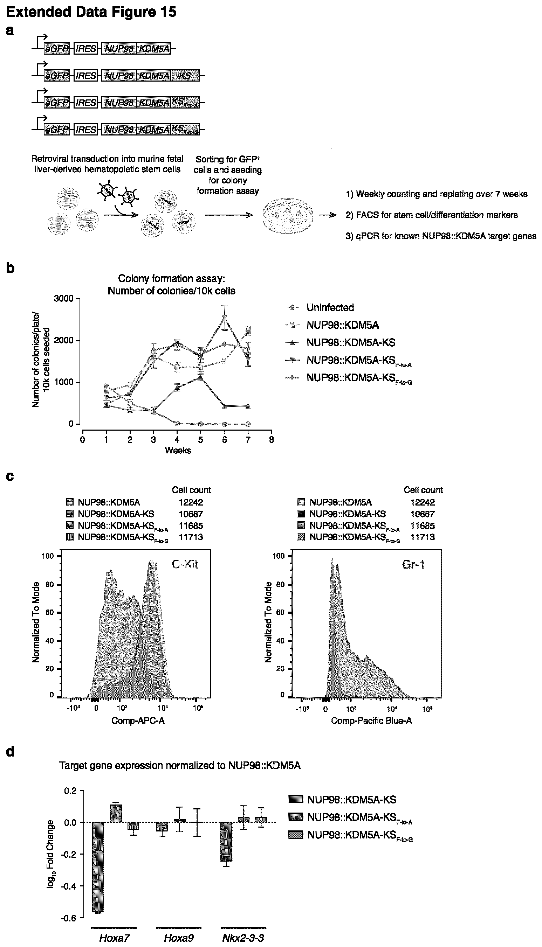

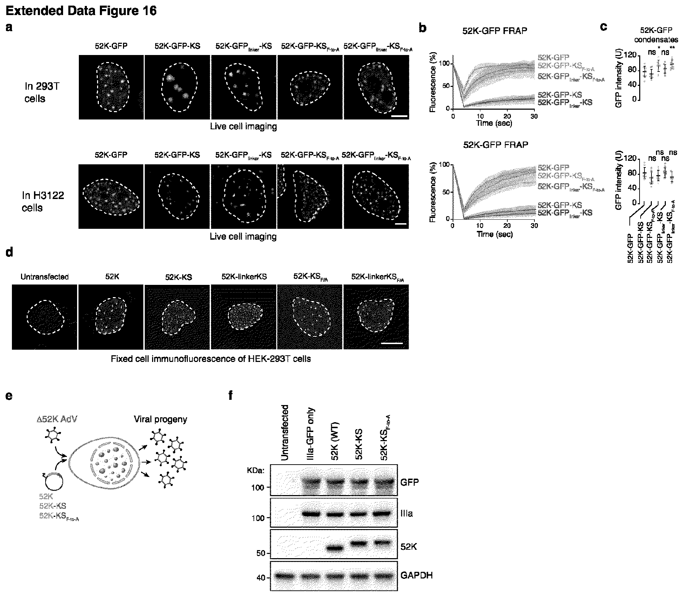

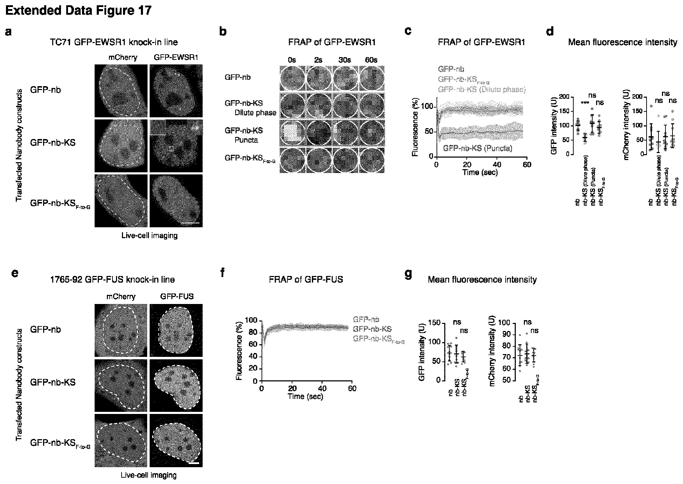

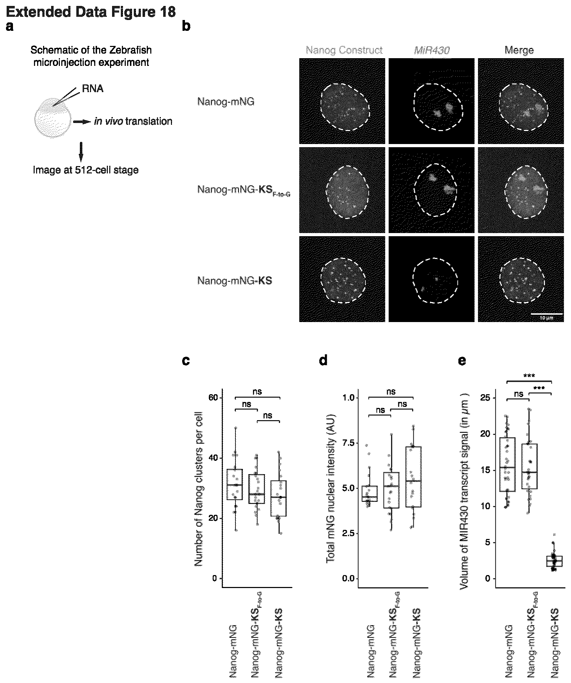

**Figure 1****. The killswitch arrests the dynamics of nucleolar condensates.**
   a. (top) Schematic depiction of the construct fsHMGB1. NoLS: nucleolar localization sequence. (bottom) Live cell fluorescence microscopy images of U2OS cells expressing ectopic mEGFP-HMGB1 proteins and RFP-FIB1. The contour of the cell nucleus is highlighted with a dashed white line. Scale bar: 10 µm.
   **b.** Fluorescence recovery after photobleaching (FRAP) of mEGFP-HMGB1 in nucleoli.
   **c.** Cell viability of U2OS cell expressing mEGFP-HMGB1 proteins. n = 3 biological replicates. P-values are from Tukey's post-hoc test versus fsHMGB1-full length after one-way ANOVA. **:*P*<10⁻²; ***:*P*<10⁻³. RLU: relative light unit.
   **d.** Scheme of the FRAP assay. The proportion of the signal recovered in the experimental time window is a proxy for the mobile fraction of protein in the bleached condensate. The proportion of the signal that does not recover in the experimental time window is a proxy for the immobile fraction of protein in the bleached condensate.
   **e.** (left) Amino acid sequences of killswitch variants within the tested mEGFP-HMGB1 proteins. (middle) Quantification of the mobile and immobile fractions of mEGFP-HMGB1 proteins in nucleoli. (right) Mean GFP fluorescence of bleached nucleoli. "n" is the number of nucleoli tested, from at least two independent experiment series. P-values are from Dunnett's multiple comparisons test versus fsHMGB1-full length after ANOVA. ***:*P*<10⁻³; ****:*P*<10⁻⁴.
   **f.** *In vitro* droplet formation by purified, recombinant GFP-tagged killswitch peptides, at the indicated concentrations. Scale bar: 5 µm. "KS": killswitch.
   **g.** Quantification of the relative amount of protein in droplets.
**Figure 2****. The killswitch arrests dynamics of endogenous cellular condensates.**
   **a.** Schematic depiction of Nanobody-Killswitch protein constructs.
   **b.** Schematic depiction of the Nanobody-based recruitment system in live cells.
   **c.** Example of the nuclear highlighting strategy. The nuclear contour of mCherry-expressing cells is highlighted with a magenta dashed line. The nuclear contour of untransfected or mCherry-negative cells is highlighted with a white dashed line throughout the figure.
   **d.** Live cell fluorescence microscopy images of GFP-NPM1, GFP-TCOF1, SRRM2-GFP, GFP-HP1α-GFP expressing cells transfected with the indicated anti-GFP-Nanobody (GFP-nb) or Nanobody-Killswitch (GFP-nb-KS) constructs. The GFP in each cell line is knocked into the endogenous gene locus.
   **e.** FRAP of the GFP in the condensates shown in panel D. n≥8 in all cases.
   **f.** Control quantification of GFP intensity in the bleached condensates. n≥8 in all cases. P-values are from Tukey's post-hoc test versus untransfected cells after one-way ANOVA. *:*P*<0.05; ***:*P*<10⁻³; ns: not significant.
**Figure 3****. The killswitch perturbs composition, component dynamics, and function of nucleolus.**
   **a.** Schematic depiction of Nuclear Fluorescence-Activated Non-membrane Condensates Isolation procedure (NuFANCI).
   **b.** Heatmap showing the protein expression profiles of NuFANCI-isolated nucleoli and their respective input samples. Protein markers used in nucleolus isolation, mCherry and msfGFP-NPM1, and protein markers tested by IF are annotated on the right. Proteins have also been annotated based on the proteome of isolated nucleoli from previous proteomics data. "N" highlights the two clusters of nucleolar proteins.
   **c.** Volcano plots of NuFANCI GFP-nb-KS targeted nucleoli (left) GFP-nb- KS_{F-to-G} targeted nucleoli (right) proteomes compared with the anti-GFP-nanobody control, tested by t-test. Non-significant proteins are shaded in gray, whereas enriched and depleted are shaded in red and blue, respectively. Protein markers tested by IF are annotated in bold.
   **d.** Fixed cell immunofluorescence images of GFP-NPM1-expressing cells, with conditions of untransfected, transfected with GFP-nb, GFP-nb-KS, GFP-nb-2xKS, or GFP-nb-KS_{F-to-G} construct. Scalebar: 5 µm.
   **e.** Quantification of mean NEPRO fluorescence intensity in the nucleolar area called by GFP-NPM1 signal in cells exemplified in **d.** *P*-values from Dunnett's T3 multiple comparisons test versus GFP-nb condition after one-way ANOVA. ****:*P*<10⁻⁴
   **f.** Quantification of mean NEPRO fluorescence intensity in the nucleus excluding nucleolar area in cells exemplified in **d.** *P*-values from Dunnett's T3 multiple comparisons test versus GFP-nb condition after one-way ANOVA. ****:*P*<10⁻⁴
   **g.** FRAP of mCherry-SURF6 and mCherry-RPL18 in HCT-116 cells expressing GFP-NPM1 from the endogenous locus. Cells were co-transfected with mCherry-SURF6 or -RPL18 together with indicated Nanobody constructs. TagBFP replaced the mCherry reporter in Nanobody expression constructs. Relative mCherry-RPL18 intensity shows significant difference in GFP-nb-KS versus RPL18 only control in 28 out or 30 timepoints measured (P<0.05, Tukey's post-hoc test after one-way ANOVA).
   **h.** Fixed cell immunofluorescence images of 5.87s rRNA in GFP-NPM1-expressing cells, transfected with either GFP-nb or GFP-nb-2xKS construct. Scalebar: 5µm.
   **i.** Quantification of 5.8 S rRNA mean fluorescence intensities in the "demixed" and "remaining portions" of nucleoli in GFP-nb-2xKS-expressing cells, exemplified in h. The mean fluorescence intensity in untransfected cells is shown as the control comparison. P-values are from Tukey's post-hoc test after one-way ANOVA. ***:*P*<10⁻³
**Figure 4****. The killswitch inhibits the dynamics and functions of oncoprotein condensates.**
   **a.** Live cell fluorescence microscopy images of U2OS cells expressing the indicated fusion oncoproteins tagged with GFP. One cell nucleus is shown in each image. The cells were co-transfected with GFP-nb, GFP-nb-KS, or GFP-nb-KS_{F-to-G} construct. Scale bar: 5µm.
   **b.** FRAP of GFP-fusion-oncoprotein condensates exemplified in panel a. n=10 in all cases.
   **c.** Control quantification of GFP intensity in the bleached condensates. n=10 in all cases. P-values are from Tukey's post-hoc test after one-way ANOVA. *:*P*<0.05; **:*P*<10⁻²; ns: not significant. Mean is shown as a black line and whiskers represent standard deviation.
   **d.** Fixed cell immunofluorescence images of RNAPII in cells expressing GFP-BRD4::NUT, GFP-BRD4::NUT-KS, or GFP-BRD4::NUT-KS_{F-to-G}. Scale bar: 5 µm.
   **e.** Pearson correlation coefficients and mean GFP intensity of cells shown in panel d. n_{(GFP-BRD4-NUT)}=34, n_{(GFP-BRD4-NUT-KS)} = 28, n_{(GFP-BRD4-NUT-KS-F-to-G)} =29. P-values are from Tukey's post-hoc test versus GFP-BRD4::NUT after one-way ANOVA. ***:*P*<10⁻³; ns: not significant.
   **f.** FRAP of EGFP-BRD4::NUT (left) and of mCherry-p300 (right) in EGFP-BRD4::NUT condensates exemplified in Extended Data Fig.14a. n=26 in all cases.
   **g.** Differential expression analysis of EGFP-BRD4::NUT fused with Killswitch compared to wild-type (left) and wild-type EGFP-BRD4::NUT compared to EGFP control in HEK293T cells measured by RNA-seq. The BRD4-NUT target genes described in a previous report ² were used to color the genes. Referred upregulated genes are colored in red and downregulated in blue. Note that most of the referenced upregulated (red) genes showed significant downregulation in Killswitch samples.
**Figure 5****. The killswitch inhibits component dynamics and function of NUP98-fusion condensates.**
   **a.** Live cell fluorescence microscopy images of U2OS cells expressing GFP-NUP98::DDX10 constructs each co-transfected with mCherry-CRM1. Scale bar: 5µm.
   **b.** FRAP of GFP-NUP98::DDX10 (top), and of mCherry-CRM1 (bottom) in GFP-NUP98::DDX10 condensates in U2OS cells exemplified in **a.** n=10 in all cases.
   **c.** (top) Control quantification of GFP intensity in the bleached condensates. (bottom) Control quantification of mCherry intensity in the bleached condensates. *P*-values are from Tukey's post-hoc test after one-way ANOVA. n=10 in all cases.
   **d.** Schematic representation of the NUP98::DDX10 control and mutagenesis constructs. The red lines in the NUP98 portion represent GLFG motifs. The schematics are scaled to the actual amino acid positions.
   **e.** FRAP of GFP-NUP98::DDX10 (left), and of mCherry-CRM1 (right) in GFP-NUP98::DDX10 condensates in U2OS cells. n=10 in all cases.
   **f.** Schematic representation of the constructs and the workflow for the generation of the GFP-NUP98::KDM5A-expressing AML model.
   **g.** Growth curve of GFP-NUP98::KDM5A-expressing primary murine AML cells, transduced with either Nanobody-KS, or Nanobody-KS_{F-to-A} constructs, co-expressing mCherry from the same vector. The cumulative cell number was recorded after sorting mCherry-positive cells.
   **h.** Fluorescence microscopy images of GFP-NUP98::KDM5A condensates in primary murine AML cells expressing Doxycycline-inducible Nanobody-KS_{F-to-A} or Nanobody-KS, after the indicated hours of Dox-induction. The nuclear contour is highlighted with a white dashed line when mCherry (expressed from the same viral vector as the Nanobody-constructs) is not yet detectable, and is highlighted with a magenta dashed line when mCherry is detectable. Additional wide-field images are shown in **Extended Data** **Fig. 15****.** Scale bar: 5µm.
   **i.** Quantification of the number of condensates and mean GFP intensity in GFP-NUP98::KDM5A-driven primary murine AML cells expressing the indicated Nanobody constructs. P-values are from unpaired two-tailed t-tests. n=100 in all cases ***:*P*<10⁻³. P.I.: Proteasome inhibitors.
   **j.** Proteasome inhibition partially rescues GFP-NUP98::KDM5A expression in cells expressing the Nanobody-KS. Fluorescence microscopy images are shown. Scale bar: 5µm.
   **k.** Images of GFP-NUP98::KDM5A nuclear condensates in HEK-293T cells that were untransfected (top row), or transfected with Nanobody-KS or Nanobody-KS_{F-to-A} constructs. Scale bar: 1 µm.
**Figure 6****. The killswitch inhibits adenoviral condensate dynamics, partitioning of capsid proteins, and viral particle assembly.**
   **a.** Live cell fluorescence microscopy images of HEK293T cells expressing the indicated 52K-proteins. The nucleus is highlighted with a dashed white line contour. Scale bar: 5 µm.
   **b.** FRAP of the GFP in the condensates shown in panel a. n=10 in all cases.
   **c.** Control quantification of GFP intensity in the bleached condensates. n=10 in all cases. P-vales Dunnett's multiple comparisons test versus 52K-GFP after one-way ANOVA. **:*P*<10⁻²; ns: not significant.
   **d.** Schematic of the 52K complementation experiment. HEK-293T cells are infected with Δ52 adenovirus, and then transfected with plasmids encoding 52K-proteins.
   **e.** Quantification of viral progeny production measured as plaque-forming units (PFU). n=3 biological replicate experiments. *P*-values are from unpaired two-tailed t-tests. **:*P*<10³.
   **f.** Western blot showing similar levels of 52K-proteins and viral structural proteins in the complementation experiment.
   **g.** The killswitch inhibits partitioning of Illa into 52K condensates. Displayed are immunofluorescence images of 52K and Illa-GFP in HEK-293T cells. Cell nuclei are highlighted with a dashed white line contour. Insets are zoomed images of highlighted condensates. Scale bar: 5 µm.
   **h.** Quantification of the partitioning of Illa-GFP into 52K condensates and the mean Illa-GFP fluorescence. n_{(52K)} = 1583, n_{(52K-KS)} = 2518, n_{(52K-KS-F-to-A)} = 651. P-vales are from Tukey's post-hoc test versus 52K after one-way ANOVA. ***:*P*<2*10⁻¹⁶.
   **i.** Alphafold model of the interaction interface between 52K and Illa. The interacting structural elements are colored (yellow: 52K, magenta: Illa), and key residues are highlighted with numbers.
   **j.** The condensate microenvironment model, and the effect of the killswitch on the condensate microenvironment. As an example, the partitioning of Illa into 52K nuclear bodies is displayed.
**Extended Data** **Figure 1****. The shuffled killswitch sequence can arrest the dynamics of nucleolar condensates.**
   **a.** (left) Model of fsHMGB1 and shuffled sequences of the killswitch (KS) within the tested mEGFP-fsHMGB1 proteins. NoLS: nucleolar localization sequence. (middle) Quantification of the mobile and immobile fractions of mEGFP-HMGB1 proteins in nucleoli. (right) Mean GFP fluorescence of bleached nucleoli. *P*-values are from Dunnett's post-hoc testing versus fsHMGB1-full length after one-wayANOVA. ***:*P*<10⁻³, **:*P*<10⁻².
   **b.** Live cell fluorescence microscopy images of U2OS cells expressing ectopic mEGFP-HMGB1 and RFP-FIB1. The cell nucleus is highlighted with a dashed white line contour. Scale bar: 10 µm.
   **c.** (left) FRAP of mEGFP-HMGB1 and F-to-Y and F-to-W variants. (middle) Quantification of the mobile and immobile fractions of mEGFP-HMGB1 proteins in nucleoli. (right) Mean GFP fluorescence of bleached nucleoli from two independent experiment series. n=20 for all samples, except n=19 for F-to-W. P-values are from Tukey's post-hoc test versus fsHMGB1-full length after ANOVA. ***:*P*<10-3.
   **d.** Live cell fluorescence microscopy images of U2OS cells expressing ectopic mEGFP-HMGB1 proteins and RFP-FIB1. The contour of the cell nucleus is highlighted with a dashed white line. Scalebar: 10 µm.
**Extended Data** **Figure 2****. The killswitch arrests the dynamics of nucleolar condensates, alters their morphology and reduces cell viability.**
   **a.** Live cell fluorescence microscopy images of U2OS cells expressing ectopic mEGFP-fsHMGB1 variants and RFP-FIB1. The cell nucleus is highlighted with a dashed white line contour. Scale bar: 5 µm.
   **b.** FRAP of the indicated mEGFP-HMGB1 variants. The quantification of the corresponding mobile/immobile fraction ratios are shown in **Fig. 1e****.**
   **c.** (left) Model of fsHMGB1 and sequences of the KS variants within the tested mEGFP-HMGB1 proteins. (right) Quantification of circularity of nucleoli in cells expressing ectopic mEGFP-HMGB1 KS variants. *P*-values from Dunnett's multiple comparison test versus fsHMGB1-full length after one-wayANOVA. ****:*P*<10⁻⁴, ***:*P*<10⁻³, *:*P*<0.05.
   **d.** Cell viability of U2OS cell expressing mEGFP-HMGB1 proteins. P-values from one-way ANOVA Dunnett's multiple comparison test versus fsHMGB1-full length. ****:*P*<10⁻⁴, ***:*P*<10⁻³, **:*P*<10⁻². n = 3 for all experiments.
**Extended Data** **Figure 3****. Self-association of killswitch is dependent on phenylalanines.**
   **a.** Schematic models of msfGFP constructs fused with C-terminal KS and KS_{F-to-G}.
   **b.** Size-exclusion chromatography chromatograms of recombinant msfGFP, msfGFP-KS and msfGFP-KS_{F-to-G}. Elution volumes and estimated sizes of proteins eluted in peaks 1 and 2 (P1, P2) are shown below.
   **c.** Coomassie gel of purified proteins before and after size-exclusion chromatography (SEC). P1: peak 1, P2: peak 2 shown in **b.**
   **d.** (top) AlphaFold 3 model of a killswitch dimer; and (bottom) Alphafold 3 model of a killswitch F-to-G mutant dimer. The depth of the color is proportional to the model confidence. Dark blue is high confidence prediction, dim yellow is low confidence.
   **e.** Contact probability heatmap of the killswitch and killswitch F-to-G mutant dimer. In this analysis, the two sequences that make up the dimer are displayed next to each other as a tandem sequence (i.e. Killswitch 1 and Killswitch 2; in total consisting of 34 amino acids), on each axis. The contact probability of each residue with any other residues within the same killswitch or the other half of the dimer are shown as a heatmap. Red arrows highlight the position of the 3 phenylalanines (positions: 12-14, and 29-31), where the contact probability is the highest between the killswitch dimers. The contacts are lost in the F-to-G mutant, shown in the bottom.
**Extended Data** **Figure 4****. Nanobody-based killswitch recruitment system arrests dynamics of condensates.**
   **a.** Live cell fluorescence microscopy images of U2OS cells expressing ectopic EGFP-NPM1 alone or co-transfected with mCherry-GFP-nanobody fusion proteins. The cell nucleus is highlighted with a dashed white line contour. Scale bar: 5 µm.
   **b.** Live cell fluorescence microscopy images of U2OS cells expressing ectopic EGFP-NPM1 and GFP-nanobody (GFP-nb) constructs with cleaved mCherry reporter. The cell nucleus is highlighted with a dashed white line contour. Scale bar: 5µm.
   **c.** Fixed-cell immunofluorescence of cells expressing ectopic EGFP-NPM1 and GFP-nb constructs with cleaved mCherry reporter. GFP-nb is recognized through HA-tag using anti-HA-tag antibody. The cell nucleus is highlighted with a dashed white line contour. Scale bar: 5 µm.
   **d.** Schematic model of the Nanobody-based recruitment system and procedure to measure immobile and mobile fractions of GFP-NPM1 molecules.
   **e.** Quantification of mobile fraction of GFP-NPM1 as a function fluorescence intensity of mCherry reporter. Curves from linear and power regression models are shown as dashed red lines. n = 10 for all samples, except n = 16 for GFP-nb-KS and -2xKS.
**Extended Data** **Figure 5****. Killswitch can be recruited via different epitope tags to immobilize NPM1 in nucleoli.**
   **a.** Schematic depiction of experiment with four different Nanobodies. Four different GFP-NPM1 transgenes were created, with ALFA-tag, V5-tag, VHH05-tag or no tag at the N-terminus. The constructs were co-transfected with the corresponding nanobody from a vector that also encodes and mCherry reporter.
   **b.** FRAP of EGFP-NPM1 tagged with the indicated epitope tags when targeted by their corresponding nanobodies. n=20 for all samples.
   **c.** (left) Mean GFP fluorescence of bleached area. (right) Mean mCherry reporter intensity in nuclei of investigated cells. P-values are from Tukey's multiple comparison test against Nb-only control after one-way ANOVA. n=20 for all samples.
   **d.** (left) Quantification of mobile fraction of GFP-NPM1 as a function fluorescence intensity of GFP-NPM1. (right) Quantification of mobile fraction of GFP-NPM1 as a function fluorescence intensity of mCherry reporter.
**Extended Data** **Figure 6****. Nanobody-based killswitch recruitment system arrests dynamics of endogenous nuclear speckle protein SRRM2 and chromocenter associated HP1α.**
   **a.** Live cell fluorescence microscopy images of HAP1 cells expressing SRRM2-GFP from endogenous locus and ectopic GFP-nb constructs with cleaved mCherry reporter. The cell nucleus is highlighted with a dashed line contour where magenta color represents transfected cell expressing GFP-nb. Scale bar: 5 µm.
   **b.** FRAP of SRRM2-GFP in HAP1 cells. Line displays the mean and shaded color +/- standard deviation, n of samples is same as in c.
   **c.** Quantification of (left) mean GFP fluorescence of bleached condensates and (right) mean mCherry fluorescence of nuclei of examined HAP1 cells. P-values are from Tukey's post-hoc test versus mCherry-alone after one-way ANOVA. Mean is shown as a black line and whiskers represent standard deviation.
   **d.** Live cell fluorescence microscopy images of HCT-116 cells expressing SRRM2-GFP from endogenous locus and ectopic GFP-nb constructs with cleaved mCherry reporter. The cell nucleus is highlighted with a dashed line contour where magenta color represents transfected cell expressing GFP-nb. Scale bar: 5 µm.
   **e.** FRAP of SRRM2-GFP in HCT-116 cells. Line displays the mean and shaded color +/standard deviation. n of samples is same as in **f.**
   **f.** Quantification of (left) mean GFP fluorescence of bleached condensates and (right) mean mCherry fluorescence of nuclei of examined HCT-116 cells. P-values are from Tukey's post-hoc test versus mCherry-alone after one-way ANOVA. Mean is shown as a black line and whiskers represent standard deviation.
   **g.** Live cell fluorescence microscopy images of mouse ES cells expressing GFP-HP1α from endogenous locus and ectopic GFP-nb constructs with cleaved mCherry reporter. Scale bar: 5 µm.
   **h.** FRAP of GFP-HP1α in mouse ES cells. Line displays the mean and shaded color +/standard deviation. n of samples is the same as in i.
   **i.** Quantification of mean GFP fluorescence of bleached chromocenters in mES cells. P-values are from Dunnett's multiple comparison test versus untransfected control after one-way ANOVA. Mean is shown as a black line and whiskers represent standard deviation.
**Extended Data** **Figure 7****. NuFANCI proteome analysis of nucleoli targeted with killswitch.**
   **a.** Principal Component (PC) analysis of NuFANCI-isolated nucleoli from untransfected cells, ActD-treated, GFP-nb, GFP-nb-KS, GFP-nb-2xK), GFP-nb-KS_{F-to-G}, and their respective input samples. The expression values were then standardized.
   **b.** Principal component analysis of NuFANCI-isolated nucleoli without input samples.
   **c.** Correlation plot of NuFANCI samples using Pearson's correlation coefficient.
   **d.** Euler diagrams showing the overlap of NuFANCI-detected proteins in nucleolus reference proteomes.
   **e.** Heatmap showing the protein expression profiles of NuFANCI-isolated nucleoli. Protein markers used in nucleolus isolation, mCherry and msfGFP-NPM1, and protein markers tested by IF are annotated on the right. Proteins have also been annotated based on the proteome of isolated nucleoli from previous proteomics data
   **f.** Barplots showing the percentage of proteins in the pooled input (top) or pooled nuFANCI samples (bottom) mapping to individual sub-compartments annotated in the SubCellBarcode database.
   **g.** Quantification of NEPRO, SURF6, and RPL18 expression in NuFANCI samples is shown in **a.** Letters represent compact letter displays of the Tukey's test, following a one-way ANOVA (*P*-value <0.0001)
   **h.** Volcano plots of NuFANCI pairwise-comparisons. Significance values are from t-tests. Non-significant proteins are shaded in gray, whereas enriched and depleted are shaded in red and blue, respectively. Protein markers tested by IF are annotated in large font.
**Extended Data** **Figure 8****. Arrested dynamics of NPM1 condensates impairs rRNA partitioning.**
   **a.** Fixed cell immunofluorescence for 5.8S rRNA in HCT116 cells expressing GFP-NPM1 from endogenous locus and ectopic GFP-nb constructs with cleaved mCherry reporter. Scale bar: 5 µm.
   **b.** Correlation of GFP-NPM1 and 5.8S rRNA staining intensities measured by Pearson's correlation coefficients (left) and quantifications of mean nuclear mCherry (middle) and GFP (right) intensities. P-values are from Tukey's post-hoc test versus mCherry-alone after one-way ANOVA. Each dot represents one nucleus. Mean is shown as a black line and whiskers represent standard deviation.
**Extended Data** **Figure 9****. Exposing cells to nucleoli-targeting cell penetrating peptides conjugated with the killswitch is cytotoxic.**
   **a.** Sequences of cell penetrating peptides used. All peptides had N-terminal TAMRA-dye for visualization.
   **b.** Live cell fluorescence microscopy images of U2OS cells expressing GFP-NPM1 from endogenous locus. Cells were exposed to 3 µM peptides for 30 minutes and imaged 3 hours after peptide addition. Scale bar: 5 µm.
   **c.** Zoom-in to regions highlighted with dashed square on **b.** Dying cell with nucleolar R10-KS peptide, condensed nuclei and increased Hoechst intensity highlighted with white arrow. Scale bar: 5 µm.
   **d.** Quantification of mean TAMRA signal intensities in nucleoplasmic areas (left), nuclear areas (middle) and nucleolar areas (right). P-values are from Tukey's multiple comparison test after one-way ANOVA.
   **e.** Percentage of healthy and dying cells from cells exposed to 3µM peptides for 30 minutes, imaged 3 hours after peptide addition. Measurements are from two biological replicates.
   **f.** FRAP of GFP-NPM1 in cells with nucleolar TAMRA signal after exposure to cell penetrating peptides. n=20 for all samples.
   **g.** Mean GFP fluorescence (left) and TAMRA fluorescence (right) of bleached area. P-values are from Tukey's multiple comparison test against R10 control after one-way ANOVA, n=20 for all samples. Mean is shown as a black line and whiskers represent standard deviation. ***:*P*<10⁻³, ns: not significant
   **h.** Scatterplot depicting nucleolar TAMRA intensity as function mean nucleolar Hoechst intensity.
**Extended Data** **Figure 10****. The killswitch arrests dynamics of fusion oncoprotein condensates.**
   **a.** Live cell fluorescence microscopy of U2OS expressing ectopic fusion onco-proteins with or without C-terminal KS. The cell nucleus is highlighted with a dashed white line contour. For SS18::SSX1, SS18::SSX2 and NONO::TFE3, genetic fusion of the killswitch leads to cytoplasmic aggregation of the protein. Note that targeting the killswitch with the Nanobody to the already formed condensates has no such effect (see **Fig. 3a****).** Scale bar: 5 µm.
   **b.** FRAP of GFP signal of GFP-fusion oncoproteins. Line displays the mean and shaded color +/- standard deviation. n = 20 for EWS::FLI1, CRTC1::MAML2, NUP98::HOXA9, NUP98::DDX10 and YAP::MAMLD1; n = 10 for SS18::SSX1, SS18::SSX2 and NONO::TFE3.
   **c.** Quantification of mean GFP fluorescence intensity in bleached area of GFP-fusion oncoproteins. P-values are from unpaired t test. Mean is shown as a black line and whiskers represent standard deviation.
   **d.** Fixed cell immunofluorescence of U2OS cells expressing ectopic EGFP-BRD4::NUT WT or -KS variants stained for H3K27Ac chromatin modification. Scale bar: 5 µm.
   **e.** (left) Correlation of GFP-BRD4::NUT and H3K27Ac staining intensities measured by Pearson's correlation coefficients. Each dot represents measurement from one cell nucleus. n = 26, 26 and 29 for -WT, -KS and -KS_{F-to-G} variants, respectively. *P*-values are from Tukey's post-hoc test after one-way ANOVA. standard deviation. (right) Quantification of mean GFP fluorescence intensity in nucleus U2OS cells expressing ectopic GFP-BRD4::NUT variants analyzed for colocalization in **d.** *P*-values are from Tukey's post-hoc test after one-way ANOVA. Mean is shown as a black line and whiskers represent standard deviation. Mean is shown as a black line and whiskers represent standard deviation.
   **f.** Fixed cell immunofluorescence images of U2OS 2-3-6 cells ectopically expressing CFP-Lacl or CFP-Lacl-NUT with or without KS and stained for endogenous RNAPII.
   **g.** Enrichment of RNAPII intensity in the Lacl foci.
   **h.** Intensity of CFP intensity at Lacl foci.
   **i.** Background RNAPII intensity in LacO tethering experiment. For panels g-i, P-values are from Tukey's post-hoc test after one-way ANOVA; * = P<0.05, ** = P<0.01, *** = P<0.001; mean is shown as a black line and whiskers represent standard deviation.
**Extended Data** **Figure 11****. Additional BRD4::NUT data.**
   **a.** (left) Representative live cell fluorescence microscopy of U2OS expressing ectopic EGFP-BRD4::NUT with or without C-terminal KS together with mCherry-p300. (middle) FRAP of the EGFP-BRD4::NUT proteins and mean GFP signals of bleached areas. P-values are from Tukey's multiple comparison test after one-way ANOVA, compared against EGFP-BRD4::NUT. In FRAP assay, number of occurrences of significant differences (***:P<10-3, *:*P*<0.05) among the 2s intervals displayed. (left) FRAP of mCherry p300.
   **d.** Principal component analysis of the RNA-Seq expression profiles of untransfected HEK293T cells, EGFP-control, EGFP-BRD4::NUT-WT, EGFP-BRD4::NUT-KS, and EGFP-BRD4::NUT- KS_{F-to-G} (PC1 vs. PC2)
   **e.** Sample distance matrix calculated using Euclidean distance.
   **f.** Quantification of EGFP expression using normalized counts from the DEseq2.
   **g.** Differential expression analysis of EGFP-BRD4::NUT-WT compared to EGFP control (top-left), EGFP-BRD4::NUT-KS (top-center), EGFP-BRD4::NUT- KS_{F-to-G} (top-right) compared to EGFP control in HEK293T cells; and EGFP-BRD4::NUT-KS compared to EGFP-BRD4::NUT-KS_{F-to-G} (bottom-left), EGFP-BRD4::NUT-KS compared to EGFP-BRD4::NUT-WT, EGFP-BRD4::NUT- KS_{F-to-G} compared to EGFP-BRD4::NUT-WT (bottom-right). The differentially expressed proteins identified by Kosno et al. 2023 was used to color the genes. Referred upregulated genes are colored in red and downregulated in blue.
   **h.** Fixed cell imaging of U2OS cells ectopically expressing EGFP-BRD4::NUT -NUT-KS and NUT-KSF-to-G and cleavable mCherry reporter with or without treatment with 5% 1,6-hexanediol.
   **i.** Quantification of effect of 1,6-hexanediol to granularity of GFP signal for cells shown in panel h represented by relative standard deviation for nuclear GFP (left). Mean intensities of cleavable mCherry reporter (middle) and mean nuclear GFP intensity (right). P-values are from Tukey's post-hoc test after one-way ANOVA. * :*P*<0.05, **: *P<0.01,* *** : *P*<0.001. Mean is shown as a black line and whiskers represent standard deviation.
**Extended Data** **Figure 12****. NUP98::DDX10 mutagenesis and 1,6-hexanediol treatment on cells expressing GFP-NUP98::DDX10 condensates targeted with the killswitch.**
   **a.** Schematic representation of the NUP98::DDX10 control and mutagenesis constructs. The schematics are in scale to the actual amino acid positions.
   **b.** Live cell fluorescence microscopy images of U2OS cells expressing the GFP-NUP98::DDX10 constructs each co-transfected with mCherry-CRM1. Scale bar: 5µm.
   **c.** FRAP of GFP-NUP98::DDX10 (top), and of mCherry-CRM1 (bottom) in GFP-NUP98::DDX10 condensates in U2OS cells exemplified in **b.** n=10 in all cases.
   **d.** (top) Control quantification of GFP intensity in the bleached condensates. (bottom) Control quantification of mCherry intensity in the bleached condensates. *P*-values are from Tukey's post-hoc test after one-way ANOVA. n=10 in all cases.
   **e.** Fixed cell imaging of U2OS cells ectopically expressing GFP-NUP98::DDX10, -DDX10-KS and -DDX10-KSF-to-G and cleavable mCherry reporter with or without treatment with 5% 1,6-hexanediol.
   **f.** Quantification of effect of 1,6-hexanediol to granularity of GFP signal represented by relative standard deviation* of GFP signal in the nuclei (left). Small regions of saturated GFP signal were allowed - see Methods. Mean intensities of cleavable mCherry reporter (middle) and intensities of mean GFP signal outside the detected NUP98::DDX10 foci (right). *P*-values are from Tukey's post-hoc test after one-way ANOVA. * : *P*<0.05, *** : *P*<0.001. Mean is shown as a black line and whiskers represent standard deviation.
**Extended Data** **Figure 13****. Targeting killswitch to NUP98::KDM5A condensates impairs proliferation of GFP-NUP98::KDM5A AML cell line.**
   **a.** Schematic model of experiment using αGFP-Nanobody-based setup to recruit KS to target GFP-NUP98::KDM5A.
   **b.** Quantification of mCherry+ cells in GFP-NUP98::KDM5A AML cell line expressing N-terminally GFP-tagged NUP98::KDM5A after 5 to 19 days of doxycycline (DOX) induction. n = 3, *P*-values are from unpaired two-tailed t test.
**Extended Data** **Figure 14****. Tethering killswitch to NUP98::KDM5A impairs transformation of HPSC to AML.**
   a. Schematic model of experiment to express NUP98::KDM5A-WT and -KS variants in murine fetal liver-derived hematopoietic stem cells.
   b. Quantification for number of colonies per plate formed by cells expressing NUP98::KDM5A-WT or -KS variants after 1-7 weeks. n = 3.
   c. FACS quantification of C-Kit and Gr-1 expression in murine liver-derived HPSCs after the 6th round of replating of cells expressing NUP98::KDM5A and -KS variants.
   d. Measurement of NUP98::KDM5A target gene expression by RT-qPCR. Data shown as log₁₀ fold change.
**Extended Data** **Figure 15****. GFP-NUP98::KDM5A is rapidly degraded after targeting killswitch to NUP98::KDM5A condensates.**
   a. Live-cell fluorescence microscopy images of GFP-NUP98::KDM5A condensates in GFP-NUP98::KDM5AAML cells expressing Doxycycline-inducible Nanobody-KS_{F-to-A} or Nanobody-KS, after the indicated hours of Dox-induction.
   b. Live-cell fluorescence microscopy images of GFP-NUP98::KDM5A condensates in sorted mCherry+ GFP-NUP98::KDM5AAML cells expressing Doxycycline-inducible Nanobody-KS_{F-to-A} or Nanobody-KS, after the 9 hours of Dox-induction. Scale bar: 5 µm.
   c. Quantification of number of GFP-NUP98::KDM5A condensates and mean GFP intensity in sorted mCherry+ GFP-NUP98::KDM5A AML cells expressing Doxycycline-inducible Nanobody-KS_{F-to-A} or Nanobody-KS, after the 9 hours of Dox-induction. Each dot represents one nucleus, n = 60 for all samples. P-values are from unpaired two-tailed t test.
**Extended Data** **Figure 16****. The killswitch arrests dynamics of 52K condensates.**
   a. Live cell fluorescence microscopy images of (top) HEK-293T cells and (bottom) H3122 cells expressing ectopic 52K-GFP and 52K-GFP-KS variants. The cell nucleus is highlighted with a dashed white line contour. Scale bar: 5 µm.
   b. FRAP experiment for 52K-GFP from (top) HEK-293T cells and (bottom) H3122 cells expressing ectopic 52K-GFP and 52K-GFP-KS variants. Line displays the mean and shaded color +/- standard deviation. Scale bar: 5 µm.
   c. Quantification of mean GFP fluorescence intensity within bleached area measured in b. Line displays the mean and whiskers represent standard deviation. n = 10 for all samples.
   d. Fixed cell immunofluorescence images from HEK293T cells expressing ectopic 52K or 52K-KS variants. The cell nucleus is highlighted with a dashed white line contour. Scale bar: 5µm.
   e. Schematic model for complementation experiment to measure viral progeny production in HEK-293T cells transduced with adenovirus lacking 52K (Δ52K) and transfected with vector encoding 52K or 52K-KS variants.
   f. Western blot showing similar levels of 52K-proteins and viral structural proteins in the complementation experiment.
**Extended Data** **Figure 17****. Killswitch arrests dynamics of endogenously GFP-tagged condensates, but not the dilute phase GFP signal.**
   a. Live cell fluorescence microscopy images of TC71 cells expressing GFP-tagged EWSR1 from endogenous locus and ectopic αGFP-Nanobody and Nanobody-KS variants. Orange and magenta boxes show zoom-ins into dilute phase and punctate GFP signal, respectively. The cell nucleus is highlighted with a dashed line contour. Scale bar: 5 µm.
   b. Representative images of the bleached regions at indicated timepoints during FRAP experiment in TC71 GFP-EWSR1 knock-in line.
   c. FRAP experiment for TC71 GFP-EWSR1 knock-in line and ectopic αGFP-Nanobody and Nanobody -KS variants. Line displays the mean and shaded color +/- standard deviation. P-values are from Dunnett's multiple comparisons test versus GFP-nb after one-way ANOVA. Scale bar: 5 µm.
   d. Quantification of GFP fluorescence intensity and mean mCherry fluorescence intensity within bleached area measured in c. Line displays the mean and whiskers represent standard deviation. P-values are from Dunnett's multiple comparisons test versus GFP-nb after one-way ANOVA.
   e. Live cell fluorescence microscopy images of 1765-92 cells expressing GFP-tagged FUS from endogenous locus and ectopic αGFP-Nanobody and Nanobody -KS variants. The cell nucleus is highlighted with a dashed line contour. Scale bar: 5 µm.
   f. FRAP experiment for 1765-92 GFP-FUS knock-in line and ectopic αGFP-Nanobody and Nanobody -KS variants. Line displays the mean and shaded color +/- standard deviation. P-values are from Dunnett's multiple comparisons test versus GFP-nb after one-way ANOVA. Scale bar: 5 µm.
   g. Quantification of GFP fluorescence intensity and mean mCherry fluorescence intensity within bleached area measured in f. Line displays the mean and whiskers represent standard deviation. P-values are from Dunnett's multiple comparisons test versus GFP-nb after one-way ANOVA.
**Extended Data** **Figure 18****. Targeting the KS peptide to transcription bodies in zebrafish embryos using Nanog results in diminished transcriptional output.**
   a. RNA coding for Nanog-mNG, Nanog-mNG-KS_{F-to-G} and Nanog-mNG-KS was injected in 1-cell stage wild type embryos.
   b. Images were taken at 512-cell stage at the midpoint between two mitoses. Nanog constructs are labelled in green and the MiR430 transcripts in magenta. Shown are maximum intensity projections (MIPs) of representative images of individual nuclei.
   c. Quantification of the number of clusters per nucleus in each condition.
   d. Quantification of the total mNeonGreen nuclear intensity, in arbitrary units.
   e. Quantification of the volume of the transcription body in all three conditions.

### Examples

### Material and Methods

### 1. Generation of DNA constructs

All oligonucleotides used to generate plasmids and for RT-qPCR in this study are listed in **Supplementary Table 1.**

### 1.1 Generation of mammalian expression vectors for expression of GFP-fused HMGB1 and NPM1

Mammalian expression vectors pRK5-mEGFP-HMGB1-WT, -Mutant and Mutant-patchless were prepared in previous study ¹ (Addgene plasmids #194548, 194550 and 194553, respectively). To generate fsHMGB1-Full-length with killswitch (KS) C16-to-A, M-to-E&D and 3F-to-E&D variants (Fig. 1e), cDNA fragments were ordered from Twist Biosciences, PCR amplified and assembled into BsrGI+Sall digested pRK5-mEGFP-HMGB1-Mutant plasmid using NEBuilder HiFi DNA assembly mastermix (NEB, E2621). Other KS variants (F-to-A, F-to-G, 0F, F12A, F13A, ΔKS 3F, 11G3F3G, KS Shuffled01-10 and ΔKS-TDP43 HP) were constructed by amplifying pRK5-mEGFP-HMGB1-Mutant vector with primers flanking the KS sequence, and KS variant inserts were generated as single strand oligonucleotide that was used to bridge double-stranded, PCR amplified vector using NEBuilder HiFi DNA assembly mastermix.

To generate PiggyBac carrier vectors, N-terminally EGFP-tagged coding sequence of human NPM1 sequence (Addgene, #131818) ³ was cloned into a backbone of the inducible Caspex expression vector (Addgene #97421) ⁴ linearized by restriction digest with Ncol (NEB, # R0193) and BsrGI (NEB, #R3575). EGFP sequence was cloned from same inducible Caspex expression vector. The KS variants were introduced into the C-terminus of NPM1 through the overhang regions of the reverse PCR primers.

pRK5-NPM1-WT and -KS plasmids were constructed by amplifying EGFP-NPM1-WT and - KS DNA fragments from PiggyBac carrier vectors described above and fragments were assembled into Agel (NEB, #R3352) +Sall (NEB, #R0138) digested pRK5 backbone (mEGFP-HMGB1-WT) using NEBuilder HiFi DNA assembly mastermix.

pRK5-meGFP-NPM1-TDP43-HP plasmid was constructed by amplifying NPM1 sequence from Addgene plasmid # 131818 and cloned via NEBuilder HiFi DNA assembly into a backbone of the pRK5-meGFP (Addgene #18696) ⁵ linearized by restriction digest with BsrGI and Accl (NEB, #R0161). The TDP43-HP sequence (AA 318-343) was introduced into the C-terminus of NPM1 through the overhang regions of the reverse PCR primer.

### GFP-nb-killswitch expression vectors

Initial design of GFP-nb expression constructs included mCherry fused to SV40-NLS-GGGGSGGGGS-linker-GFP-nb-GGGGSGGGGS linker, followed by 0-2 repeats of killswitch peptide in pRK5 vector backbone. To generate this, mCherry was amplified from pET45-mCherry-NPM1 vector (Addgene plasmid #194546) ¹, cDNA sequences for SV40-NLS GGGGSGGGGS-linker (SEQ ID NO: 18)-GFP-nb-GGGGSGGGGS-(0-2 repeats of killswitch) were ordered from Twist Biosciences. LaG-16 variant of GFP-nb ⁶ (Addgene plasmid #128788) ⁷ was used in this study. DNA fragments were PCR amplified and assembled into Agel+Sall digested pRK5-mEGFP-HMGB1 plasmid (Addgene #194550) using NEBuilder HiFi DNA assembly mastermix.

Since expression mCherry-GFP-nb fusion protein had unintended effects on nucleoli **(Extended Data** **Fig. 4a****)** two cleavage sites (P2A and T2A) were introduced between mCherry and GFP-nb to ensure minimal levels of fusion protein while enabling the use of mCherry fluorescence as reporter for GFP-nb expression level. Unless stated otherwise, nanobody construct with cleavable mCherry was used in experiments using GFP-nb. In addition, HA-tag was introduced between T2A and SV40-NLS so that localization of GFP-nb into target condensate could be verified with immunofluorescence **(Extended Data** **Fig. 4c****).** To generate these expression vectors, pRK5-mCherry-GFP-nb vector was PCR amplified using primers that introduce HA-tag before SV40-NLS, and an insert containing GSG-P2A-GSG-T2A sequence was generated as single strand oligonucleotide that was used to bridge double-stranded PCR amplified pRK5-mCherry-GFP-nb vector using NEBuilder HiFi DNA assembly mastermix. pRK5-mCherry-P2A-T2A-HA-tag-SV40-NLS-GFP-nb-1xKS, -2xKS, - 1xKS-F-to-A and -1xKS-F-to-G vectors were generated by amplifying mCherry-P2A-T2A-HA-tag sequence from vector described above and GFP-nb variant sequences from previously prepared pRK5-mCherry-GFP-nb vectors and fragments were assembled into Agel+Sall digested pRK5 vector backbone with NEBuilder HiFi DNA assembly mastermix. Vectors with 2xKS-F-to-A and 2xKS-F-to-G variants were generated by amplifying the vector with primers flanking the KS sequence and 2xKS-F-to-A and -F-to-G sequences were generated as single strand oligonucleotide that was used to bridge double-stranded, PCR amplified vector using NEBuilder HiFi DNA assembly mastermix. For experiments involving mCherry-RPL18 and mCherry-SURF6 (Fig. 3g), mCherry reporter in GFP-nb construct was replaced by TagBFP.

To generate this vector, mCherry and GFP-nb sequences were cut out by Agel+Sall digestion, and TagBFP and P2A-T2A-HA-tag-SV40NLS-GFP-nb sequences were amplified by PCR and assembled back into pRK5 backbone using NEBuilder HiFi DNA assembly mastermix. TagBFP sequence was a kind gift from Edda Shulz lab.

### Killswitch expression vectors with other nanobodies

Sequences for nanobodies against V5-tag (Addgene plasmid 201475 ⁸, ALFA-tag (Addgene plasmid 159986 ⁹) and VHH05-tag (Addgene plasmid 171570 ¹⁰) were ordered as synthetic DNA from Twist Biosciences. mCherry-P2A-T2A-SV40NLS-linker sequences were cloned from previously generated GFP-nb vector and assembled with nanobody sequences with NEBuilder HiFi DNA assembly mastermix. Linker and killswitch sequences were introduced to C-termini of nanobodies in reverse PCR primers.

V5-tag, ALFA-tag or VHH05-tag was fused to N-terminus of EGFP-NPM1 by introducing sequences into the Forward cloning primers when cloning EGFP-NPM1 from PiggyBac carrier vector into Agel and Sall digested pRK5 backbone.

### pUC19 repair templates

Repair templates with msfGFP flanked by 550-900 bp homology arms in N-termini of NPM1, TCOF1, HP1α, EWSR1, and FUS and C-terminus of SRRM2 were generated into pUC19 vector backbone (NEB, N3041S). msfGFP was amplified from previously generated pRK5-mEGFP-HMGB1 plasmid (addgene #194550). DNA fragments for the 5' and 3' homology arms of NPM1, HP1α, EWSR1 and FUS were ordered from Twist Biosciences. The homology arms of TCOF1 were amplified from the gDNA of HCT-116. The repair template of each target gene was assembled into Sall+KpnI (NEB, #R3142) or Hindlll (NEB, #R0104) + BamHI (NEB, #R0136) digested pUC19 vector using NEBuilder HiFi DNA assembly mastermix.

### Generation of GFP-fusion oncoprotein expression vectors

For expressing the N-terminally mEGFP-tagged fusion oncoprotein constructs **(****Fig. 4a** **and** **5d****),** fusion partner sequence of each fusion oncoprotein of NUP98::HOXA9, NUP98::DDX10, SS18::SSX1, SS18::SSX2, NONO::TFE3, YAP::MAMLD1, TAZ::CAMTA1, and PAX::FOXO1 were amplified from U2OS cDNA. The cDNA was first amplified using suitable primers; then the PCR products were amplified as DNA template using primers containing overhang for Gibson assembly reaction. EWS::FLI1 was cloned from Addgene plasmid # 102813 ¹¹. CRTC1::MAML2 was cloned from Addgene plasmid #154265 ¹². NUP98::DDX10 "Mut2" was cloned from synthesized NUP98 sequences from Twist Biosciences that are ligated to DDX10 sequence through Gibson ligation. The KS sequence was introduced through the overhang of the reverse PCR primers. The fusion oncoproteins were then cloned via NEBuilder HiFi DNA assembly into a backbone of the pRK5-meGFP (Addgene #18696) linearized by restriction digest with BsrGI and Accl.

To generate expression plasmids with EGFP-BRD4::NUT, EGFP sequence was amplified from pRK5-EGFP-NPM1 plasmid described above and cDNA for BRD4::NUT fusion protein was amplified from Addgene plasmid #171630 ¹³ as two separate fragments, while generating KS and KS-F-to-G fusions in the C-terminus with reverse primers. BRD4::NUT and EGFP fragments were assembled into Agel+Sall digested pRK5 vector backbone (pRK5-mEGFP-HMGB1) using NEBuilder HiFi DNA assembly mastermix.

For 1.6-hexanediol experiments, mCherry-P2A-T2A sequence was fused N-terminally to EGFP-BRD4::NUT constructs by cloning it from previously prepared pRK5-mCh-P2A-T2A nanobody construct.

### pRK5-mCherry constructs

To generate N-terminal mCherry fusions to SURF6, RPL18 and P300, mCherry sequence was cloned from previously prepared mCherry-P2A-T2A nanobody vectors and GGGSGGGS linker (SEQ ID NO: 19) C-terminal to mCherry was introduced in reverse primer. Coding sequences of SURF6, RPL18 and P300 were amplified from HEK293T cDNA and assembled with mCherry sequence into Agel and Sall digested pRK5 backbone using NEBuilder HiFi assembly mastermix.

To generate C-terminal mCherry fusion to CRM1, mCherry sequence was cloned from previously prepared mCherry-P2A-T2A nanobody vectors and GGGSGGGS linker N-terminal to mCherry was introduced. Coding sequences of CRM1 as amplified from U2OS cDNA and assembled with mCherry sequence into Agel and Accl digested pRK5 backbone using NEBuilder HiFi assembly mastermix.

### Constructs for NUP98::KDM5A experiments

The GFP-NUP98::KDM5A construct was generated by restriction enzyme-guided removal of the IRES sequence from a plasmid for constitutive expression of NUP98::KDM5A as previously described ¹⁴. For generation of GFP-IRES-NUP98::KDM5A-KS/KS_FtoA/KS_FtoG constructs the KS was amplified from OE_KS/FtoA/FtoG-mEGFP and introduced in frame into the GFP-IRES-NUP98-KDM5A vector using Gibson assembly. The TRE3G-mCherry-P2A-nanobody-KS was cloned into a lentiviral entry vector. The retroviral plasmid pCMV-gag/pol was acquired from Cell Biolabs, San Diego, USA.

### 52K expression vectors

To generate mammalian expression plasmids containing C-terminally tagged 52K fusion proteins **(****Fig. 6d****-h, Extended Data** **Fig. 16d****),** plasmid backbones were linearized by restriction digest, and complete plasmids reassembled from linearized backbone and DNA fragments using Gibson assembly Master Mix (NEB, #E2611) using the manufacturers guidelines. All restriction enzymes were purchased from New England Biolabs and were compatible with digest reactions in rCutsmart buffer (NEB, #B6004S). Gene fragments corresponding to all variants of KS were purchased as double stranded DNA from Azenta Life Sciences using the FragmentGENE service **(Supplementary Table 1).** For cloning of GFP-tagged fusion proteins **(****Fig. 6a****, Extended Data** **Fig. 16a****),** plasmids were assembled from p52K-GFP ¹⁵ linearized by digestion with BsrGI (NEB, #R3575) and the corresponding KS gene fragment. For cloning of fusion proteins without GFP tags, p52K ¹⁵ was digested with Nhel (NEB, #R3131) & Sall (NEB, #R0138) restriction enzymes to excise the existing 52K open reading frame and linearize the plasmid backbone. An open reading frame encoding 52K, lacking the stop codon, and containing complementary sequence required for Gibson assembly was PCR amplified using specific primers. A three-fragment assembly was performed using digested backbone, PCR-amplified open reading frame, and the corresponding KS gene fragment. The mammalian expression plasmid encoding GFP-tagged minor capsid protein Illa was previously described ¹⁵.

### Generation of DNA constructs for Zebrafish experiments

A plasmid containing full length Nanog mNeonGreen was generated before ¹⁶. The KS and KS_{F-to-G} control peptide sequences were cloned to the C-terminal end of Nanog-mNeonGreen. These plasmids were used as a template to synthesize RNA using the SP6 mMessage mMachine in vitro transcription kit (Invitrogen AM1340), according to manufacturer's instructions.

### 2. Cell culture

Cells were cultured under standard conditions (37°C and 5% CO₂) in sterile, TC-treated, non-pyrogenic, polystyrene tissue culture dishes (Corning). U2-OS (ATCC #HTB-96) HEK293T (ATCC #CRL-3216), HCT-116 (ATCC #CCL-247), and A673 (kindly gifted by Dr. Heinrich Kovar) cell lines were cultured in DMEM GlutaMAX (Gibco, 31966047). HAP1-SRRM2^{tr0 17} and TC71 (DMSZ, #ACC516) cells were cultured in IMDM (Gibco, 12440053). H3122 (CLS, #300484) and 1765-92 (kindly gifted by Dr. Pierre Åman) cells were cultured in RPMI 1640 Medium (Thermo, 61870036). All media included 10% fetal bovine serum (FBS, Gibco, 10438-026) and 100 U/ml penicillin-streptomycin (Gibco, 15140148).

### 3. Cell viability assay

150,000 cells were seeded on 6 well plate, transfected next day with 500 ng of pRK5-msfGFP-HMGB1-Mutant KS variants using FuGENE HD (Promega, E2311) according to manufacturer's instructions. GFP expressing cells were sorted using FACSAria II instrument (BD) on the following day, 10,000 cells per well were seeded on 96 well plates and viability was measured 48 h later using CellTiter-Glo 2.0 reagents (Promega, G9242) (Fig. **1c****, Extended Data** **Fig. 2d****).**

### 4. Generation of GFP knock-in cell lines

Generation of repair templates with msfGFP flanked by 550-900 bp homology arms in N-terminus of NPM1, TCOF1, HP1α, EWSR1, FUS and C-terminus of SRRM2 into pUC19 vectors is described above. Linear repair template DNA fragments were generated by PCR, gel extracted and purified using QIAquick gel extraction kit (Qiagen, 28704).

### 5. Transplantation-based models and NUP98::KDM5A cell line generation

GFP-NUP98::KDM5A mouse model-derived cell lines were established as described previously ¹⁴. Briefly, GFP-NUP98::KDM5A cell lines were generated by retroviral co-transduction of MSCV -eGFP-N U P98:: KDM5A with MSCV-rtTA3-IRES-NrasG12D-EF1a-Luc2 of murine fetal liver cells (C57BL/6, Ly5.2). 3.74*10⁶ (6.1% GFP+) cells were transplanted into sub-lethally irradiated (4,5 Gy) recipient mice (C57BL/6, Ly5.1) via tail vain injection (Fig. 5f). Disease progression was monitored by whole-body luminescence imaging as previously described. Mice were sacrificed upon disease onset and bone marrow (BM) and spleen cells were harvested. Stable cell lines were established by continuous culture of BM cells for 4 weeks without supplemented cytokines. All animal studies were performed according to ethical animal license protocols and were approved by the responsible authorities of the Austrian government (BMBWF-68.205/0199-V/3b/2018).

### 6. Live cell imaging

All live-cell imaging experiments were performed with LSM880 Airyscan microscope equipped with a Plan-Apochromat ×63/1.40 oil differential interference contrast objective, while keeping cells in incubation at 37°C and 5% CO₂. Cells were seeded on 8-well chamber slides (Ibidi, 80826-90), 30000 cells per well, transfected 24h later and imaged 24h after transfection. U2OS and HEK293T cells were transfected using FuGENE HD; HCT-116, TC71, 1765-92, H3122 and V6.5 mES cells with Lipofectamine 3000 according to manufacturer's instructions. Hoechst 33342 (0.2µg/ml, Thermo Scientific, 62249) was added into cell culture medium for nuclear staining. To visualize nucleoli in living cells, RFP-fibrillarin fusion protein was expressed by transfecting cells with pTagRFP-C1-fibrillarin plasmid (Addgene, 70649) ¹⁸ together with plasmids for msfGFP-fsHMGB1-full-length variants.

For expressing NUP98::KDM5A and KS constructs, HEK 293T cells were seeded on 8-well chamber slides (Ibidi, 80826-90) and cultivated until 70% confluency was reached. For transfection, 1 ng of plasmid DNA and 2.5% Polyethyleneimine (Polyscience, 26292) were mixed in 200 µl of opti-MEM I (Gibco, 31985062) and incubated for 20 min. The mixture was then added dropwise to each well. After overnight incubation, the medium was exchanged to fresh pre-warmed growth medium before live cell imaging. To visualize cell nuclei, cells were incubated with 5 µM DRAQ5 (NobusBio, NBP2-81125) 10 min before imaging.

In fluorescence recovery after photobleaching (FRAP) experiments, two regions of interest (ROI) were determined: a rectangular ROI1, and smaller, circular ROI2 that covered the object to be bleached. GFP signal was bleached within ROI2 using a 488 nm laser with 70-100% intensity, 5-20 iterations, and GFP signal recovery was measured using 1-2 s intervals for 40-60 s. For co-FRAP assays with both GFP and mCherry signal, the mCherry signal was bleached using a 561 nm laser the same way as the GFP signal. Laser intensity, number of iterations and size of ROIs varied between experiments, but were always identical within an experiment. Fluorescence intensities were acquired from 6-20 ROIs from separate condensates in each experiment, quantified using ZEN Black 2.3 software and reported as relative values to the pre-bleaching time point (Fig. **1b****,** **2e****,** **3g****,** **4b****,** **4f****,** **5b****,** **5e****,** **6b****, Extended Data** **Fig. 1a****,** **4e****,** **5a****,** **6b, 6e, 6h****,** **9f****,** **10b****,** **11b****,** **12c****,** **16b****,** **18c****, 18f).** Figures were generated using GraphPad PRISM9 and with R package ggplot2. In FRAP experiments with cells transfected with mCherry-P2A-T2A-GFP-nb vectors, an image was acquired from ROI1 using Hoechst, mCherry and GFP channels before photobleaching, and mCherry signal within nuclear area (defined by Hoechst signal) was used to quantify mCherry expression level **(Extended Data** **Fig. 4e****,** **5b****,** **6c, 6f****,** **8b****).** To quantify nuclear TagBFP intensity mean intensity was measured from a square of 1.4 µm² manually placed in nuclear region outside of nucleolus.

### 7. Nuclear fluorescence-activated non-membrane condensates isolation (NuFANCI)

The NuFANCI method is adopted from the FANCI method ¹⁹. Endogenously msfGFP-NPM1-tagged cells were used for the NuFANCI experiment. 6 million cells were plated in 10 cm dishes, cultured for 24 h followed by medium change and transfected using Lipofectamine 3000 with the GFP-nanobody constructs. The Actinomycin D-condition cells were treated with 400 nM Actinomycin D (Sigma, A1410-2MG) for 1 h before harvesting. 24 h after transfection, the untransfected, ActD, and GFP-nanobody-transfected cells were trypsinized, harvested, then pelleted into 1.5-mL Eppendorf tubes. The cells were fixed with 1 mL 1% formaldehyde diluted in cell culture medium from 16% formaldehyde (Thermo, 28906) for 10 min at room temperature with rotation. The fixation was stopped by adding 1 M glycine (Jena bioscience, CSS-510) to a final concentration of 200 mM for 5 min at room temperature with rotation. The cells were washed twice with cold PBS, each with spin of 1,000 xg at 4°C for 3 min, and the pellets were kept on ice. Next, the fixed cells were sorted on BD FACSAria Fusion to collect mCherry+ cells from the transfected samples and mCherry- cells from the untransfected samples into 15-mL Protein LoBind^{®} Tubes (Eppendorf, 0030122216) coated with FACS buffer (2% FBS, 2 mM EDTA, in PBS). Roughly 850K and 500K events were collected from the transfected and untransfected samples, respectively. The sorted cells were pelleted in 1.5-mL Protein LoBind^{®} Tubes (Eppendorf, 0030108116). Next, the pellets were thoroughly resuspended in 1 mL lysis buffer B0 (50 mM HEPES pH7.5, 150 mM KCI, 1% IGEPAL CA-630, complete protease inhibitor (Sigma 11873580001) and PhosSTOP (Merck 4906837001)) supplied with final concentration of 1 mM DTT, 1:1000 RNAse inhibitor (NEB, M0314L), and 2 µg/mL DAPI. Samples were incubated on ice for 5 min, transferred to Covaris milliTUBE (Covaris, 520130) and then sonicated using Covaris E220 (PIP: 140, Duty factor: 5, Duration: 120 sec). 30 µL of each sample was reserved as the input material for MS. The rest of each sample was transferred to 1.5-mL Protein LoBind^{®} Tubes and sorted on BD FACSAria Fusion with SSC threshold 1000 into 1.5-mL Protein LoBind^{®} Tubes. Roughly 400 K events were collected from each sample. The collected nucleoli were spun down at 10,000 xg for 10 min at 4°C followed by washing once with cold PBS.

For Mass-spectrometry sample preparation, the nucleoli samples were supplied to reach 1x buffer 4 (2x buffer 4: 100 mM Tris, pH 7.5, 50 mM NaCl, and 4 mM MgCl₂) and incubated with 1000 RPM shaking at 65°C for 1 h and then 10 min at 95°C. Then each sample was sonicated on a Qsonica Q700 sonicator equipped with microtip (Bioke, Q4417) with amplitude 5 for 10 sec until no visible particles in the tube. Next, each sample were cooled on ice briefly before added a final concentration of 25 U/µL of Benzonase (Thermo, Thermo 70-746-3) and incubated at 37°C for 30 min with 1000 RPM shaking. Then, the samples were supplied to reach 1x buffer 5 (2x buffer 5: 6 M GdmCl, 20 mM TCEP, 80 mM chloroacetamide) and incubated at 37°C for 1 hr with 1000 RPM shaking. Next, each sample was added ice-cold 1 mL 100% acetone and precipitated at -20°C overnight. In the next day, the samples were centrifuged at 20,000 xg for 10 min at 4°C, discarded supernatant, washed once with ice-cold 100% ethanol, centrifuged at 20,000 xg for 10 min at 4°C, discarded supernatant and air-dry the pellet briefly to dry most of ethanol but not completely dry. Next, the input and nucleoli samples were resuspended in 50 and 30 µL 100 mM (NH₄)HCO₃ respectively. Then the samples were sonicated on a Qsonica Q700 sonicator equipped with microtip with amplitude 5 for 10 sec until no visible particles in the tube. The concentration of the samples were measured in duplicate on Qubit 3 with Qubit protein assay kit (Thermo, Q33211). Roughly 2 mg proteins were yielded from each nucleolus sample. Next, 150 ng of each sample was digested with 5 ng Trypsin and 5 ng Lys-C filled to final volume of 20 µL with 100 mM (NH₄)HCO₃ overnight at 37°C with 800 RPM shaking. The peptides were acidified with formic acid to a final concentration of 2% and 150 ng of the digests were loaded onto Evotip Pure (Evosep, Odense, Denmark) tips in accordance to the manufacturer's protocol. Peptide separation was carried out by nanoflow reverse phase liquid chromatography (Evosep One, Evosep) with the Aurora Elite column (15 cm x 75 µm ID, C18 1.7 µm beads, lonOpticks, Victoria, Australia) and the 20 samples a day method (Whisper Zoom 20 SPD). The LC system was coupled online to a timsTOF SCP mass spectrometer (Bruker Daltonics, Bremen, Germany) using the data-dependent acquisition (DDA) with parallel accumulation serial fragmentation (PASEF) method. The mass spectrometry data were processed with MaxQuant (v2.6.6.0; Max Planck Institute for Biochemistry, Martinsried, Germany) and searched against the human UniProtKB proteome UP000005640 (revi. 2024 09 11). Additional modified sequences as outlined in **Supplementary Table** 1 were used accordingly. The "match between run" and "label-free quantification" features were employed independently for the NuFANCI and input samples. The mass spectrometry data have been deposited to the ProteomeXchange Consortium (http://proteomecentral.proteomexchange.org) via the PRIDE partner repository ²⁰ with the dataset identifier PXD058854.

### 8. Proteomics analysis

Mass spectrometry data were acquired using TimsTOF SCP (Bruker). The raw peak files were processed using MaxQuant ²¹. LFQ values were calculated separately for NuFANCI and input, with a match between runs applied separately for NuFANCI and input. Alphastats v0.6.9 ²² were used to process the MaxQuant output. Protein group matrices were used as input for alphastats, and data were preprocessed to remove contaminants and reversed proteins. Principal component analysis of the NuFANCI and input proteomes was carried out using 500 most variable proteins using ANOVA, and LFQ values were standardized **(Extended Data** **Fig. 7a****).** For PCA of the subset, NuFANCI was performed with a VST-transformed matrix (Extended Data Fig. 9b). Correlation plots were calculated using the SciPy package ²³ in python v3.10 and plotted with Seaborn **(Extended Data** **Fig. 7c****).** Heatmaps was plotted using log2 LFQ values and clustered using Euclidean distance with "ward" method and plotted using seaborn (Fig. 3b and Extended Data Fig. 9e). Protein expression plots were generated using Seaborn (Extended Data Fig. 9f). Volcano plots data was calculated using Alphastats "diff_expression_analysis" function set to "t-test" and then plotted with seaborn **(****Fig. 3c****, Extended Data** **Fig. 7g****).**

### 9. Cell-penetrating-peptide (CPP) experiments

Peptides RRRRRRRRRR (SEQ ID NO: 20), RRRRRRRRRRMMMMNKLVLAQFFFSCL (SEQ ID NO: 21) and RRRRRRRRRRMMMMNKLVLAQGGGSCL (SEQ ID NO:22) with N-terminal TAMRA labels were synthesized by Peptide Specialty Laboratories (Heidelberg, Germany) and reconstituted in DMSO into 2 mM stocks. GFP-NPM1 U2OS cells were seeded on 8-well chamber slides (Ibidi, 80826-90) with a density of 60 000 cells per well. On the next day, cells were washed two times with PBS and exposed to 3 µM peptides in PBS for 30 min in 37°C. Wells were washed once with PBS and then cells were kept in 37°C incubator for 3 h in presence of 0.2 µg/ml Hoechst 33342 (Thermo Scientific, 62249) in cell culture medium, followed by imaging with LSM880 microscope. We noticed that R10 control peptide was often in cytoplasmic foci and only rarely in the nucleolus. To facilitate cellular distribution of R10 control peptide, cells were exposed to Texas Red filter light using HXP lamp for 30 sec, followed by 30 sec waiting period before image acquisition. Imaging of R10-KS and R10-KS-F-to-G was done without additional illumination step.

To analyze nuclear and nucleolar TAMRA signals in cells treated with cell penetrating peptides, nuclei were first segmented using Otsu thresholding (Click Thresholding: 12, 255) and nucleoli were detected by GFP-NPM1 signal (Click Thresholding: 6, 255). Mean nuclear, nucleoplasmic (outside nucleoli) and nucleolar TAMRA signal intensities were measured.

For the images of R10-KS peptide treated cells, Z-positions for dying cells were processed separately, as the aggregated cytoplasmic and extracellular TAMRA signal biased the use of max intensity projections. We note that for the TAMRA intensity calculations, in some cases a nucleus could be detected on different Z-positions and may be counted twice. For this reason, number of dying cells, indicated by the presence of small, condensed nuclei and increased Hoechst staining intensity, was counted by visual inspection. Images were acquired from two biological replicates and included combined 356, 460 and 511 cells for peptides R10, R10-KS and R10-KS-F-to-G, respectively. Replicates were pooled for TAMRA intensity measurements **(Extended Data** **Fig. 9d****).**

### 10. Image analysis for live cell imaging

### Generation of FRAP curves

The recorded fluorescence intensity from each timepoint of each FRAP ROI was normalized by the signal intensity of the first time point. The replicates of each time point in the same FRAP series were plotted in GraphPad and Rstudio with the error bar representing standard deviation.

### Calculation of mobile and immobile fraction

The mobile fraction **(****Fig. 1e****, Extended Data** **Fig. 1a****,** **4e****)** of the FRAP ROI was calculated by using the signal intensity normalized as described above with the following equation: $mobile fraction = \frac{last time point signal intensity - after bleach signal intensity}{1 - after bleach signal intensity}$ $immobile fraction = 1 - mobile fraction$

### Experiments related to NUP98::KDM5A

Raw files were imported into Arivis Vision 4D (Arivis AG, Berlin, Germany) and an automated segmentation pipeline was designed manually. This pipeline consisted of median-based denoising, background correction, Cellpose deep-learning segmentation for cells and nuclei, intensity threshold segmentation for condensates, particle finder and size and sphericity filters. The segmented objects (cells, nuclei and condensates) were manually proofread, and settings were adjusted if necessary. For the data shown in **Fig. 5h****,** ROIs where set based on the mCherry signal, and the number of condensates and mean GFP intensity was calculated for at least 100 individual cells per condition. The same approach was used for the data shown in **Extended Data** **Fig. 15c****,** but at least 60 cells for each condition were analyzed.

### 11. Immunofluorescence

For immunofluorescence experiments performed in GFP-NPM1 knock-in HCT116 cells **(****Fig. 3h****, Extended Data** **Fig. 8a****)** and EGFP-BRD4::NUT expressing cells **(****Fig. 3g****-h, Extended Data Fig. 8g-h),** cells were seeded on 8-well or 18-well chamber slides (Ibidi, 80826-90 and 81816) with 30,000 or 12,000 cells per well, transfected 24 h later and fixed 24 h after transfection with 4% paraformaldehyde (PFA) in PBS for 10-15 minutes. Cells were permeabilized with 0.5% Triton X-100 (Thermo Fisher, 85111) in PBS for 10-15 min, incubated in blocking buffer containing 1% BSA (BSA Fraction V, Gibco, 15260037) and 0.1% Triton X-100 in PBS followed by overnight staining with primary antibodies in +4°C with gentle agitation. Slides were washed 5 times with blocking buffer, incubated with secondary antibodies (Alexa Fluor 647 donkey anti-mouse or anti-rabbit antibodies, Jackson Immuno Research, 715-605-150 and 711-605-152, 1:1000 dilutions) in blocking buffer for 1 h in room temperature, washed twice with blocking buffer, stained with 0.5 µg/ml DAPI in PBS (Invitrogen, D1306) and washed 3 times with PBS. Primary antibodies used were: 5.8S rRNA (Novus, NB100-662SS, 1:500), POLR1A (Santa cruz, sc-48385, 1:100), NEPRO (Santa cruz, sc-376579, 1:100), RNAPII (Abcam, ab26721, 1:500), SURF6 (Santa cruz, sc-515439, 1:100), RPL18 (Sigma Aldrich, hpa046572, 1:200), H3K27Ac (Abcam, ab4729, 1:1000). Imaging was performed with LSM880 Airyscan microscope equipped with a Plan-Apochromat ×63/1.40 oil differential interference contrast objective.

### 12. Image analysis for immunofluorescence

### Pearson's correlation coefficients from IF images

Pearson's correlation coefficients between GFP and IF staining intensities within nuclei were analyzed using ImageJ 2.14.0/1.54f **(****Fig. 4d****, Extended Data** **Fig. 8b****,** **10e****).** First, nuclei were segmented into ROIs using thresholding on the DAPI channel and AnalyzeParticles tool, and Pearson's correlation coefficients between GFP and AF647/Cy5 reported by Coloc2 plugin were collected, and reported for each nucleus with mCherry or GFP expression. Thresholds for DAPI, mCherry and GFP channels varied between experiments, but were kept constant when thresholding images within each experiment.

### Enrichment of GFP-IIIa in 52K condensates

Enrichment of GFP-Illa in 52K condensates was analyzed using ZEN software and Zones of Influence analysis tool to identify 52K condensates with AlexaFluor647 channel using Click Thresholding (Value Lower: 113, Value Upper: 246), and background as ring element (Segmentation Zoi Ring Distance: 3, Ring Thickness: 3) surrounding 52K objects. Enrichment of mean GFP signal in 52K condensates over mean background signal is displayed for individual 52K foci **(****Fig. 6h****).** Background GFP intensities are calculated as mean GFP signal at ring elements surrounding 52K foci, and are displayed in **Fig. 6h****.**

### 13. Protein purification

Overexpression of recombinant protein in BL21 (DE3) (NEB M0491S) was performed as described ²⁴.

### 14. In vitro droplet assay

For *in vitro* droplet formation experiments **(****Fig. 1f**), we measured the concentration of purified msfGFP-tagged proteins with a NanoDrop2000 (Thermo Scientific) and subsequently diluted protein to the required concentration in Storage Buffer (50 mM Tris pH 7.5, 125 mM NaCl, 1 mM DTT, 5% DMSO, 10% glycerol). The *in vitro* droplet formation assay was performed as previously described ²⁵. Protein preparations were mixed 1:1 with 2.5 µL 20% PEG-8000 in de-ionized water (w/v). The resulting 5 µL was pipetted on a chambered coverslip (Ibidi, 80826-90). Images were acquired after 3 minutes equilibration of the drop on the slide, with an LSM880 confocal microscope equipped with a Plan-Apochromat-63x/1.40 oil DIC objective with a 1x zoom. Quantification of condensate formation was based on at least 10 images acquired in at least two independent image series per condition.

### 15. Image analysis of in vitro droplet formation

Protein droplets were detected using ZEN blue 3.4 Image Analysis and Intellesis software packages. By use of a previously trained Intellesis model in spectral mode we achieved image segmentation of individual pixels into objects (droplet area) or background (image background). Relative amounts of condensed protein were calculated by division of the sum of GFP signal in objects defined as droplet area by the overall sum of GFP signal in the field of view. All values were calculated using R-Studio. Plots were generated using GraphPad PRISM9. To fit data to a sigmoidal curve we applied the in-build non-linear regression function (Sigmoidal; x is concentration) **(****Fig. 1g**).

### 16. RNA-sequencing

For experiments involving EGFP-BRD4::NUT **(****Fig. 4g****),** 450,000 HEK293T cells were seeded on 6 well plates and transfected the next day with 3 µg pRK5-EGFP-BRD4::NUT plasmids using 9 µl PEI STAR transfection reagent (Tocris, 7854). Total RNA was isolated from cells 24 h after transfection using RNEasy mini kit with in-column DNase digestion (Qiagen). RNA sequencing libraries were prepared KAPA HyperPrep Kit with RiboErase (Roche, KK8562) and sequenced in 100 bp paired-end mode with Illumina NovaSeq2 for 55-65M fragments per sample.

Raw RNA-seq data were filtered and trimmed using TrimGalore v0.6.10 (https://doi.org/10.5281/zenodo.7598955) with default settings. Filtered data from HEK293K cells were mapped to a custom human genome hg38, including the EGFP sequence cloned using the STAR aligner ²⁶ to hg38 human genome. Count-read tables were generated by using the same program. Differential expression analysis was performed using the DEseq2 package ²⁷ in R, version 4.4 ²⁸. Differentially expressed genes were defined as having a fold change ≥ 1.2, Benjamini-Hochberg P ≤ 0.01, and a minimum mean read count across the experimental samples of 50 reads.

Principal component analysis was performed using the PCAPlot function from the DEseq2 package on the normalized read matrix that was transformed using the variance-stabilizing transformation function from the DEseq2 package and plotted using ggplot2 **(Extended Data** **Fig. 11d****).** The distance matrix was calculated with the dist function in R using the Euclidean distance and visualized with a pheatmap in R **(Extended Data** **Fig. 11e****).** Volcano plots were created using ggplot2 **(****Fig. 4g** and **Extended Data** **Fig. 11g****).**

For the reference data on BRD4::NUT-targets from a previous study (Kosno et al.)², raw data was downloaded from GEO submission (GSE233302) and processed as described above.

Differentially expressed genes were defined with a fold change cut-off of 1.2 and a p-value adjusted to 0.01. Only BRD-NUT and control samples were used.

### 17. Real-time PCR analysis

For experiments involving expression of NUP98::KDM5A and KS variants, total RNA was extracted using the Monarch Total RNA Miniprep Kit. Reverse transcription was done using RevertAID RT Kit (Thermo Scientific) and quantitative PCR was done using the SsoAdvanced Universal SYBR Green Supermix (Bio-Rad, Hercules, CA, USA) or AceQ Universal SYBR qPCR Master Mix (Vazyme, Red Maple Hi-tech Industry Park, Nanjing, PRC) on a Bio-Rad CFX-Connect Real-Time PCR Detection System. Results were normalized to GAPDH and analyzed using the 2-ddC(t) method **(Extended Data Fig. 14d).**

### 18. 1,6-Hexanediol treatments

Cleavable mCherry was included in EGFP-BRD4::NUT expression vector to better distinguish and compare transfected cells. U2OS cells were seeded on 8-well chamber slides (Ibidi, 80826-90) at density of 30 000 cells per well and transfected the following day with pRK5-mCherry-P2A-T2A-EGFP-BRD4::NUT plasmids using FuGENE HD and 150 ng plasmid per well according to manufacturer's instructions. On the next day, cells were treated with 5% 1,6-hexanediol (Sigma Alrich, 240117) in cell culture medium for 5 or 15 minutes, fixed with 4% PFA for 10 minutes, washed and stored in PBS. Nuclei were stained using 0.5 µg/ml DAPI in PBS (Invitrogen, D1306).

For the EGFP-BRD4::NUT 1.6-hexanediol experiments, images were analyzed using ZEN Blue 3.9 software. Nuclei were segmented using automatic Otsu thresholding and mean intensity, standard deviation and max intensities from GFP and mCherry channels were measured for each nucleus. Nuclei with no expression (mean mCherry intensity < 1.5) abnormally high expression (mCherry expression > 40), and cells with saturated GFP signals were excluded. Images were acquired from three biological replicate experiments, and in the end, measurements were pooled and combined into single plots **(Extended Data** **Fig. 11i****).**

To analyze 1,6-hexanediol treated GFP-NUP98::DDX10 expressing cells, nuclei were first segmented using Otsu thresholding (Click Thresholding: 2, 255) and GFP-NUP98::DDX10 foci in nuclei were detected using GFP signal (Click Thresholding: 9, 255). Nuclear background areas outside the foci were determined with inverted thresholds and area were shrunk with Erode set to 2. To reliably measure diffuse GFP in the nucleus, GFP acquisition settings were set accordingly. However, because of the high contrast of GFP intensity in NUP98::DDX10 foci vs. nucleoplasm, GFP signal was often saturated at NUP98::DDX10 foci already at cells with low GFP expression. As a compromise, we excluded all nuclei with saturated foci detected in areas larger than 8 pixels. Nuclei with no expression (mean mCherry intensity < 1.5) very high expression (mCherry expression > 40). Images were acquired from two biological replicate experiments. Measurements were pooled and combined into single plots **(Extended Data** **Fig. 12f****).** Due to small regions of saturated GFP that were permitted, mean GFP intensities for NUP98::DDX10 samples are not plotted, standard deviations of GFP intensity are to be interpreted with caution and instead the background GFP is considered most reliable.

### 19. Colony formation assay for NUP98::KDM5A

Each experiment was performed in triplicates **(Extended Data** **Fig. 14b****).** Briefly, 25*10³ Murine leukemia cells were seeded in methylcellulose (MethoCult M3434, Stem Cell Technologies) and colonies were scored and re-plated (10*10³ cells or 5*10³ cells) every 7 days.

### 20. Flow cytometry for NUP98::KDM5A

For characterization of cells derived from the NUP98::KDM5A colony formation assay, cells were washed with PBS and resuspended in PBS with 0.5% FCS, followed by staining for 30 min with dilutions (1:200) of the following antibodies (all from Biolegend, San Diego, CA, USA): anti-mouse Gr-1/Ly-6C BV421 (clone RB6-8C5) and anti-mouse CD117/c-Kit APC (clone 2B8). Samples were analyzed on a BD FACSCanto II. Data analysis was done using the FlowJo (FlowJo LLC, Ashland, OR, USA) software package **(Extended Data Fig. 14c).**

### 21. Competitive cell proliferation assay for NUP98::KDM5A

To investigate the effect of the KS on proliferation, a competition-based proliferation assay was performed using GFP-NUP98::KDM5A AML cells transfected with doxycycline-inducible nanobody-KS constructs at an infection rate of approximately 25-30% as described ¹⁴. 5x10⁵ cells were seeded in 24-well plates in triplicates. Doxycycline was added to the media and fluorescence expression of mCherry (coupled to nanobody-KS constructs) was measured every 2-3 days using a Cytoflex S instrument (Beckman Coulter, USA). The effect of the nanobody-KS constructs on cell fitness was monitored via time-resolved measurements of mixed populations of competing nanobody-KS constructs-expressing mCherry-positive vs. nanobody-KS constructs/mCherry-negative cells. Data analysis was done using the FlowJo (FlowJo LLC, Ashland, OR, USA) software package and values were normalized to day 3 after Doxycycline induction (**Extended Data** **Fig. 13b**).

### 22. Growth curves for NUP98::KDM5A

Cells were sorted for mCherry and seeded in biological triplicates and treated with Doxycycline every 48 or 72 hours. Cell numbers were determined in regular intervals with the Intellicyt iQue Screener (Essen BioScience, Sartorius Group) and integrated with ForeCyte Software (Essen Bioscience, Ann Arbor, Michigan, USA) (**Fig**. **5g**).

### 23. Viruses and infections

For retrovirus production related to NUP98::KDM5A experiments, Platinum-E cells were co-transfected with 20 µg transfer vector and 5 µg pCMV-gag/pol using polyethyleneimine (branched, MW 25.000, Sigma-Aldrich, Vienna, Austria). Virus supernatant was harvested 48 and 56 h post transfection, filtered (0.45 µm) and supplemented with recombinant mIL-3 (10 ng/ml), mIL-6 (10 ng/ml) (both PeproTech, Vienna, Austria) and mSCF (100 ng/ml). Murine progenitor cells were spinoculated with viral supernatants (1:2 diluted) for 45 min at 37°C at 500xg in the presence of polybrene (4 µg/ml) (Merck Chemicals and Life Science, Germany). For lentivirus production Lenti-X 293T cells were transfected with 4 µg transfer vector, 2 µg psPAX2 and 1 µg pMD2.G using PEI. Lentivirus was harvested 48 and 72 hours post transfection and filtered (0.45 µm). Target cells were spinoculated after addition of virus supernatant (1:3 diluted) followed by centrifugation for 90 min at 37°C at 1000xg in the presence of polybrene (5 µg/ml).

For experiments related to 52K constructs, human adenovirus type C5 (Ad5) wild-type (WT) was purchased from ATCC (Cat#: VR-5), propagated on HEK-293 cells, purified via cesium chloride density ultra-centrifugation, and stored in 40% glycerol at -20°C for infections. The Ad5 Δ52K mutant pm8001 (Δ52K) ²⁹, a gift from P. Hearing (Stony Brook University, NY), was propagated on transgenic cell line (A549) expressing WT 52K ¹⁵, purified via cesium chloride density ultra-centrifugation, and stored in 40% glycerol at -20°C for infections. Viruses were purified using two sequential rounds of ultracentrifugation in cesium chloride density gradients. To achieve a cryoprotective solution for storage, virus was diluted as followed: 2-parts virus in cesium chloride, 1-part 5x viral dilution solution (40 mM Tris pH8, 400 mM NaCl, 0.4% BSA in H₂O), 2-parts 100% glycerol. Viral titers were determined by infectious focus forming assay as described in the Viral Progeny Production section. All infections were carried out using a multiplicity of infection (MOI) of 10 unless stated otherwise and harvested at indicated hours post-infection (hpi). To infect cells, virus was diluted in cell culture media without FBS. After 2 h at 37°C, culture media containing 10% FBS was added. For virus yield assays, the virus infection media was removed after 2 h, and cells were washed 1x in PBS before addition of culture medium to remove excess virus.

### Adenoviral progeny production

Infected cells were harvested by scraping and lysed by four cycles of freeze-thawing in liquid nitrogen and a 37°C water bath. Cells were harvested at 48 h p.i. unless stated otherwise. Cell debris was removed from lysates by centrifugation at max speed, 4°C, 5 min. For analysis of virus yield, lysates were diluted serially in DMEM supplemented with 2% FBS and 1% Pen/Strep and used to infect A549 cells. Infection media was removed 2h after infection, cells were washed 1x in PBS to remove excess virus, and cells were overlaid with growth media. Cells were incubated for 24 h prior to fixation in 4% PFA and analyzed by immunofluorescence confocal microscopy using immunostaining of the viral DNA binding protein (DBP) as an indicator of infection. For each of the three independent replicates, three fields of view were captured and the percentage of DBP positive cells was determined. The serial dilution resulting in closest to 50% DBP-positive cells was selected for calculation of progeny production. Total cell number was determined by counting cells grown in parallel under equivalent conditions. The number of focus-forming-units (FFU) was calculated as the product of total cell number and % of antigen positive cells, adjusting for the Poisson distribution. Virus input was determined by harvesting infected cells at 4 h p.i., the number of infectious units determined, and the mean of replicates calculated (**Fig. 6e**). For complementation of Δ52K mutant virus infection, cells were infected and then subsequently transfected at 2h post infection.

### 24. Zebrafish embryo manipulations

Zebrafish were maintained and raised under standard conditions, and according to Swiss regulations (canton Vaud, license number VD-H28). Wild type (TLAB) fish were used for this study. Embryos were collected immediately after fertilization. The embryos were grown at 28°C and the developmental stage was determined as described by Kimmel ³⁰. 180 pg of each RNA was injected in the yolk of 1-cell stage embryos. The embryos were allowed to develop in a 28°C incubator and then manually dechorionated using forceps. Embryos were transferred to mounting medium (0.8% low melting agarose containing 15% v/v OptiPrep (Sigma Aldrich, D1156)) at 37oC and mounted on an Ibidi glass bottom µ-dish (Ibidi, 81158-400).

### Microscopy and image analysis for Zebrafish embryo

Embryos were imaged on a Nikon spinning disc, using Nikon SR HP Plan Apo 100x / 1.35 Sil WD 0.3 objective, in a temperature-controlled chamber set at 28oC. 4D imaging x, y, z and time was performed by keeping a z-stack of 27 µm and z-step of 0.3 µm with a time resolution of 2 min. Microscopy images were further analyzed and processed using ImageJ. Whole nuclei at the 512-cell stage were first segmented in 3D and further analysis was performed using the "3D objects Counter" plugin ³¹. The generated data frames in .csv format were exported to RStudio software for plotting and visualization. Statistical analysis was performed using GraphPad prism. Kruskal Wallis test with Dunn's multiple comparisons were performed to calculate the significance between different groups.

### 25. SDS-PAGE and immunoblot analysis

### For the experiments performed relating to the adenovirus-52K experiments

SDS-PAGE and immunoblot analysis was carried out using standard methods. In brief, protein samples were prepared using lithium dodecyl sulfate (LDS) loading buffer (Thermo-Fisher, Cat#: NP0007) supplemented with 25 mM DTT and boiled at 95°C for 10 min. Equal amounts of protein lysate were separated by SDS-PAGE. Proteins were transferred onto methanol activated Polyvinylidene Fluoride (PVDF) membrane (Millipore Sigma, Cat#: IPFL00010) at 30 V for 60-120 min and blocked in blocking buffer (5% milk in TBST supplemented with 0.05% sodium azide) for 1 h at room temperature. Membranes were incubated overnight at 4°C with primary antibodies diluted in blocking buffer, washed for 30 min in TBST, incubated for 1 h at room temperature with HRP-conjugated secondary antibody diluted in blocking buffer, and washed again for 30 min in TBST.

The following primary antibodies were used: Anti-adenovirus Type 5 antibody raised against whole adenovirus capsids; recognizing late proteins Hexon, Penton, Fiber (Abcam Cat#: ab6982), species: rabbit, polyclonal, WB 1:10,000. Antibody to 52K (gift from P. Hearing, Stony Brook University, NY) ³², species: rabbit, polyclonal, WB 1:10,000. DBP (gift from A. Levine) ³³, species: mouse, clone: B6-8, WB 1:1000. GAPDH (GeneTex, Cat#: GTX100118, Lot: 43712), species: rabbit, polyclonal, WB 1:5,000. For immunoblot analysis, Horseradish peroxidase-conjugated goat anti-rabbit secondary antibody (Jackson Laboratories, Cat#: 111-035-045) was used at a concentration of 1:10,000.

Proteins were visualized with Pierce ECL Western Blotting Substrate (Thermo-Fisher, Cat#: 34577) and detected using a Syngene G-Box using GeneSys acquisition software. Images were processed and assembled in Adobe Illustrator CS6 (**Fig. 6f****, Extended Data** **Fig. 16f**).

### 26. Reproducibility and statistics

All experimental observations were confirmed by independent repeat experiments. No blinding or sample randomization was employed during data capture or analysis. No data were excluded from data analysis. No statistical tests were performed to pre-determine sample size. Unless stated otherwise, the mean of numerical data is shown, with error bars representing standard deviation of the sample. Statistics were performed using GraphPad Prism version 9 and RStudio version 2024.04.2 and "multicomp" package. All t-tests are two-sided. A normal distribution was assumed when determining statistical tests. Equal variance was not assumed.

### 27. Alphafold models

Alphafold models were predicted using Alphafold v3 ³⁴ in multimeric mode using default parameters. Models were visualized with ChimeraX v1.6 ³⁵. Contacts plot were generated with custom scripts.

### Results

We previously identified a pathogenic frameshift variant of the nuclear protein HMGB1 that causes a rare complex malformation syndrome ¹. The frameshift generates a *de novo* C-terminal tail which leads to the mispartitioning of the mutant HMGB1 protein into the nucleolus, the largest biomolecular condensate in human cells ¹. The mutant HMGB1 arrests the dynamics of the nucleolus, ultimately leading to cell death (**Fig**. **1a****-c**) ¹. The nucleolar arrest appears to be caused by the C-terminal 17 amino acids, as deleting these residues does not change nucleolar mispartitioning, but rescues nucleolar dynamics and cell viability (**Fig. 1a****-c**) ¹. We therefore hypothesized that the 17-amino acid micropeptide sequence, hereafter referred to as the "killswitch", could be engineered into a generally applicable tool to alter material properties of specific condensates, and as a consequence, to probe condensate microenvironments and functions in living cells.

We performed systematic mutagenesis of the killswitch to identify the sequence features and molecular mechanism that drive nucleolar arrest. GFP-tagged mutant HMGB1 transgenes were transiently expressed in human osteosarcoma U2OS cells, and nucleolar dynamics was quantified as the ratio of mobile versus immobile fractions of GFP-HMGB1 in nucleoli using fluorescence recovery after photobleaching (FRAP) (**Fig. 1d**). We found that mutation of the cluster of three phenylalanines to negatively charged residues, alanines or glycines was sufficient to rescue nucleolar dynamics to the same degree as deleting the entire killswitch sequence (**Fig. 1e**). Mutation of individual phenylalanines did not lead to a rescue, suggesting that all three phenylalanines are necessary (**Fig. 1e**). The phenylalanines, however, required the rest of the killswitch sequence, as mutation of the other 14 residues into glycines, or fusing three phenylalanines to a mutant HMGB1 lacking the killswitch sequence also rescued nucleolar dynamics (**Fig. 1e**). Shuffling the killswitch sequence further revealed that clustering of the phenylalanines was in itself not necessary, but enhanced nucleolar arrest (**Extended Data** **Fig. 1a****-d**). The rescue of nucleolar dynamics correlated with rescue of the circularity of nucleoli and cell viability (**Extended Data** **Fig. 2a****-d**). Similar results were observed when the killswitch was recruited to nucleoli via ectopic expression of the nucleolar protein NPM1 genetically fused to the killswitch sequence. These results suggest that phenylalanines drive nucleolar arrest, but require the additional sequence context of the killswitch.

Droplet assays and size-exclusion chromatography revealed phenylalanine-dependent self-association of the killswitch *in vitro.* The purified, GFP-tagged, recombinant killswitch protein, but not the F-to-G mutant formed droplets in a concentration-dependent manner in the presence of physiological salt (125mM NaCl) and crowding agent (10% PEG 8000) (**Fig. 1f-****g**). Consistent with the results of the droplet assays, ~18% of the GFP-tagged killswitch protein, but <1% of the F-to-G mutant eluted as multimers after size-exclusion chromatography (**Extended Data** **Fig. 3a****-c**). Modelling the structure of the killswitch with AlphaFold 3 ³⁴ further confirmed that it is a helix-prone peptide that self-interacts and that the phenylalanines are necessary for the killswitch-killswitch interactions (**Extended Data** **Fig. 3d-****e**). Taken together, we conclude that the killswitch has the ability to self-associate, and the phenylalanines have an important contribution to the valence of self-association.

We noted additional unique features of the killswitch. For example, the sequence itself does not appear in the human proteome). A similar, short hydrophobic patch in TDP-43 was described before, and oxidization of methionines in the patch is associated with the emergence of solid TDP43 aggregates ^{36,37}. In contrast to the killswitch, the TDP-43 hydrophobic patch failed to arrest nucleolar dynamics when tested with two different recruitment systems in cells. In addition, mutation of methionine residues in the killswitch did not affect its ability to solidify nucleoli (**Fig. 1e**). The killswitch therefore appears to be non-natural in humans, and likely impacts condensate dynamic properties through a different mechanism than the TDP-43 hydrophobic patch.

As a potentially universal approach to target the killswitch to any cellular condensate, we developed a VHH antibody (Nanobody)-based recruitment system. We generated a DNA vector encoding the killswitch fused to an anti-GFP Nanobody ⁶, with the idea that the anti-GFP Nanobody (GFP-nb) would enable recruitment of the killswitch to any GFP-labelled condensate (**Fig. 2a****-b**). The DNA vector also contained an mCherry reporter expressed bicistronically, because we found that fusion of the Nanobody to mCherry was sufficient to perturb GFP-NPM1 labelled nucleoli, potentially because of the large size of the fusion protein (**Extended Data** **Fig. 4a****-b**). Thus, the Nanobody-expressing cells, but not the Nanobody itself are identified with mCherry in this system (**Fig. 2b****-c**), which was corroborated using a Nanobody variant attached to an HA-tag (**Extended Data** **Fig. 4c**).

As an initial test of the Nanobody-system, the killswitch was recruited to several endogenous condensates including nucleoli, heterochromatin bodies and nuclear speckles in cultured cells. Nucleoli are multilayered condensates that consist of a granular component, dense fibrillar component and fibrillar center, and are involved in rRNA transcription and ribosome biogenesis ^{38,39}. We generated HCT-116 human cell lines in which a GFP tag was knocked-into the endogenous *NPM1* and TCOF1 loci, to GFP-label the granular component and fibrillar center, respectively. The cell lines were transfected with Nanobody-killswitch expression vectors. FRAP experiments revealed significantly slower recovery after photobleaching of both NPM1 and TCOF1 in the cells that expressed Nanobody-Killswitch, compared to untransfected cells, cells that expressed the Nanobody alone, or cells that expressed the Nanobody fused to the F-to-A killswitch mutant (**Fig. 2e****-f**). As expected for specific targeting, the effect in the FRAP assay correlated with the amount of mCherry marker expressed in the corresponding cell (**Extended Data** **Fig. 4d****-e**). the results were corroborated with three additional Nanobodies (α-ALFA, α-V5, α-VHH05) targeting ectopically tagged GFP-NPM1 protein (**Extended Data** **Fig. 5d****-d**). Immobilization of condensate-forming proteins was also observed in multiple human cell lines in which nuclear speckles were labelled with a GFP tag on endogenous SRRM2, a key component of speckles (**Fig. 2d****-f, Extended Data** **Fig. 6a****-f**) ¹⁷; and in murine embryonic stem cells, in which chromocenters were labelled with a GFP tag on endogenous HP1α, an important heterochromatin protein (**Fig. 2d****-f, Extended Data** **Fig. 6g****-i**) ^{40,41}. In the case of chromocenters, two copies of the killswitch fused to the Nanobody were necessary to elicit an effect (**Fig. 2d****-f, Extended Data** **Fig. 6g****-i**). In the case of nuclear speckles, an effect was observed with a single copy of the killswitch in HCT-116 cells, and the effect of two tandem killswitch copies was more prominent (**Fig. 2d****-f, Extended Data** **Fig. 6d-****f**). Over time, SRRM2-GFP collapsed into large, dense, spherical foci in cells expressing the killswitch (**Fig. 2d****, Extended Data** **Fig. 6a, 6d**). Two tandem copies of the killswitch also led to a gradual demixing of GFP-NPM1 into dense foci (**Fig. 2d**). These results suggest that the killswitch alters material properties of (i.e., "immobilizes" or "solidifies") various proteins in nucleoli, nuclear speckles in human cells, and chromocenters in murine stem cells.

Investigating the consequences of solidifying components of the nucleoli with the killswitch revealed several insights and surprises. NPM1 is known as an essential scaffolding protein of the granular component of nucleoli, where ribosome assembly occurs ⁴²⁻⁴⁵. To map changes in the composition of nucleoli targeted with the killswitch, we developed a Fluorescence Activated Cell Sorting (FACS)-based method ¹⁹ to isolate nucleoli directly from cell lysates termed "nuFANCI" (**Fig. 3a**). Mass Spectrometry was performed on nucleoli isolated from GFP-NPM1-encoding cells expressing Nanobody-killswitch constructs. Comparison of the proteome of the isolated nucleoli with previous reference data ⁴⁶⁻⁴⁸ confirmed strong enrichment of known nucleolar proteins in our samples (**Fig. 3b**, **Extended Data** **Fig. 7a****-f**). Comparative analysis revealed 20 proteins that were significantly depleted (≥2-fold, *P*≤0.01) from nucleoli targeted with the killswitch over control cells, and the proteins were enriched for RNA-binding proteins (P<0.05, g:GOS test) (**Fig. 3c****, Extended Data** **Fig. 7g****-h, Table S2**). Depletion of NEPRO, a known nucleolar interaction partner of NPM1 was confirmed with immunostaining (**Fig. 3d****-f**). The compositional change of nucleoli was associated with changes in component mobility and function. For example, a significant reduction of the mobility of RPL18-mCherry measured with FRAP was observed in cells where GFP-NPM1 was targeted with the killswitch (**Fig. 3g****,**), but no change in the mobility of SURF6-mCherry, while the level of neither protein was affected (**Fig. 3g****,**). Moreover, the NPM1-dense nucleolar regions "demixed" from 5.8 S rRNA, suggesting a potential functional defect (**Fig. 3h****-i, Extended Data** **Fig. 8a****-b**). Finally, when added to cell culture media, killswitch conjugated with an oligo-arginine micropeptide (R10), was taken up by cells, localized to the nucleolus as expected ⁴⁹⁻⁵², and killed cells under conditions where the R10 alone or R10-KS_{F-to-G} controls did not (**Extended Data** **Fig. 9a****-e**). Cell death was associated with immobilization of GFP-NPM1 (**Extended Data** **Fig. 9f****-h**). Collectively, these results revealed selective changes in nucleolar composition, component mobility and function in nucleoli when NPM1 is immobilized with the killswitch.

Condensate alterations have been linked to hundreds of human diseases, notably, cancers driven by fusion oncoproteins, and rare genetic diseases ^{1,53,54}. We therefore tested the effect of recruiting the killswitch to multiple disease-specific condensates.

Around 2,000 fusion transcripts that arise from cancer-associated chromosomal rearrangements are predicted to produce cancer-specific proteins that may form condensates ⁵⁴. Recent studies have provided insights into the cellular functions of a few fusion oncoprotein condensates, however, the functions and pathomechanism for most of them are yet to be discovered ^{55,56}. We therefore tested the impact of recruiting the killswitch to well-characterized fusion oncoproteins known to form condensates. In these experiments, GFP-tagged fusion oncoproteins were co-expressed with Nanobody-killswitch constructs. For all eight fusion oncoprotein condensates tested (EWS::FLI1, SS18::SSX2, CRTC1::MAML2, NUP98::HOXA9, NUP98::DDX10, NONO::TFE3, TAZ::CAMTA1 and PAX3::FOXO1), the Nanobody-Killswitch had negligible effect on the formation of condensates, but significantly reduced fluorescence recovery after photobleaching (**Fig. 4a****-c**). Consistent results were observed when the killswitch was genetically fused to the fusion oncoproteins (**Extended Data** **Fig. 10a****-c**). These results suggest that the killswitch altered the material properties of a diverse set of condensate-forming fusion oncoproteins.

We selected two fusion oncoprotein-condensates for further analyses, and found specific changes in condensate composition, component dynamics and cellular function elicited by the killswitch. BRD4::NUT is a fusion between the bromodomains of BRD4 and the intrinsically disordered region of NUT, and is a key oncogenic driver in NUT-midline carcinoma ⁵⁷. The NUT portion of the fusion recruits p300 that catalyzes the acetylation of histones that are the substrates of the bromodomains. This process results in the formation of large BRD4::NUT condensates that can incorporate ~2MB of genomic DNA 13,58. Consistent with this model, BRD4::NUT fusion oncoprotein condensates were enriched for H3K27Ac in immunofluorescence experiments (**Extended Data** **Fig. 10d****-e**) ⁵⁸. RNA Polymerase II (RNAPII) was enriched in BRD4::NUT condensates, but to our surprise, RNAPII enrichment was significantly reduced in condensates formed by the BRD4-NUT-killswitch protein (**Fig. 4d-****e**), while the level and co-localization of H3K27Ac was unaltered (**Extended Data** **Fig. 10d-****e**). The depletion of RNAPII was confirmed in a cell-based artificial condensate model system (**Extended Data** **Fig. 10f****-i**) ⁵⁹. Fusion of the killswitch to BRD4-NUT also reduced the dynamics of ectopically expressed mCherry-p300 in the condensates (**Fig. 4f****, Extended Data** **Fig. 11a****-c**). The reduction of RNAPII enrichment in BRD4::NUT-KS condensates was associated with reduced transcription of target genes measured with RNA-Seq (**Fig. 4g****, Extended Data** **Fig. 11d****-g**). Treatment of the cells with 1-6 hexanediol dissolved BRD4::NUT but not BRD4::NUT-KS condensates, further confirming that material properties of the condensates were affected by the killswitch (**Extended Data** **Fig. 11h****-i**). These results indicate that the killswitch inhibits RNAPII partitioning and dynamics of p300 in BRD4::NUT condensates, which results in reduced transcriptional activity.

As second case study of fusion oncoprotein-condensates, we focused on NUP98-fusion proteins that are important drivers in acute myeloid leukemia (AML) ⁶⁰. In these leukemias, the intrinsically disordered region of the nuclear pore protein NUP98 is fused to domains of various nuclear proteins often involved in chromatin modification and transcriptional control, e.g., HOXA9, DDX10, NSD1 and KDM5A ⁶¹⁻⁶³. In contrast to endogenous NUP98, oncogenic NUP98 fusion proteins are not associated with the nuclear pore complex, and instead form nuclear condensates ⁶¹⁻⁶³. To measure dynamic changes in condensates, FRAP experiments were performed on mCherry-tagged CRM1 (XPO1), a known client protein enriched in NUP98::DDX10 condensates ⁶³. We observed slower recovery of mCherry-CRM1 fluorescence in cells expressing GFP-NUP98::DDX10 fused to the killswitch (**Fig. 5a****-c**). To directly test whether reduced CRM1 mobility is caused by altered material properties of NUP98::DDX10 we designed mutants in which we clustered GLFG motifs, because clustering of aromatic residues has been linked to arrested condensate dynamics ^{64,65}. Similar to the effect of the killswitch, clustering of GLFG motifs in NUP98 indeed reduced mobility of both GFP-NUP98::DDX10 and CRM1 in condensates ("Mut2") (**Fig. 5d****-e, Extended Data** **Fig. 12a****-d**). Treatment of the cells with 1,6 hexanediol dissolved NUP98::DDX10 but not NUP98::DDX10-KS condensates, further confirming that material properties of the condensates were affected by the killswitch (**Extended Data** **Fig. 12e****-f**). These results suggest that the killswitch alters the microenvironment of oncoprotein condensates, which affects composition and dynamics of client proteins within the condensates.

Finally, we tested whether the killswitch can reveal insights into cellular activities associated with the disease-specific condensates *in vivo.* We previously established a murine AML model, in which fetal liver-derived HPSCs are transformed via expression of the NUP98::KDM5A fusion protein and subsequent transplantation into recipient mice. NUP98::KDM5A-dependent AML cells are then isolated from the bone marrow (**Fig. 5f**) ^{14,63}. To investigate the effect of the killswitch on oncogenic NUP98::KDM5A condensates, we introduced doxycycline-inducible anti-GFP-Nanobody-Killswitch and anti-GFP-Nanobody-Killswitch F-to-A constructs in a stable AML cell line in which NUP98::KDM5A is tagged with an N-terminal GFP tag (**Fig. 5f**). The vector also contained the bicistronic mCherry reporter (**Fig. 5f**). Growth curves of mCherry-sorted cells revealed that the Dox-induced expression of the Nanobody-Killswitch essentially blocked proliferation while the Nanobody-killswitch F-to-A mutant did not have such an effect (**Fig. 5g**). The inhibitory effect of the killswitch was also observed in a competition-based proliferation assay (**Extended Data** **Fig. 13a****-b**). In a second approach, genetic fusion of the killswitch to GFP-NUP98::KDM5A impaired the transformation of mouse fetal liver-derived primary HPSCs into leukemic cells as measured by reduced replating capacity, altered immunophenotype and reduced expression of NUP98::KDM5A target genes (**Extended Data Fig. 14ad**). These results suggest that the killswitch is sufficient to inhibit the growth of leukemia cells whose proliferation is dependent on NUP98::KDM5A condensates.

To investigate whether the proliferation defect is caused by alterations in NUP98::KDM5A condensates, we performed fluorescence microscopy. To our surprise, expression of the Nanobody-Killswitch - assessed by the appearance of mCherry in the cells - led to an almost instantaneous reduction in the number of NUP98::KDM5A condensates and the level of the fusion oncoprotein, whereas the F-to-A killswitch variant had no such effect (**Fig. 5h****-i, Extended Data** **Fig. 15a****-c**). Short term treatment (3h) of the cells with proteasome inhibitors partially rescued the expression level of the fusion protein in cells expressing the Nanobody-killswitch, though the NUP98::KDM5A protein formed large amorphous bodies (**Fig. 5j**). Despite multiple attempts, assessment of the material properties of the oncofusion condensates with FRAP in the AML cells failed because of the low-level expression of the protein. Therefore, to gain further insights into the effect of the killswitch on NUP98::KDM5A condensates, we transfected the fusion protein and the Nanobody-killswitch constructs into HEK-293T cells. FRAP experiments confirmed that the killswitch arrested the dynamics of NUP98::KDM5A condensates (**Fig. 5k**). These results suggest that the antiproliferative effect of the killswitch in NUP98::KDM5A condensate-driven AML cells may be associated with altering the material properties of NUP98::KDM5A condensates. Furthermore, the unexpected finding of rapid proteasome-dependent degradation of the fusion protein upon killswitch expression revealed that leukemia cells do not tolerate any perturbation of cancer-driving fusion oncoprotein-containing condensates.

As another example of disease-specific condensates, we targeted the killswitch to adenoviral nuclear bodies. Adenoviruses encode a packaging protein called 52K that forms nuclear condensates essential for the assembly of infectious viral particles from viral genomes and structural proteins ¹⁵. We first tested whether the killswitch can arrest the dynamics of 52K condensates, by performing FRAP after transient expression of GFP-tagged 52K protein fused to the killswitch. Wild-type 52K condensates recovered near all fluorescence within seconds after photobleaching (**Fig. 6a****-c, Extended Data** **Fig. 16a****-c**). In contrast, virtually no recovery was observed when bleaching GFP-52K-KS condensates, and fluorescence recovery was mostly rescued when using the F-to-A killswitch mutant (**Fig. 6a****-c, Extended Data** **Fig.16a-****c**). These results suggest that the killswitch arrests the dynamics of adenoviral 52K condensates.

The impact of the killswitch on the function of adenoviral 52K condensates was tested in a complementation assay. HEK-293T cells were infected with Δ52K mutant adenovirus and then were either left untransfected or were transfected with a vector encoding wild type and killswitch-tagged 52K variants. Infected cells were harvested for analysis at 48 hours post-infection, and infectious progeny production was measured in focus forming units (**Fig. 6d**). We note that we used the genetic killswitch fusion in the complementation assay, because fixed cell immunofluorescence suggested that the GFP-tag had a slight effect on condensate formation by the 52K protein **Extended Data** **Fig. 16a, 16d****).** The killswitch had a dramatic effect in the complementation assay, suppressing the production of viral particle production by > 90% (**Fig. 6e**), a significant portion of which was rescued by the F-to-A killswitch variant, suggesting that the suppression of viral particle production was at least in-part due to the killswitch (**Fig. 6e**)**.** As a key control, Western blot analysis confirmed that viral structural proteins and the 52K-variants were expressed at similar levels across all conditions (**Fig. 6f**). To investigate the mechanistic basis of the ability of killswitch to suppress viral particle assembly, we assessed recruitment of adenovirus minor capsid protein Illa into condensates, since recruitment of Illa is known to be critical for progeny production ¹⁵. Significantly less Illa-GFP was detected in 52K condensates tagged with the killswitch, compared to the wild type 52K condensates and 52K condensate tagged with the F-to-A killswitch variant (**Fig. 6g****-h, Extended Data** **Fig. 16e****-f**), while the mean nuclear concentration of Illa-GFP was similar (**Fig. 6h**). These results were surprising, because the portion of 52K which is necessary for interaction with Illa is known and present in the experiment ¹⁵, and the interaction interface between the two proteins is unaffected by the presence of the killswitch when modelled with Alphafold 3 (**Fig. 6i**). The data therefore suggest that Illa partitions into 52K condensates facilitated by the microenvironment of 52K condensates rather than merely by stoichiometric interactions (**Fig. 6j**). Taken together these results revealed that the killswitch arrests the dynamics of 52K adenoviral condensates, and inhibits the production of viral particles, which is associated with reduced partitioning of the viral structural protein Illa into 52K condensates.

Here we present a micropeptide killswitch as a principal method that complements biochemical and genetic perturbation to investigate functions of biomolecular condensates. The killswitch is unique in that it has a controllable and titratable effect on the material properties of a variety of endogenous condensates in living cells. We found that the killswitch immobilized constituent proteins of nucleoli, nuclear speckles, chromocenters, fusion oncoprotein condensates and adenoviral nuclear bodies. We note that we did not find evidence that the killswitch affects the mobility of the soluble fraction of the targeted protein in the systems we tested (**Extended Data** **Fig. 17a****-g**). For the condensates tested here, the killswitch altered condensate composition, component dynamics and function, and even had an effect on the transcriptional output of transcription bodies in Zebrafish embryos (**Extended Data** **Fig. 18a****-e**) ^{16,66}. The changes appeared selective to certain condensate components, suggesting that various proteins likely partition into condensates through different underlying molecular features. The killswitch had more dramatic effects on some condensates (e.g., NUP98-fusion condensates) and marginal effect on others (e.g. chromocenters), revealing differences in the nature of the interactions between the condensate-forming proteins in various the condensates.

One of the most intriguing prediction about biomolecular condensates is that they create microenvironments that facilitate partitioning of other biomolecules into the condensates in a manner not explained by stoichiometric interactions ⁶⁷⁻⁷⁰. Current empirical evidence for this model includes measuring concentrations of small molecules that exceed the number of potential binding sites mostly within artificially assembled condensates ⁷¹⁻⁷⁶, and most recently, demonstrating a pH gradient within nucleolar condensates in cell lysates ⁷⁷. Using the killswitch, we provide evidence for unique microenvironments within multiple condensates in live cells. For example, the killswitch altered the composition and inhibited the dynamics of client proteins in nucleoli, oncoprotein condensates and viral nuclear bodies, revealing insights about their *in vivo* functions. We emphasize that the killswitch affected protein composition of condensates even though all the interacting regions in the native proteins are present. We anticipate that the killswitch will facilitate future insights into the compositional biases of condensates in relation to their functions, enable the distinction of the set of proteins residing in condensates from their soluble pool, facilitate investigation of compositional and functional specificity of diverse condensates, and ultimately accelerate manipulation of the material properties of condensate-forming proteins as therapeutics.

### Supplementary Table 1. Oligonucleotides for the manufacture of pRK5-HMGB1 KS variants used in the study

### Primer name and sequence

HMGB1_Fw (SEQ ID NO: 23)
   gggatcactctcggcatggacgagctgtacacggcgATGGGCAAAGGAGATCCTAAGA
HMGB1-Mut-C-to-A Rev (SEQ ID NO: 24)
   tggcggccaagcttctgcaggtcgactTTATAGCGCGGAGAAGAAGAACTG
HMGB1-Mut-4F-to-EDED Rev (SEQ ID NO: 25)
   tggcggccaagcttctgcaggtcgactTTATAGACAGGATTCATCTTCCTGCG
HMGB1-Mut-4M-to-EDED Rev (SEQ ID NO: 26)
   tggcggccaagcttctgcaggtcgactTTATAGACAGGAGAAGAAGAACTGCG
HMGB1-Mut-3F-to-3A Rev (SEQ ID NO: 27)
   tggcggccaagcttctgcaggtcgactTTATAGACAGGAAGCAGCAGCCTG
HMGB1-Mut-3F-to-3G Rev (SEQ ID NO: 28)
   tggcggccaagcttctgcaggtcgactTTATAGACAGGATCCTCCTCCCTG
HMGB1-Mut-backbone-amp Fw(SEQ ID NO: 29)
   TAAtctagagtcgacctgcagaagc
HMGB1-Mut-backbone-amp Rev (SEQ ID NO: 30)
   CTTCTTCTTCATCTTCATCTTCTTCATCTTCCTCCTCC
HMGB1-mut-11G-3F-3G-insert (SEQ ID NO: 31) 
AAGATGAAGAAGATGAAGATGAAGAAGGAGGAGGAGGAGGAGGAGGAGGA
GGAGGATTCTTCTTCGGAGGAGGATAAtctagagtcgacctgcagaagcttggc
HMGB1-mut-F12A-insert (SEQ ID NO: 32) 
AAGATGAAGAAGATGAAGATGAAGAAGAAGATGATGATGATGAATAAGTTGGTTCTAGCG
CAGGCGTTCTTCTCCTGTCTATAAtctagagtcgacctgcagaagcttggc
HMGB1-mut-F13A-insert (SEQ ID NO: 33) 
AAGAAGATGAAGATGAAGAAGAAGATGATGATGATGAATAAGTTGGTTCTAGCGCAGTTC
GCGTTCTCCTGTCTATAAtctagagtcgacctgcagaagcttggc
HMGB1-mut-F13A-F14A-insert (SEQ ID NO: 34) 
AAGATGAAGAAGATGAAGATGAAGAAGAAGATGATGATGATGAATAAGTTGGTTCTAGCG
CAGTTCGCTGCTTCCTGTCTATAAtctagagtcgacctgcagaagcttggc
GFP-HMGB1-mut-del-KS-bb-amp Fw (SEQ ID NO: 35)
   tctagagtcgacctgcagaagc
HMGB1-WT-bb-amp Rev (SEQ ID NO: 36)
   TTCATCATCATCATCTTCTTCTTCATCTTCATCTTCT
HMGB1-WT-bb-amp Fw2 (SEQ ID NO: 37)
   tctagagtcgacctgcagaagcttggccgccatgg
HMGB1-WT-KS-insert (SEQ ID NO: 38) 
GAAGATGAAGAAGAAGATGATGATGATGAAATGATGATGATGAATAAGTTGGTTCTAGCG
CAGTTCTTCTTCTCCTGTCTATAAtctagagtcgacctgcagaagcttg
HMGB1-mut-delKS-bb-amp Rev (SEQ ID NO: 39)
   CTTCTTCTTCATCTTCATCTTCTTCATCTTCCTCCTCC
HMGB1-mut-delKS-TDP43-insert (SEQ ID NO: 40) 
GATGAAGATGAAGAAGAAGATTAATCCAGCCATGATGGCTGCCGCCCAGGCAGCACTAC
AGAGCAGTTGGGGTATGATGGGCATGTTAGCCAGCCAGTAAtctagagtcgacctgcagaa
Ins_HMGB1_mut_ks_s1 (SEQ ID NO: 41) 
gaagaagatgaagatgaagaagaagTGTGTTAAGTTGTCCATGAATGCGCAGTTCCTACTAATGTT
CATGATGTTCTAAtctagagtcgacctgcagaagc
Ins_HMGB1_mut_ks_s2 (SEQ ID NO: 42) 
gaagaagatgaagatgaagaagaagTGTTTCATGTTGAATAAGTTCCTAGTTATGCAGATGTCCTT
CATGCTAGCGTAAtctagagtcgacctgcagaagc
Ins_HMGB1_mut_ks_s3 (SEQ ID NO: 43) 
gaagaagatgaagatgaagaagaagTTCATGGCGTTCCTAAATGTTTTGCTAAAGATGCAGTGTAT
GTCCTTCATGTAAtctagagtcgacctgcagaagc
Ins_HMGB1_mut_ks_s4 (SEQ ID NO: 44) 
gaagaagatgaagatgaagaagaagGCGATGATGAATTTCTTCCAGTTGATGTTCCTAGTTAAGTC
CATGCTATGTTAAtctagagtcgacctgcagaagc
Ins_HMGB1_mut_ks_s5 (SEQ ID NO: 45) 
gaagaagatgaagatgaagaagaagATGATGTTGGCGCTAAAGTCCTGTTTCGTTATGATGCAGC
TATTCTTCAATTAAtctagagtcgacctgcagaagc
Ins_HMGB1_mut_ks_FFFclch_6 (SEQ ID NO: 46) 
gaagaagatgaagatgaagaagaagATGATGATGATGAATAAGTTGGTTTTCTTCTTCCTAGCGCA
GTCCTGTCTATAAtctagagtcgacctgcagaagc
Ins_HMGB1_mut_ks_FFFclch_7 (SEQ ID NO: 47) 
gaagaagatgaagatgaagaagaagATGATGATGATGTTCTTCTTCAATAAGTTGGTTCTAGCGCA
GTCCTGTCTATAAtctagagtcgacctgcagaagc
Ins_HMGB1_mut_ks_FFFscr_8 (SEQ ID NO: 48) 
gaagaagatgaagatgaagaagaagATGATGTTCATGATGAATAAGTTGTTCGTTCTAGCGCAGTC
CTGTTTCCTATAAtctagagtcgacctgcagaagc
Ins_HMGB1_mut_ks_FFFscr_9 (SEQ ID NO: 49) 
gaagaagatgaagatgaagaagaagATGTTCATGATGATGAATTTCAAGTTGGTTCTAGCGTTCCA
GTCCTGTCTATAAtctagagtcgacctgcagaagc
Ins_HMGB1_mut_ks_FFFscr_10 (SEQ ID NO: 50) 
gaagaagatgaagatgaagaagaagATGATGATGTTCATGAATAAGTTCTTGGTTCTAGCGCAGTT
CTCCTGTCTATAAtctagagtcgacctgcagaagc
Ins_HMGB1_mut_ks_FtoY (SEQ ID NO: 51) 
GAAGAAGATGAAGATGAAGAAGAAGATGATGATGATGAATAAGTTGGTTCTAGCGCAGTA
CTATTACTCCTGTCTATAATCTAGAGTCGACCTGCAGAAGC
Ins_HMGB1_mut_ks_FtoW (SEQ ID NO: 52)

GAAGAAGATGAAGATGAAGAAGAAGATGATGATGATGAATAAGTTGGTTCTAGCGCAGT
GGTGGTGGTCCTGTCTATAATCTAGAGTCGACCTGCAGAAGC

### References

1 Mensah, M. A. et al. Aberrant phase separation and nucleolar dysfunction in rare genetic diseases. Nature, doi:10.1038/s41586-022-05682-1 (2023).
2 Kosno, M., Currie, S. L., Kumar, A., Xing, C. & Rosen, M. K. Molecular features driving condensate formation and gene expression by the BRD4-NUT fusion oncoprotein are overlapping but distinct. Sci Rep 13, 11907, doi:10.1038/s41598-023-39102-9 (2023).
3 Wang, A. J., Han, Y., Jia, N., Chen, P. & Minden, M. D. NPM1c impedes CTCF functions through cytoplasmic mislocalization in acute myeloid leukemia. Leukemia 34, 1278-1290, doi:10.1038/s41375-019-0681-8 (2020).
4 Myers, S. A. et al. Discovery of proteins associated with a predefined genomic locus via dCas9-APEX-mediated proximity labeling. Nature methods 15, 437-439, doi:10.1038/s41592-018-0007-1 (2018).
5 Harvey, C. D., Yasuda, R., Zhong, H. & Svoboda, K. The spread of Ras activity triggered by activation of a single dendritic spine. Science 321, 136-140, doi:10.1126/science.1159675 (2008).
6 Fridy, P. C. et al. A robust pipeline for rapid production of versatile nanobody repertoires. Nature methods 11, 1253-1260, doi:10.1038/nmeth.3170 (2014).
7 Prole, D. L. & Taylor, C. W. A genetically encoded toolkit of functionalized nanobodies against fluorescent proteins for visualizing and manipulating intracellular signalling. BMC Biol 17, 41, doi:10.1186/s12915-019-0662-4 (2019).
8 Zeghal, M. et al. Development of a V5-tag-directed nanobody and its implementation as an intracellular biosensor of GPCR signaling. J Biol Chem 299, 105107, doi:10.1016/j.jbc.2023.105107 (2023).
9 Jin, F. et al. Fluorescence-detection size-exclusion chromatography utilizing nanobody technology for expression screening of membrane proteins. Commun Biol 4, 366, doi:10.1038/s42003-021-01891-y (2021).
10 Xu, J. et al. Protein visualization and manipulation in Drosophila through the use of epitope tags recognized by nanobodies. eLife 11, doi:10.7554/eLife.74326 (2022).
11 Loganathan, S. N. et al. BET bromodomain inhibitors suppress EWS-FLl1-dependent transcription and the IGF1 autocrine mechanism in Ewing sarcoma. Oncotarget 7, 43504-43517, doi:10.18632/oncotarget.9762 (2016).
12 Musicant, A. M. et al. CRTC1/MAML2 directs a PGC-1alpha-IGF-1 circuit that confers vulnerability to PPARgamma inhibition. Cell reports 34, 108768, doi:10.1016/j.celrep.2021.108768 (2021).
13 Alekseyenko, A. A. et al. The oncogenic BRD4-NUT chromatin regulator drives aberrant transcription within large topological domains. Genes & development 29, 1507-1523, doi:10.1101/gad.267583.115 (2015).
14 Schmoellerl, J. et al. CDK6 is an essential direct target of NUP98 fusion proteins in acute myeloid leukemia. Blood 136, 387-400, doi:10.1182/blood.2019003267 (2020).
15 Charman, M. et al. A viral biomolecular condensate coordinates assembly of progeny particles. Nature 616, 332-338, doi:10.1038/s41586-023-05887-y (2023).
16 Kuznetsova, K. et al. Nanog organizes transcription bodies. Current biology : CB 33, 164-173 e165, doi:10.1016/j.cub.2022.11.015 (2023).
17 Ilik, I. A. et al. SON and SRRM2 are essential for nuclear speckle formation. eLife 9, doi:10.7554/eLife.60579 (2020).
18 Wang, T. et al. Identification and characterization of essential genes in the human genome. Science 350, 1096-1101, doi:10.1126/science.aac7041 (2015).
19 Zhou, Y. et al. RNA damage compartmentalization by DHX9 stress granules. Cell 187, 1701-1718 e1728, doi:10.1016/j.cell.2024.02.028 (2024).
20 Perez-Riverol, Y. et al. The PRIDE database resources in 2022: a hub for mass spectrometry-based proteomics evidences. Nucleic acids research 50, D543-D552, doi:10.1093/nar/gkab1038 (2022).
21 Sinitcyn, P. et al. MaxDIA enables library-based and library-free data-independent acquisition proteomics. Nature biotechnology 39, 1563-1573, doi:10.1038/s41587-021-00968-7 (2021).
22 Krismer, E., Bludau, I., Strauss, M. T. & Mann, M. AlphaPeptStats: an open-source Python package for automated and scalable statistical analysis of mass spectrometry-based proteomics. Bioinformatics 39, doi:10.1093/bioinformatics/btad461 (2023).
23 Virtanen, P. et al. SciPy 1.0: fundamental algorithms for scientific computing in Python. Nature methods 17, 261-272, doi:10.1038/s41592-019-0686-2 (2020).
24 Basu, S. et al. Unblending of Transcriptional Condensates in Human Repeat Expansion Disease. Cell 181, 1062-1079 e1030, doi:10.1016/j.cell.2020.04.018 (2020).
25 Asimi, V. et al. Hijacking of transcriptional condensates by endogenous retroviruses. Nature genetics, doi:10.1038/s41588-022-01132-w (2022).
26 Dobin, A. et al. STAR: ultrafast universal RNA-seq aligner. Bioinformatics 29, 15-21, doi:10.1093/bioinformatics/bts635 (2013).
27 Love, M. I., Huber, W. & Anders, S. Moderated estimation of fold change and dispersion for RNA-seq data with DESeq2. Genome biology 15, 550, doi:10.1186/s13059-014-0550-8 (2014).
28 R-Core-Team. A language and environment for statistical computing. R Foundation for Statistical Computing http://www.R-project.org/. (2021).
29 Gustin, K. E. & Imperiale, M. J. Encapsidation of viral DNA requires the adenovirus L1 52/55-kilodalton protein. J Virol 72, 7860-7870, doi:10.1128/JVI.72.10.7860-7870.1998 (1998).
30 Kimmel, C. B., Ballard, W. W., Kimmel, S. R., Ullmann, B. & Schilling, T. F. Stages of embryonic development of the zebrafish. Dev Dyn 203, 253-310, doi:10.1002/aja.1002030302 (1995).
31 Bolte, S. & Cordelieres, F. P. A guided tour into subcellular colocalization analysis in light microscopy. J Microsc 224, 213-232, doi:10.1111/j.1365-2818.2006.01706.x (2006).
32 Ostapchuk, P., Yang, J., Auffarth, E. & Hearing, P. Functional interaction of the adenovirus IVa2 protein with adenovirus type 5 packaging sequences. J Virol 79, 2831-2838, doi:10.1128/JVI.79.5.2831-2838.2005 (2005).
33 Reich, N. C., Sarnow, P., Duprey, E. & Levine, A. J. Monoclonal antibodies which recognize native and denatured forms of the adenovirus DNA-binding protein. Virology 128, 480-484, doi:10.1016/0042-6822(83)90274-x (1983).
34 Abramson, J. et al. Accurate structure prediction of biomolecular interactions with AlphaFold 3. Nature 630, 493-500, doi:10.1038/s41586-024-07487-w (2024).
35 Pettersen, E. F. et al. UCSF ChimeraX: Structure visualization for researchers, educators, and developers. Protein Sci 30, 70-82, doi:10.1002/pro.3943 (2021).
36 Conicella, A. E., Zerze, G. H., Mittal, J. & Fawzi, N. L. ALS Mutations Disrupt Phase Separation Mediated by alpha-Helical Structure in the TDP-43 Low-Complexity C-Terminal Domain. Structure 24, 1537-1549, doi:10.1016/j.str.2016.07.007 (2016).
37 Jiang, L. L. et al. Structural transformation of the amyloidogenic core region of TDP-43 protein initiates its aggregation and cytoplasmic inclusion. J Biol Chem 288, 19614-19624, doi:10.1074/jbc.M113.463828 (2013).
38 Lafontaine, D. L. J., Riback, J. A., Bascetin, R. & Brangwynne, C. P. The nucleolus as a multiphase liquid condensate. Nature reviews. Molecular cell biology 22, 165-182, doi:10.1038/s41580-020-0272-6 (2021).
39 Pederson, T. The nucleolus. Cold Spring Harbor perspectives in biology 3, doi:10.1101/cshperspect.a000638 (2011).
40 Brandle, F., Fruhbauer, B. & Jagannathan, M. Principles and functions of pericentromeric satellite DNA clustering into chromocenters. Semin Cell Dev Biol 128, 26-39, doi:10.1016/j.semcdb.2022.02.005 (2022).
41 Probst, A. V. & Almouzni, G. Pericentric heterochromatin: dynamic organization during early development in mammals. Differentiation 76, 15-23, doi:10.1111/j.1432-0436.2007.00220.x (2008).
42 Grisendi, S. et al. Role of nucleophosmin in embryonic development and tumorigenesis. Nature 437, 147-153, doi:10.1038/nature03915 (2005).
43 Mitrea, D. M. et al. Nucleophosmin integrates within the nucleolus via multi-modal interactions with proteins displaying R-rich linear motifs and rRNA. eLife 5, doi:10.7554/eLife.13571 (2016).
44 Mitrea, D. M. et al. Self-interaction of NPM1 modulates multiple mechanisms of liquid-liquid phase separation. Nature communications 9, 842, doi:10.1038/s41467-018-03255-3 (2018).
45 Okuwaki, M., Saito, S., Hirawake-Mogi, H. & Nagata, K. The interaction between nucleophosmin/NPM1 and the large ribosomal subunit precursors contribute to maintaining the nucleolar structure. Biochim Biophys Acta Mol Cell Res 1868, 118879, doi:10.1016/j.bbamcr.2020.118879 (2021).
46 Andersen, J. S. et al. Nucleolar proteome dynamics. Nature 433, 77-83, doi:10.1038/nature03207 (2005).
47 Orre, L. M. et al. SubCellBarCode: Proteome-wide Mapping of Protein Localization and Relocalization. Molecular cell 73, 166-182 e167, doi:10.1016/j.molcel.2018.11.035 (2019).
48 Stenstrom, L. et al. Mapping the nucleolar proteome reveals a spatiotemporal organization related to intrinsic protein disorder. Molecular systems biology 16, e9469, doi:10.15252/msb.20209469 (2020).
49 Schneider, A. F. L., Kithil, M., Cardoso, M. C., Lehmann, M. & Hackenberger, C. P. R. Cellular uptake of large biomolecules enabled by cell-surface-reactive cell-penetrating peptide additives. Nat Chem 13, 530-539, doi: 1 0.1 038/s41557-021-00661-x (2021).
50 Brunner, J. & Barton, J. K. Targeting DNA mismatches with rhodium intercalators functionalized with a cell-penetrating peptide. Biochemistry 45, 12295-12302, doi:10.1021/bi061198o (2006).
51 Schneider, A. F. L., Wallabregue, A. L. D., Franz, L. & Hackenberger, C. P. R. Targeted Subcellular Protein Delivery Using Cleavable Cyclic Cell-Penetrating Peptides. Bioconjug Chem 30, 400-404, doi:10.1021/acs.bioconjchem.8b00855 (2019).
52 Kwon, I. et al. Poly-dipeptides encoded by the C9orf72 repeats bind nucleoli, impede RNA biogenesis, and kill cells. Science 345, 1139-1145, doi:10.1126/science.1254917 (2014).
53 Banani, S. F. et al. Genetic variation associated with condensate dysregulation in disease. Developmental cell 57, 1776-1788 e1778, doi:10.1016/j.devcel.2022.06.010 (2022).
54 Tripathi, S. et al. Defining the condensate landscape of fusion oncoproteins. Nature communications 14, 6008, doi:10.1038/s41467-023-41655-2 (2023).
55 Shirnekhi, H. K., Chandra, B. & Kriwacki, R. W. The Role of Phase-Separated Condensates in Fusion Oncoprotein-Driven Cancers. Annual Review of Cancer Biology 7, 73-91, doi:10.1146/annurev-cancerbio-061421-122050 (2023).
56 Jevtic, Z., Allram, M., Grebien, F. & Schwaller, J. Biomolecular Condensates in Myeloid Leukemia: What Do They Tell Us? Hemasphere 7, e923, doi:10.1097/HS9.0000000000000923 (2023).
57 French, C. A. et al. BRD4-NUT fusion oncogene: a novel mechanism in aggressive carcinoma. Cancer research 63, 304-307 (2003).
58 Rosencrance, C. D. et al. Chromatin Hyperacetylation Impacts Chromosome Folding by Forming a Nuclear Subcompartment. Molecular cell 78, 112-126 e112, doi:10.1016/j.molcel.2020.03.018 (2020).
59 Janicki, S. M. et al. From silencing to gene expression: real-time analysis in single cells. Cell 116, 683-698, doi:10.1016/s0092-8674(04)00171-0 (2004).
60 Bolouri, H. et al. The molecular landscape of pediatric acute myeloid leukemia reveals recurrent structural alterations and age-specific mutational interactions. Nature medicine 24, 103-112, doi:10.1038/nm.4439 (2018).
61 Ahn, J. H. et al. Phase separation drives aberrant chromatin looping and cancer development. Nature 595, 591-595, doi:10.1038/s41586-021-03662-5 (2021).
62 Chandra, B. et al. Phase Separation Mediates NUP98 Fusion Oncoprotein Leukemic Transformation. Cancer discovery 12, 1152-1169, doi:10.1158/2159-8290.CD-21-0674 (2022).
63 Terlecki-Zaniewicz, S. et al. Biomolecular condensation of NUP98 fusion proteins drives leukemogenic gene expression. Nature structural & molecular biology 28, 190-201, doi:10.1038/s41594-020-00550-w (2021).
64 Martin, E. W. et al. Valence and patterning of aromatic residues determine the phase behavior of prion-like domains. Science 367, 694-699, doi:10.1126/science.aaw8653 (2020).
65 Holehouse, A. S., Ginell, G. M., Griffith, D. & Boke, E. Clustering of Aromatic Residues in Prion-like Domains Can Tune the Formation, State, and Organization of Biomolecular Condensates. Biochemistry 60, 3566-3581, doi:10.1021/acs.biochem.1c00465 (2021).
66 Ugolini, M. et al. Transcription bodies regulate gene expression by sequestering CDK9. Nature cell biology 26, 604-612, doi:10.1038/s41556-024-01389-9 (2024).
67 King, M. R., Ruff, K. M. & Pappu, R. V. Emergent microenvironments of nucleoli. Nucleus 15, 2319957, doi:10.1080/19491034.2024.2319957 (2024).
68 Kilgore, H. R. & Young, R. A. Learning the chemical grammar of biomolecular condensates. Nat Chem Biol, doi:10.1038/s41589-022-01046-y (2022).
69 Holehouse, A. S. & Pappu, R. V. Functional Implications of Intracellular Phase Transitions. Biochemistry 57, 2415-2423, doi:10.1021/acs.biochem.7b01136 (2018).
70 Mitrea, D. M., Mittasch, M., Gomes, B. F., Klein, I. A. & Murcko, M. A. Modulating biomolecular condensates: a novel approach to drug discovery. Nat Rev Drug Discov 21, 841-862, doi:10.1038/s41573-022-00505-4 (2022).
71 Kilgore, H. R. et al. Distinct chemical environments in biomolecular condensates. Nat Chem Biol 20, 291-301, doi:10.1038/s41589-023-01432-0 (2024).
72 Klein, I. A. et al. Partitioning of cancer therapeutics in nuclear condensates. Science 368, 1386-1392, doi:10.1126/science.aaz4427 (2020).
73 Basu, S. et al. Rational optimization of a transcription factor activation domain inhibitor. Nature structural & molecular biology, doi:10.1038/s41594-023-01159-5 (2023).
74 Uechi, H. et al. Small molecule modulation of a redox-sensitive stress granule protein dissolves stress granules with beneficial outcomes for familial amyotrophic lateral sclerosis models. bioRxiv, 721001, doi:10.1101/721001 (2024).
75 Lyons, H. et al. Functional partitioning of transcriptional regulators by patterned charge blocks. Cell 186, 327-345 e328, doi:10.1016/j.cell.2022.12.013 (2023).
76 Thody, S. A. et al. Small Molecule Properties Define Partitioning into Biomolecular Condensates. bioRxiv, 2022.2012.2019.521099, doi:10.1101/2022.12.19.521099 (2022).
77 King, M. R. et al. Macromolecular condensation organizes nucleolar sub-phases to set up a pH gradient. Cell, doi:10.1016/j.cell.2024.02.029 (2024).

## Claims

1. A conjugate comprising:
(a) a peptide which is capable of immobilizing a biomolecule that is a component of a biomolecular condensate and of disrupting the function of the biomolecular condensate;
(b) optionally a linker; and
(c) a targeting moiety capable of targeting the conjugate to a biomolecular condensate.

2. The conjugate of claim 1, wherein the targeting moiety (c)
(i) is a condensate-forming polypeptide,
(ii) is a moiety that binds to a component of a biomolecular condensate, or
(iii) is a moiety that is capable of cell penetration.

3. The conjugate of claim 1 or 2, which is a fusion polypeptide wherein component (a), component (b), if present, and component (c) are covalently bound to each other.

4. The conjugate of any one of claims 1-3, wherein the targeting polypeptide (c) does not contain an amino acid sequence from a HMGB1 polypeptide.

5. The conjugate of any one of claims 1-4, wherein the peptide (a) has a length of about 10 to about 100 amino acids, of about 12 to about 50 amino acids, of about 15 to about 20 amino acids.

6. The conjugate of any one of claims 1-5, wherein the peptide (a) is capable of self-association and comprises an amino acid sequence having at least 2, particularly at least three aromatic amino acid residues selected from F, W and Y, more particularly selected from F.

7. The conjugate of any one of claims 1-6, wherein the peptide (a) comprises or consists of (i) an amino acid sequence selected from:
- MMMMNKLVLAQFFFSCL (SEQ ID NO: 1)
- MMMMNKLVLAQAFFSCL(SEQ ID NO: 2)
- MMMMNKLVLAQFAFSCL(SEQ ID NO: 3)
- MMMMNKLVLAQFFFSAL(SEQ ID NO: 4)
- EDEDNKLVLAQFFFSCL (SEQ ID NO: 5)
- MMMFMNKFLVLAQFSCL (SEQ ID NO: 6)
- MFMMMNFKLVLAFQSCL (SEQ ID NO: 7)
- MMFMMNKLFVLAQSCFL (SEQ ID NO: 8)
- MMMMFFFNKLVLAQSCL (SEQ ID NO: 9)
- MMMMNKLVFFFLAQSCL (SEQ ID NO: 10)
- MMLALKSCFVMMQLFFN (SEQ ID NO: 11)
- AMMNFFQLMFLVKSMLC (SEQ ID NO: 12)
- FMAFLNVLLKMQCMSFM (SEQ ID NO: 13)
- CFMLNKFLVMQMSFMLA (SEQ ID NO: 14)
- CVKLSMNAQFLLMFMMF (SEQ ID NO: 15)
- MMMMNKLVLAQYYYSCL(SEQ ID NO: 16)
- MMMMNKLVLAQWWWSCL(SEQ ID NO: 17) or
(ii) an amino acid sequence comprising at least 14, at least 15 or at least 16 contiguous amino acids from an amino acid sequence according to (i); or
(iii) an amino acid sequence which has a sequence identity of at least 85%, or of at least 90% to an amino acid sequence according to (i) and/or (ii).

8. The conjugate of any one of claims 1-7, wherein the targeting moiety (c) is capable of specifically targeting the conjugate to a condensate caused by, associated with or accompanied by a pathological condition, e.g., a cancer-associated condensate or a pathogen-associated condensate.

9. The conjugate of any one of claims 1-17, wherein the targeting moiety (c) (ii) is an immunoglobulin molecule specifically recognizing a component of a biomolecular condensate, particularly a single-chain immunoglobulin molecule, more particularly a VHH antibody.

10. A nucleic acid molecule encoding the conjugate of any one of claims 1-9 optionally in operative linkage with an expression control sequence or a vector comprising the nucleic acid molecule.

11. A recombinant cell transformed or transfected with the nucleic acid molecule or the vector of claim 10.

12. A pharmaceutical composition comprising the conjugate of any one of claims 1-9, or the nucleic acid molecule or the vector of claim 10 as an active agent and a pharmaceutically acceptable carrier.

13. The conjugate of any one of claims 1-9, the nucleic acid molecule or the vector of claim 10 or the pharmaceutical composition of claim 11 for use as a medicament, e.g., for the use in human medicine.

14. The conjugate of any one of claims 1-9, the nucleic acid molecule or the vector of claim 10 or the pharmaceutical composition of claim 11 for use in the treatment of a disorder associated with the presence or with an increased amount of a pathological biomolecular condensate.

15. **In** vitro use of The conjugate of any one of claims 1-9, the nucleic acid molecule or the vector of claim 10 for arresting the dynamics of a mammalian cell.
